# FASCICULE DE BREVET EUROPEEN

(11) **EP 2 294 036 B1**
(45) Date de publication et mention de la délivrance du brevet: **05.09.2012**
(21) Numéro de dépôt: 09772658.2
(22) Date de dépôt: 11.06.2009
(51) Int. Cl.: C07C 7/13, B01D 53/04

(54) **SOLIDE HYBRIDE CRISTALLIN POREUX REDUCTIBLE POUR LA SEPARATION DE MELANGES DE MOLECULES AYANT DES DEGRES ET/OU UN NOMBRE D'INSATURATIONS DIFFERENTS**
REDUZIERBARER, PORÖSER, KRISTALLINER HYBRIDFESTSTOFF ZUR AUFTRENNUNG VON MISCHUNGEN VON MOLEKÜLEN MIT VERSCHIEDENEN GRADEN UND/ODER EINER UNTERSCHIEDLICHEN ANZAHL AN UNGESÄTTIGTEN EINHEITEN
REDUCIBLE POROUS CRYSTALLINE HYBRID SOLID FOR THE SEPARATION OF MIXTURES OF MOLECULES HAVING DIFFERENT DEGREES AND/OR A DIFFERENT NUMBER OF UNSATURATIONS

(30) Priorité: 11.06.2008 FR 0803245
(43) Date de publication de la demande: 16.03.2011
(73) Titulaire: Centre National de la Recherche Scientifique - CNRS, 75794 Paris Cedex 16 (FR); Université de Caen Basse Normandie, 14032 Caen Cedex 05 (FR); Université de Versailles Saint-Quentin-en-Yvelines, 78053 Versailles Cédex (FR); Korea Research Institute of Chemical Technology (KRICT), Yuseong-Gu Daejeon 305-343 (KR)
(72) Inventeur: SERRE, Christian, F-78370 Plaisir (FR); VIMONT, Alexandre, F-14810 Merville-Franceville (FR); LLEWELLYN, Philip, F-13011 Marseille (FR); CHANG Jong-San, Daejeon-301151 Coree (KR); HORCAJADA CORTES, Patricia, F-78190 Trappes (FR); FEREY, Gérard, F-75015 Paris (FR); DATURI, Marco, F-14610 Epron (FR); HWANG, Young-Kyu, Daejeon 305720 (KR)
(74) Mandataire: Novagraaf Technologies
(86) Numéro de dépôt international: PCT/FR2009/000699
(87) Numéro de publication internationale: WO 2010/000975

(56) Documents cités:
- WO-A-2007/113085
- WAGENER ET AL: "Opportunuties and challenges at the interface between pertrochemistry and refinery: Separation of Propane/Propene mixtures by selective adsorption on metal organic frameworks" DGMK TAGUNGSBERICHT, vol. 2007-2, 2007, pages 213-220, XP008104016 HAMBURG

## Description

### Priorité

La présente invention revendique le droit de priorité à la demande de brevet français N° 08/03245 déposée le 11 juin 2008. Cette demande est incorporée, par la présente, par référence, dans son intégralité.

### DESCRIPTION

### Domaine technique

La présente invention se rapporte à l'utilisation des solides constitués de réseau métal-organique ou « Metal-Organic Framework » (MOF), cristallin poreux pour la séparation de mélanges de molécules ayant des degrés et/ou un nombre d'insaturations différents.

Les solides MOF de la présente invention peuvent être utilisé pour la séparation de mélanges oléfine/paraffine. Ils peuvent être utilisés par exemple pour la séparation de mélanges propane/propéne. Ils peuvent également être utilisés pour la séparation de mélanges d'acétylène et de dioxyde de carbone.

Les références entre crochets [X] renvoient à la liste des références à la fin des exemples.

### État de la technique

Les réseaux métal-organique ou « Metal-Organic Framework » (MOF) sont des polymères de coordination de charpente hybride inorganique-organique comprenant des ions métalliques et des ligands organiques coordinés aux ions métalliques. Ces matériaux sont organisés en réseau mono-, bi- ou tridimensionnels où les clusters métalliques sont reliés entre eux par des ligands espaceurs de façon périodique. Ces matériaux ont une structure cristalline, sont le plus souvent poreux et sont utilisés dans de nombreuses applications industrielles telles que le stockage de gaz, l'adsorption de liquides, la séparation de liquides ou de gaz, la catalyse, etc.

On peut citer par exemple la demande de brevet US 2003-0078311 [ref. 1] qui décrit un procédé réactionnel faisant intervenir un système de catalyse comprenant un matériau MOF à base de Zinc. Ce même matériau est également utilisé pour le stockage de gaz dans le brevet US 6,929,679 [ref. 2].

En outre, les matériaux MOF basés sur des réseaux de même topologie sont qualifiés de « isoréticulaires ». Ces réseaux organisés dans l'espace ont permis d'obtenir une porosité plus homogène. Ainsi, le brevet US 6,930,193 [ref. 3] décrit plusieurs matériaux IRMOF (Isoreticular Metal-Organic Framework) à base de zinc utilisés pour le stockage de gaz.

Par ailleurs, la séparation de mélanges de composés en phase gazeuse ou liquide constitue une préoccupation industrielle majeure, touchant notamment les secteurs de l'industrie chimique, pétrochimique et/ou pharmaceutique. Par exemple, séparer les oléfines des paraffines constitue l'une des problématiques les plus importantes dans le domaine de la séparation. Le recouvrement des oléfines s'opère à partir de mélanges d'oléfines et de paraffines obtenus à partir des liquéfiats issus de résidus gazeux du craquage de dérivés pétroliers. La demande mondiale est en augmentation croissante et l'intérêt est grandissant pour développer de nouvelles méthodes de séparation peu coûteuses sur le plan énergétique. Citons par exemple le propylène qui est un précurseur fondamental pour la synthèse de nombreux polymères tels que le polypropylène. Sa production se heurte à la difficulté de séparer ce dernier du propane avec un coût économiquement acceptable.

Les technologies actuelles possèdent de nombreux inconvénients. Par exemple, celles-ci utilisent la distillation cryogénique ou la séparation à haute pression de mélanges C₂H₄/C₂H₆ et/ou C₃H₆/C₃H₈ [ref. 36]. Cependant, les différences de volatilité entre les oléfines et les paraffines sont restreintes, nécessitant des systèmes à plateaux multiples (>100) à haut reflux, ce qui est donc très onéreux.

Récemment, si de nombreuses alternatives à la séparation cryogénique ont été décrites par le passé (membranes, technologies d'adsorption...), [ref. 4] il existe depuis peu un effort de recherche sur la mise au point de nouveaux adsorbants pour purifier les oléfines.

Si des études utilisant des charbons [ref. 5] ou des gels de silices [ref. 6] ont été précédemment reportées, une alternative repose sur des silices mésoporeuses ou des polymères dans lesquels des cations de type Ag⁺ ou Cu²⁺ ont été introduits par imprégnation. [ref. 7] Les ions sont éventuellement réduits par traitement thermique sous vide, ce qui favorise l'interaction de ces matériaux avec les oléfines [ref. 8] en accord avec le modèle de Dewar-Chatt-Duncanson. [ref. 9] Néanmoins, ces interactions ne sont pas aussi fortes que celles rencontrées en chimie de coordination et donc il est assez facile de désorber l'alcène en chauffant ou réduisant la pression dans le système. Dans le cas présent, les sélectivités alcène/alcane de ce type d'adsorbant sont typiquement entre 2 et 8 pour une silice mésoporeuse chargée en ions argent ou cuivre. [Ref 7] Leurs capacités d'adsorption sont comparables à celles des zéolithes et sont relativement faibles : proches de 1 mmol.g⁻¹ à 1 bar et température ambiante. Par ailleurs, la diffusivité des molécules, particulièrement des molécules de grande taille, est relativement limitée pour ce type de matériaux dû à leur taille de pores. D'autre part, la sélectivité de ces matériaux peut difficilement être modulée/ajustée en fonction du mélange à séparer, par exemple pour un mélange propène/propane.

Les zeolithes ont également été utilisées en tant qu'adsorbants pour la séparation de mélanges oléfines/paraffines. Ce sont principalement des zéolithes cationiques de type 5A ou 13X qui ont, par exemple, une sélectivité éthylène/éthane entre 12 et 15 (à 323 K). [ref. 10] Le problème avec ces adsorbants vient du fait que le produit désiré est l'oléfine qui est très fortement adsorbée dans le matériau. Il est alors complexe de récupérer l'oléfine, ce qui constitue un frein au développement des procédés de séparation reposant sur ces matériaux.

D'autre part, l'utilisation de zéolithes, qui ont une taille de pores limitée, conduit à des problèmes de diffusivité intracristalline dans les pores et donc d'une forte résistance de transfert de masse impliquant une très mauvaise régénérabilité. Remédier à ses problèmes nécessite une température de désorption élevée, entre 100 et 200°C, avec des risques de polymériser l'oléfine dans les pores de l'adsorbant et donc de diminuer la sélectivité et la capacité d'adsorption. [ref. 11] En pratique, ces zéolithes acides ne sont pas utilisées sur le plan industriel pour de telles applications.

Si les zéolithes acides présentent de nombreux inconvénients pour la séparation des oléfines/paraffines, il semble qu'un regain d'intérêt concerne actuellement les zéolithes neutres « siliciques » ou aluminophosphates de type 8-Rings (Chabazite). [ref. 12] En effet, malgré une sélectivité thermodynamique négligeable en l'absence de cations dans les pores, ces dernières présentent néanmoins de bonne sélectivités dynamiques avec des rapports de coefficients de diffusion alcène/alcane de l'ordre de 10⁴, suggérant que ces dernières pourraient être utilisées pour la purification des oléfines. L'origine de la séparation est géométrique avec une taille de pores (range : 3.7-4.2*4.1-4.5 Å) et une tortuosité (elliptique) plus favorables aux alcènes qu'aux alcanes. Pour un procédé de purification de type PSA (Pressure Swing Adsorption), il est essentiel d'obtenir l'oléfine avec une très grande pureté définie par s=(KC₃H₆/KC₃H₈)/SR(DC₃H₆/DC₃H₈) avec K : constante d'équilibre et D : coefficient de diffusion. Les adsorbants de type CHA ou DD3R ont des performances en terme de pureté de 99 %, confirmant la possibilité de les utiliser pour ce type de séparation. Des restrictions existent néanmoins : afin de ne pas perdre la sélectivité, il faut se placer à moins de 45 % de la capacité d'équilibre (loi d'Henry) et la fréquence des cycles doit correspondre à celle de l'espèce qui diffuse le plus vite (propylène), soit ici entre 5 et 2000 secondes pour ces deux adsorbants.

Dans le même ordre d'idée, la zéolithe purement silicique ITQ-12 possède la particularité d'avoir une sélectivité propane/propylène fonction de la température. [ref. 13] Si à 30°C, l'alcène est adsorbé 100 fois plus vite que le propane, à 80°C une déformation des pores conduit à une adsorption exclusive du propylène et des tests préliminaires de type PSA montrent une production de propylène à une pureté de 99.5 %. Cependant, le coût élevé de cet adsorbant (i.e. utilisation de HF et de l'agent structurant 1,3,4 triméthylimidazolium) constitue un obstacle à son utilisation dans les procédés industriels.

Enfin, certains MOFs ou Metal-Organic-Frameworks ont été étudiés dans le cadre de la séparation des gaz. Par exemple, on peut citer l'étude concernant le trimésate de cuivre HKUST-1 (HKUST pour Hong Kong University). [ref. 14] Ce carboxylate de cuivre(II) poreux possède un système de cages de diamètre 9 Å, une surface spécifique (pour le solide réalisé lors de cette étude) de 1800 m².g⁻¹ et des dimères de cuivre. Ces derniers perdent par déshydratation leurs molécules d'eau coordinnées, laissant place à des centres métalliques cuivre(II) insaturés. Ceux-ci possèdent une acidité de Lewis permettant la coordination de molécules insaturées de type NO. [ref. 15] Une étude préliminaire de l'interaction du propane et du propylène a été réalisée sur ce solide. Les chaleurs d'adsorption calculées de ces deux molécules sont assez proches avec le HKUST-1, -30 KJ/mol (propane) et -33 KJ/mol (propylène), laissant entrevoir une faible séparation thermodynamique. Dans ce rapport, un test de perçage sur colonne chargée en solide activé préalablement, montre une différence de temps de rétention entre le propane, retenu pendant 40 minutes à 20°C, et le propylène qui ne commence à sortir de la colonne qu'après 60 minutes. La sélectivité calculée, égale à 2, à partir de ces courbes de perçage n'est cependant pas suffisante pour envisager l'application de ce matériau à un procédé de séparation des mélanges oléfines/paraffines à l'échelle industrielle. De plus, la sélectivité de ces matériaux peut difficilement être modulée/ajustée en fonction du mélange à séparer, par exemple pour un mélange propène/propane.

On peut également citer le brevet US 6,491,740 [ref. 16] qui décrit l'utilisation d'un matériau MOF à base de cuivre II pour éliminer des contaminants (CH4, H2, N2, 02) d'un mélange d'hydrocarbures ou pour retirer des hydrocarbures d'un flux gazeux. Le solide décrit dans les exemples est le HKUST-1 ; les mêmes remarques que celles faites dans le paragraphe précédent concernant les limitations et déficiences de ce matériau s'appliquent donc. On peut également noter que les MOFs à base de cuivre sont relativement onéreux. De plus, leur stabilité en présence d'eau en solution aqueuse n'est pas très bonne. [ref 15] L'utilisation d'un matériau MOF à base de cuivre (Cu₃(BTC)₂) pour la séparation de propane et propylène à été décrite par Wagener et al (DGMK Tagungsbericht, vol 2007-2, pages 213-220)

Il n'existe actuellement pas d'adsorbant satisfaisant au cahier des charges de la séparation industrielle paraffine/oléfine avec à la fois une sélectivité, un coût et des propriétés de diffusion adéquates et l'absence de polymérisation du propylène. La séparation alcanes/alcènes se fait donc actuellement par voie cryogénique et est très coûteuse énergétiquement. Trouver un adsorbant permettant de substituer cette méthode onéreuse tout en satisfaisant aux critères requis (sélectivité, diffusivité, régénération...) constituerait un progrès majeur dans ce domaine.

Parmi les inconvénients des procédés déjà connus, on peut citer par exemple:
- coûts de mise en oeuvre prohibitifs.
- manque de sélectivité du système/matériau de séparation.
- diffusivité insuffisante des espèces à séparer dans le matériau de séparation
- problèmes de lixiviation avec les matériaux de séparation existants.

Il existe donc un besoin réel de développer des systèmes permettant de séparer efficacement des mélanges oléfines/paraffines, ainsi que d'autres mélanges de molécules ayant des degrés et/ou un nombre d'insaturations différents.

### Exposé de l'invention

La présente invention a précisément pour but de répondre à ces besoins et inconvénients de l'art antérieur en proposant l'utilisation d'un solide MOF cristallin poreux comprenant des sites métalliques réduits pour séparer un mélange de molécules ayant des degrés et/ou un nombre d'insaturations différents ledit solide comprenant une structure tridimensionnelle de motifs répondant à la formule (I) suivante :
MₘOₖXₗLₚ (I) dans laquelle :
   - chaque occurrence de M représente indépendamment un ion d'un métal de transition Mz+ ou sa forme réduite M(z-1)+ choisi dans le groupe comprenant Fe, Mn, Co, Ni et V, dans lequel z est 3 ou 4; étant entendu que le rapport y=M(z-1)+/Mz+ est compris entre 0<y≤x; où x est la fraction d'ions Mz+ accessibles du solide MOF;
   - m est 1 à 12 ;
   - k est 0 à 4 ;
   - l est 0 à 18 ;
   - p est 1 à 6 ;
   - X est un anion choisi dans le groupe comprenant OH⁻, Cl⁻, F⁻, I⁻, Br⁻, SO₄²⁻, NO₃⁻, ClO₄⁻, PF₆⁻, BF₄⁻, R-(COO)ₙ⁻ où R est tel que défini ci-dessous, R¹-(COO)ₙ⁻, R¹-(S0₃)ₙ⁻, R¹-(PO₃)ₙ⁻, où R¹ est un hydrogène, un alkyle en C₁ à C₁₂, linéaire ou ramifié, éventuellement substitué, un aryle en C₆ à C₁₀ éventuellement substitué; où n représente un entier de 1 à 4 ;
   - L est un ligand espaceur comprenant un radical R comportant q groupements carboxylates 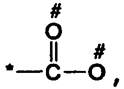 où
      - q est 1, 2, 3, 4, 5 ou 6 ;
      - * désigne le point d'attachement du carboxylate avec le radical R ;
      - # désigne les points d'attachement possibles du carboxylate à l'ion métallique ;
      - R représente :
         (i) un radical C₁₋₁₂alkyle, C₂₋₁₂alcène ou C₂₋₁₂alcyne ;
         (ii) un radical aryle mono- ou poly-cyclique, fusionné ou non, comprenant 6 à 50 atomes de carbone ;
         (iii) un hétéroaryle mono- ou poly-cyclique, fusionné ou non, comprenant 1 à 50 atomes de carbone ;
         (iv) un radical organique comprenant un élément métallique choisi dans le groupe comprenant le ferrocène, la porphyrine, la phtalocyanine ;
le radical R étant éventuellement substitué par un ou plusieurs groupes R², indépendamment choisis dans le groupe comprenant C₁₋₁₀alkyle, C₂₋₁₀alcène; C₂₋₁₀alcyne; C₃₋₁₀cycloalkyle; C₁₋₁₀hétéroalkyle; C₁₋₁₀haloalkyle; C₆₋₁₀aryle; C₃₋₂₀hétérocyclique; C₁₋₁₀alkylC₆₋₁₀aryle ; C₁₋₁₀alkylC₃₋₁₀hétéroaryle ; F ; Cl ; Br; I; -NO₂; -CN ; -CF₃; -CH₂CF₃; -OH; -CH₂OH; -CH₂CH₂OH; -NH₂; - CH₂NH₂ ; -NHCHO ; -COOH ; -CONH₂; -SO₃H; - CH₂SO₂CH₃; -PO₃H₂; ou une fonction -GR^{G1} dans laquelle G est -O-, -S-, -NR^{G2}-, -C(=O)-, -S(=O)-, -SO₂-, -C(=O)O-, - C(=O)NR^{G2}-, -OC(=O)-, -NR^{G2}C(=O)-, -OC(=O)O-, - OC(=O)NR^{G2}-, -NR^{G2}C(=O)O-, -NR^{G2}C(=O)NR^{G2}-, -C(=S)-, où chaque occurrence de R^{G2} est indépendamment des autres occurrences de R^{G2} un atome d'hydrogène ; ou une fonction C₁₋₁₂alkyle, C₁₋₁₂hétéroalkyle, C₂₋₁₀alcène ou C₂₋₁₀alcyne, linéaire, ramifiée ou cyclique, éventuellement substituée ; ou un groupe C₆₋₁₀aryle, C₃₋₁₀hétéroaryle, C₅₋₁₀hétérocyclique, C₁₋₁₀alkyleC₆₋₁₀aryle ou C₁₋₁₀alkyleC₃₋₁₀hétéroaryle dans lequel le radical aryle, hétéroaryle ou hétérocyclique est éventuellement substitué ; ou bien, lorsque G représente -NR^{G2}-, R^{G1} et R^{G2} conjointement avec l'atome d'azote auquel ils sont liés forment un hétérocycle ou un hétéroaryle éventuellement substitué.

Dans certains modes de réalisation, M peut représenter V et z être 4 (couple redox V⁴⁺/V³⁺).

Dans certains autres modes de réalisation, M peut représenter Ni et z être 3 (couple redox Ni³⁺/Ni²⁺).

Avantageusement, chaque occurrence de M représente indépendamment un ion d'un métal de transition Mz+ ou sa forme réduite M(z-1)+ choisi dans le groupe comprenant Fe, Mn, Co, Ni (z est alors 3) et V (z est alors 4).

Dans la présente, on entend par x, fraction d'ions M^{z+} accessibles du solide MOF, le ratio entre le nombre d'ions M^{z+} accessibles du solide MOF et le nombre total d'ions M^{z+} présents dans le solide MOF. Le nombre d'ions M^{z+} accessibles du solide MOF représente le nombre d'ions M^{z+} du solide MOF susceptibles d'être réduits en ions M^{(2- 1)+}. Ainsi, x représente la fraction maximale d'ions M^{(z-1)+} qu'un solide MOF selon l'invention puisse contenir.

Dans le contexte de la présente, x est strictement supérieur à zéro.

Dans certains modes de réalisation x peut représenter toute fraction comprise entre zéro (borne 0 excluse) et 1/3 (borne 1/3 incluse). Avantageusement, x peut représenter 1/3. Dans ce mode de réalisation, il s'agira de MOFs réduits par le phénomène d'auto-réduction (i.e., dans un premier temps, une température <150°C, voire < 100°C, permet de retirer les molécules d'eau et de solvant coordinées sur les sites M^{z+}. Au-dessus de 100°C, en particulier au-dessus de 150°C, les sites M^{z+} commencent à être réduits. La réduction peut s'accompagner de l'élimination d'un ligand chargé négativement (-1) pour préserver l'électroneutralité du solide MOF). Par exemple, x peut être 0.10, 0.15, 0.20, 0.25, 0.30 ou 1/3, cette dernière valeur correspondant au maximum théorique d'activation du matériau MOF par réduction des sites M^{z+} par le phénomène d'autoréduction (i.e., l'électroneutralité du solide MOF étant préservée par l'élimination de ligands chargés négativement (-1)).

Dans certains modes de réalisation x peut être supérieur à 1/3. Il s'agira là de MOFs comprenant au moins un ligand susceptible d'être oxydé (ligand redox), comme par exemple l'hydroquinone dont la forme oxydée est la quinone. Dans ce cas, la réduction du solide MOF poreux peut être accompagnée par compensation de charge due non seulement à la perte d'un contre-ion du métal (comme le mode de réalisation précédent), mais aussi due à l'oxydation du ligand organique. On peut donc parvenir à augmenter la teneur en ion réduit M(z-1)+ au-delà du ratio x=1/3. Le lecteur pourra se référer à l'Exemple 31 ci-dessous pour un exemple de réalisation. En théorie, le taux de réduction des ions Mz+ peut atteindre jusqu'à 100% (i.e., x=1). Par exemple, x peut être 0.35, 0.40, 0.45, 0.50, 0.60, 0.70, 0.80, 0.90 voire 1.

Dans le cadre de la présente invention, le terme « substitué » désigne par exemple le remplacement d'un radical hydrogène dans une structure donnée par un radical R² tel que défini précédemment. Lorsque plus d'une position peut être substituée, les substituants peuvent être les mêmes ou différents à chaque position.

On entend par « ligand espaceur » au sens de la présente invention, un ligand (incluant par exemple les espèces neutres et les ions) coordiné à au moins deux métaux, participant à l'éloignement entre ces métaux et à la formation d'espaces vides ou pores. Le ligand espaceur peut comprendre 1 à 6 groupements carboxylates, tels que définis précédemment, qui peuvent être monodentates ou bidentates, c'est-à-dire comprendre un ou deux points d'attachement au métal. Les points d'attachement au métal sont représentés par le signe # dans les formules. Lorsque la structure d'une fonction A comporte deux points d'attachement #, cela signifie que la coordination au métal peut se faire par l'un, l'autre ou les deux points d'attachement.

On entend par « alkyle » au sens de la présente invention, un radical carboné linéaire, ramifié ou cyclique, saturé ou insaturé, éventuellement substitué, comprenant 1 à 12 atomes de carbone, par exemple 1 à 10 atomes de carbone, par exemple 1 à 8 atomes de carbone, par exemple 1 à 6 atomes de carbone.

On entend par « alcène » au sens de la présente invention, un radical alkyle, tel que défini précédemment, présentant au moins une double liaison carbone-carbone.

On entend par « alcyne » au sens de la présente invention, un radical alkyle, tel que défini précédemment, présentant au moins une triple liaison carbone-carbone.

On entend par « aryle » au sens de la présente invention, un système aromatique comprenant au moins un cycle satisfaisant la règle d'aromaticité de Hückel. Ledit aryle est éventuellement substitué et peut comprendre de 6 à 50 atomes de carbone, par exemple 6 à 20 atomes de carbone, par exemple 6 à 10 atomes de carbone.

On entend par « hétéroaryle » au sens de la présente invention, un système comprenant au moins un cycle aromatique de 5 à 50 chaînons parmi lesquels au moins un chaînon du cycle aromatique est un hétéroatome, notamment choisi dans le groupe comprenant le soufre, l'oxygène, l'azote, le bore. Ledit hétéroaryle est éventuellement substitué et peut comprendre de 1 à 50 atomes de carbone, de préférence 1 à 20 atomes de carbone, de préférence 3 à 10 atomes de carbone.

On entend par « cycloalkyle » au sens de la présente invention, un radical carboné cyclique, saturé ou insaturé, éventuellement substitué, qui peut comprendre 3 à 20 atomes de carbone, de préférence 3 à 10 atomes de carbone.

On entend par « haloalkyle » au sens de la présente invention, un radical alkyle tel que défini précédemment, ledit système alkyle comprenant au moins un halogène.

On entend par « hétéroalkyle » au sens de la présente invention, un radical alkyle tel que défini précédemment, ledit système alkyle comprenant au moins un hétéroatome, notamment choisi dans le groupe comprenant le soufre, l'oxygène, l'azote, le bore.

On entend par « hétérocycle » au sens de la présente invention, un radical carboné cyclique comprenant au moins un hétéroatome, saturé ou insaturé, éventuellement substitué, et qui peut comprendre 2 à 20 atomes de carbone, de préférence 5 à 20 atomes de carbone, de préférence 5 à 10 atomes de carbone. L'hétéroatome peut être par exemple choisi dans le groupe comprenant le soufre, l'oxygène, l'azote, le bore.

On entend par « alkoxy », « aryloxy », « hétéroalkoxy » et « hétéroaryloxy » au sens de la présente invention, respectivement un radical alkyle, aryle, hétéroalkyle et hétéroaryle liés à un atome d'oxygène.

On entend par « alkylthio », « arylthio », « hétéroalkylthio » et « hétéroarylthio » au sens de la présente invention, respectivement un radical alkyle, aryle, hétéroalkyle et hétéroaryle liés à un atome de soufre.

Par « structure tridimensionnelle », on entend une succession ou répétition tridimensionnelle de motifs de formule (I) tel que l'on entend de façon conventionnelle dans le domaine des matériaux MOFs, que l'on caractérise également comme « polymères métallo-organiques ».

Dans la présente, le terme « insaturation » désigne une liaison multiple (double ou triple) ou un doublet libre présent sur un hétéroatome, choisi parmi O, S, N et P.

Dans la présente, la liaison multiple peut lier :
- deux atomes de carbone (C=C, C≡C),
- un atome de carbone et un hétéoatome (C=O, C=S, -C=N-, C≡N), ou
- deux hétéroatomes (N=N, P=O, P=S).

Le terme « degré d'insaturation » se réfère à la nature d'une liaison multiple, à savoir une double liaison ou une triple liaison, celle-ci ayant un degré d'insaturation supérieur (3) à une double liaison (2).

Le nombre d'insaturations est le compte d'insaturations d'une molécule, tel que défini ci-dessus. Il s'agit de la somme *N* des liaisons multiples et doublets libres présents sur une molécule donnée, tels que définis ci-dessus.

A titre d'exemple, les insaturations peuvent se présenter sous la forme d'une double liaison C=C, d'une triple liaison C≡C, d'une fonction carboxyle -CO₂H, d'une fonction oxo C=O, d'une fonction aldéhyde -C(=O)H, d'une fonction cyanure -CN, d'une fonction nitro -NO₂, d'une fonction ester -CO₂R, C=S, -C=N-, C≡N, N=N, P=O, P=S, d'un groupe phényle, d'un groupe aryle et/ou d'un groupe hétéroaryle.

L'invention a également pour objet un procédé pour séparer un mélange de molécules ayant des degrés et/ou un nombre d'insaturations différents par adsorption préférentielle des molécules à degré et/ou nombre d'insaturations supérieur sur un solide MOF cristallin poreux tel que défini ci-dessus, ledit procédé comprenant au moins une étape réactionnelle consistant
(i) à mélanger dans un solvant polaire :
   - au moins une solution comprenant au moins un précurseur inorganique métallique se présentant sous la forme de métal M, d'un sel métallique de M ou d'un complexe de coordination comprenant un ion métallique de M
   - au moins un ligand L' comprenant un radical R comportant q groupements *-C(=O)-R³, où
      - M, q et R sont tels que définis précédemment,
      - * désigne le point d'attachement du groupement avec le radical R,
      - R³ est choisi dans le groupe comprenant un OH, un OY, avec Y est un cation alcalin, un halogène, un
   radical -OR⁴, -O-C(=O)R⁴, -NR⁴R^{4'}, où R⁴ et R^{4'} sont des radicaux alkyles en C₁₋₁₂, pour obtenir un matériau MOF ;
(ii) à activer le matériau MOF obtenu en (i) ; et
(iii) à mettre en contact le matériau MOF obtenu à l'étape (ii) avec un mélange de molécules ayant des degrés et/ou un nombre d'insaturations différents.

M est un ion d'un métal de transition choisi dans le groupe comprenant Fe, Mn, Co, Ni et V, comme défini précédemment.

Sauf indication contraire, les divers modes de réalisation qui suivent concernant les matériaux MOF s'appliquent autant à l'utilisation qu'au procédé de l'invention précités.

La structure cristalline particulière des solides MOF selon l'invention, ainsi que la possibilité de réduire certains sites métalliques (sites métalliques accessibles) en ions M^{(z-1)+} sans affecter la structure de ces solides, procurent à ces matériaux des propriétés spécifiques particulièrement adaptées à la séparation de mélanges de molécules ayant des degrés et/ou un nombre d'insaturations différents.

Dans les solides MOF de l'invention, M est choisi dans le groupe comprenant Fe, Mn, Co, Ni et V. Les solides MOF de l'invention peuvent également contenir un mélange de ces métaux. M est avantageusement le Fe, sous sa forme oxydée (Fe(III)), ou sa forme réduite (Fe(II)).

Comme indiqué précédemment, M est un ion de métal de transition M^{z+} dans lequel z est 3. Ainsi, chaque occurrence de M peut être indépendamment Fe(III), Mn(III), Co(III) ou V(III), ou la forme réduite Fe(II), Mn(II), Co(II) ou V(II), respectivement.

M peut également représenter V(IV), ou sa forme réduite V(III). Dans ce cas, z représente 4.

M peut également représenter Ni(III), ou sa forme réduite Ni(II). Dans ce cas, z représente 3.

Lorsque M est un mélange de métaux, pour chaque métal, z peut être avoir une valeur identique ou différente.

Dans un mode de réalisation de l'invention, les solides de l'invention comprennent une structure tridimensionnelle de motifs de formule (I) dans laquelle M peut représenter un seul type de métal, par exemple le Fe, sous forme Fe(II) et/ou Fe(III) (i.e, z=3).

En général, le rapport y=Fe²⁺/Fe³⁺ est strictement supérieur à zéro.

Dans certains modes de réalisation le rapport y=Fe²⁺/Fe³⁺ peut être compris entre 0<y≤1/3.

Dans d'autres modes de réalisation, y peut être supérieur à 1/3, lorsque le solide MOF poreux contient au moins un ligand redox susxeptible d'être oxydé. Ainsi, dans certains modes de réalisation, 0<y≤1 (voir Exemple 31 pour un exemple de mode de réalisation).

Dans un autre mode de réalisation de l'invention, les solides de l'invention peuvent comprendre une structure tridimensionnelle de motifs de formule (I) dans laquelle M peut représenter un mélange de différents métaux, par exemple le Fe et le Mn, dans lequel pour chaque métal z est égal à 3.

Dans un autre mode de réalisation de l'invention, les solides de l'invention peuvent comprendre une structure tridimensionnelle de motifs de formule (I) dans laquelle M peut représenter un mélange de différents métaux, par exemple le Fe, le Co, le Ni ou le Mn, dans lequel pour chaque métal z est égal à 3, ou le V dans lequel z repésente 4.

Dans un mode de réalisation particulier, M^{z+} représente le Fe trivalent octaédrique avec z égal à 3. Dans ce mode de réalisation, le Fe présente un nombre de coordination de 6.

Par « nombre de coordination », on entend le nombre d'anions avec lesquels le cation M est lié.

L'utilité des matériaux MOFs selon l'invention repose sur le fait que la structure du matériau MOF demeure stable après réduction d'ions métalliques M^{z+} du matériau en ions M^{(z-1)+}. En effet, de manière inattendue, il a été découvert que les matériaux MOF selon l'invention (i.e., carboxylates de métal, où le métal peut être Fe, Mn, Co, Ni, V, ou une combinaison de ceux-ci) conservent leur structure intègre lorsque tout ou partie des ions accessibles M^{z+} du matériau MOF sont réduits en ions M^{(z-1)+} correspondants. Cette propriété est remarquable car elle permet d'utiliser les matériaux MOF de l'invention pour séparer un mélange de molécules ayant des degrés et/ou un nombre d'insaturations différents. Elle permet en outre de moduler la sélectivité du matériau MOF en question dans le processus de séparation.

En effet, les centres métalliques insaturés M^{z+} et M^{(z-1)+} possédent un caractère accepteur d'électrons (acide de Lewis) et peuvent former des complexes π avec des molécules possédant un caractère donneur d'électrons (base de Lewis), tel que des molécules comprenant une ou plusieurs insaturations au sens de la présente invention. Dans le cas où la molécule comprenant une insaturation est un alcène ou un alcyne, la stabilisation des complexes ainsi formés peut être rationalisée d'après le modèle de Dewar-Chatt-Duncanson [ref. 9], considérant d'une part la structure électronique de la double ou triple liaison carbone-carbone de l'oléfine et d'autre part des orbitales vacantes du site d'adsorption: la liaison entre l'alcène ou l'alcyne implique (i) une délocalisation des électrons des orbitales π liantes de l'hydrocarbure insaturé vers les orbitales vacantes du site d'adsorption (interaction donneur-accepteur par liaison σ) (ii) une délocalisation des electrons des orbitales d partiellement remplies du site d'adsorption vers les orbitales π* anti-liantes de l'hydrocarbure insaturé (liaison π). L'ion métallique réduit M^{(z-1)+} possède un électron d supplémentaire par rapport à M^{z+}, ce qui renforce la liaison π avec l'hydrocarbure et accroit ainsi la stabilité du complexe formé. Ainsi, le matériau MOF de formule (I) comprenant à la fois des ions métalliques M^{z+} et leur forme réduite M^{(z-1)+} (le rapport y=M^{(z-1)+} /M^{z+} est strictement supérieur à zéro) va pouvoir interagir plus fortement avec de telles molécules.

Le raisonnement ci-dessus peut être tenu à l'identique pour les molécules contenant un ou plusieurs doublets libres. De la même manière, le matériau MOF de formule (I) comprenant à la fois des ions métalliques M^{z+} et leur forme réduite M^{(z-1)+} (le rapport y=M^{(z-1)+}/M^{z+} est strictement supérieur à zéro) va pouvoir interagir plus fortement avec de telles molécules.

Les inventeurs ont mis en évidence expérimentalement cette propriété des solides MOF de l'invention, comme décrit dans la partie « Exemples » ci-dessous.

Dans un mode de réalisation particulier, la réduction des ions métalliques M^{z+} du matériau MOF peut être effectuée en activant le matériau à une température suffisamment élevée pour conduire à la réduction de sites M^{z+} en ions M^{(z-1)+} par le phénomène d'auto-réduction. Voir par exemple, Battiston *et al.,* [ref. 41] ; Magnacca *et al.* [ref. 42]. Dans un premier temps, une température <150°C, voire <100°C, permet de retirer les molécules d'eau et de solvant coordinées sur les sites M^{z+}. Au-dessus de 100°C, en particulier au-dessus de 150°C, les sites M^{z+} commencent à être réduits. La réduction peut s'accompagner de l'élimination d'un ligand chargé négativement (-1) pour préserver l'électroneutralité du solide MOF. La réduction peut avoir lieu aussi à l'aide d'un pompage sous vide ou d'un agent réducteur, comme spécifié plus loin.

Dans un autre mode de réalisation, la réduction des ions métalliques M^{z+} du matériau MOF peut être précédée par un traitement du matériau avec une source d'ions F⁻. Par exemple, la source d'ions F- peut être KF, CsF, LiF, MgF₂, CaF₂, ou un sel fluoré d'amine comme NH₄F. Avantageusement, il peut s'agir de NH₄F, par exemple en solution aqueuse.

Sans vouloir être lié en aucune façon à une théorie particulière, il semble pouvoir être avancé que ce traitement permet d'échanger les nitrates présents dans les pores du matériaux par des ions F⁻. Ce type d'échange anionique a été observé dans d'autres matériaux (Philip L. Llewellyn, Sandrine Bourrelly, Christian Serre, Alexandre vimont, Marco Daturi, Lomig Hamon, Guy De Weireld, Jong-San Chang, Do-Young Hong, Young Kyu Hwang, Sung Hwa Jhung, Gérard Férey "High Uptakes of CO2 and CH4 in Mesoporous Metal Organic Frameworks MIL-100 and MIL-101", Langmuir, 2008, 24, 7245-7250; [ref 57]).

Ainsi, le traitement préalable avec une source d'ions F⁻ permet de réduire les sites métalliques M^{z+} à plus basse température (les ions F⁻ étant plus petits et plus légers, et par conséquent plus labiles), et d'obtenir un matériaux réduit de surface spécifique plus importante.

Dans un mode de réalisation particulier, la teneur du matériau MOF de formule (I) en ions réduits M^{(z-1)+} peut être contrôlée/ajustée afin de moduler la sélectivité du solide MOF vis-à-vis des espèces à séparer. La teneur en ions réduits M^{(z-1)+} peut être modulée en exposant le solide MOF à une température plus ou moins élevée et/ou pendant un temps d'activation plus ou moins long. Dans ce contexte, par « temps d'activation », on entend le temps pendant lequel la réduction des sites métalliques accessibles M^{z+} est effectuée. En général, à conditions égales, plus la température est élevée, plus la teneur en ions réduits M^{(z-1)+} du matériau MOF est élevée. De la même manière, plus le temps d'activation est long, plus la teneur en ions réduits M^{(z-1)+} du matériau MOF est élevée. Comme discuté dans le paragraphe ci-dessus, l'activation thermale peut être précédée par un traitement avec une source d'ions F⁻, telle KF, CsF, LiF, MgF₂, CaF₂, ou un sel fluoré d'amine comme NH₄F. Ceci a pour effet de permettre de réduire la température et/ou le temps d'activation.

Les méthodes de préparation de matériaux MOF sont bien connues de l'homme du métier, et ne seront pas développées dans le cadre de la présente demande.

On entend par « site métallique insaturé M^{z+} » des ions M^{z+} dont le nombre de coordination est inférieur au nombre maximal de ligands auxquels l'atome métallique M^{z+} peut être lié par une liaison σ. Par exemple, lorsque le solide MOF de formule (I) est à base d'ion Fe³⁺ trivalent octaédrique, le nombre de coordination maximal des ions Fe³⁺ est 6. Ainsi, un site métallique insaturé dans un MOF de formule (I) où M est Fe et z est 3 peut être un ion Fe³⁺ de coordination 5. Par exemple, dans un tel solide où les ions métalliques Fe³⁺ de coordinance 6 peuvent être coordinés à des molécules d'eau (coordinance 6), des sites métalliques Fe³⁺ insaturés peuvent être obtenus par chauffage du matériau MOF sous vide pour éliminer les molécules d'eau et/ou de solvant des sites Fe³⁺ accessibles. Le même raisonnement peut être tenu pour les ions métalliques Mn³⁺, V³⁺ et Co³⁺: des ligands neutres peuvent être éliminés des sites accessibles Mn³⁺ , V³⁺ et/ou Co³⁺ par chauffage à une température adéquate pour générer des sites Mn³⁺, V³⁺ et/ou Co³⁺ insaturés. Le même raisonnement peut être tenu pour les couples redox V⁴⁺/V³⁺ et Ni³⁺/Ni²⁺.

Après chauffage à une température plus élevée, des sites Fe²⁺, Mn²⁺, V²⁺ et/ou Co²⁺ insaturés peuvent être obtenus par le phénomène d'auto-réduction évoqué précédemment. De même, des sites V³⁺ insaturés peuvent être obtenus par auto-réduction d'ions V⁴⁺. De la même manière, des sites Ni²⁺ insaturés peuvent être obtenus par auto-réduction d'ions Ni³⁺.

Les ions métalliques peuvent être isolés ou regroupés en « clusters » métalliques. Les solides MOF selon l'invention peuvent par exemple êtres construits à partir de chaînes d'octaèdres ou de trimères d'octaèdre.

On entend par « cluster métallique » au sens de la présente invention un ensemble d'atomes contenant au moins deux ions métalliques liés par des liaisons ionocovalentes, soit directement par des anions, par exemple O, OH, Cl, etc., soit par le ligand organique.

De plus, les solides MOF selon l'invention peuvent se présenter sous différentes formes ou « phases » compte tenu des diverses possibilités d'organisation et de connexions des ligands à l'ion métallique ou au groupement métallique.

On entend par « phase » au sens de la présente invention une composition hybride comprenant au moins un métal et au moins un ligand organique possédant une structure cristalline définie.

L'organisation spatiale cristalline des solides de la présente invention est à la base des caractéristiques et propriétés particulières de ces matériaux. Elle régit notamment la taille des pores, qui a une influence sur la surface spécifique des matériaux et sur les caractéristiques d'adsorption. Elle régit également la densité des matériaux, celle-ci étant relativement faible, la proportion de métal dans ces matériaux, la stabilité des matériaux, la rigidité des structures, etc.

En outre, la taille des pores peut être ajustée par le choix de ligands L appropriés.

Dans un mode de réalisation particulier, le ligand L du motif de formule (I) des solides MOF de la présente invention peut être un ligand di-, tri-, tétra- ou hexacarboxylate choisi dans le groupe comprenant :

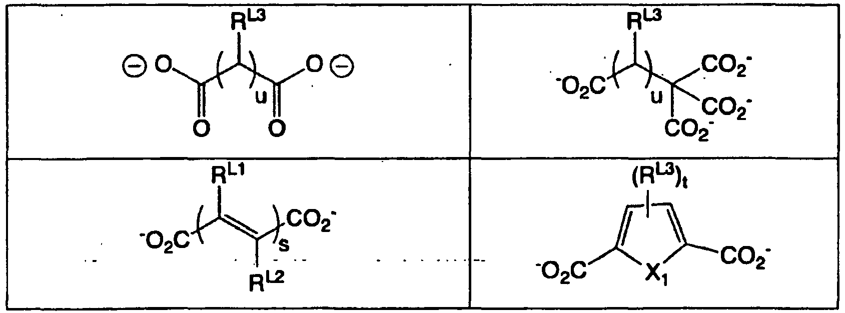

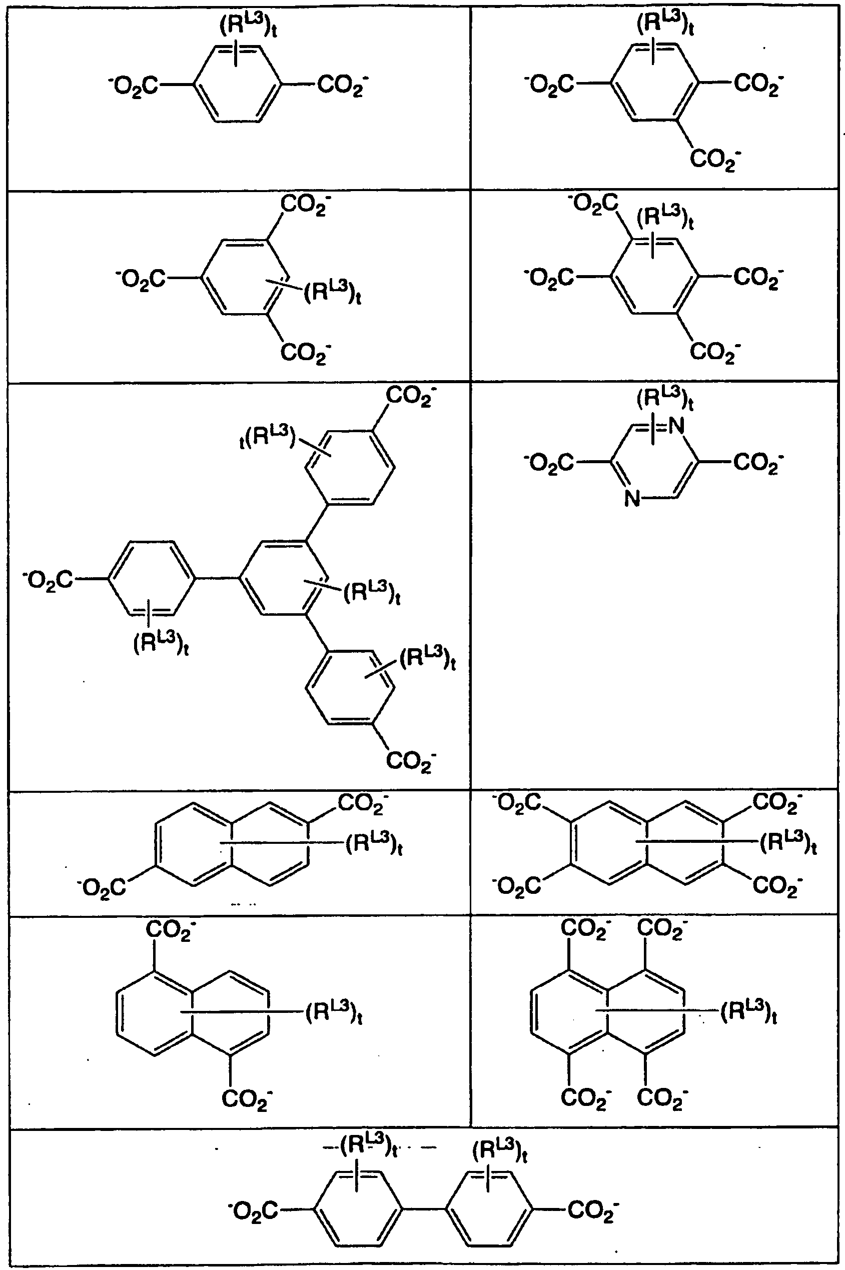

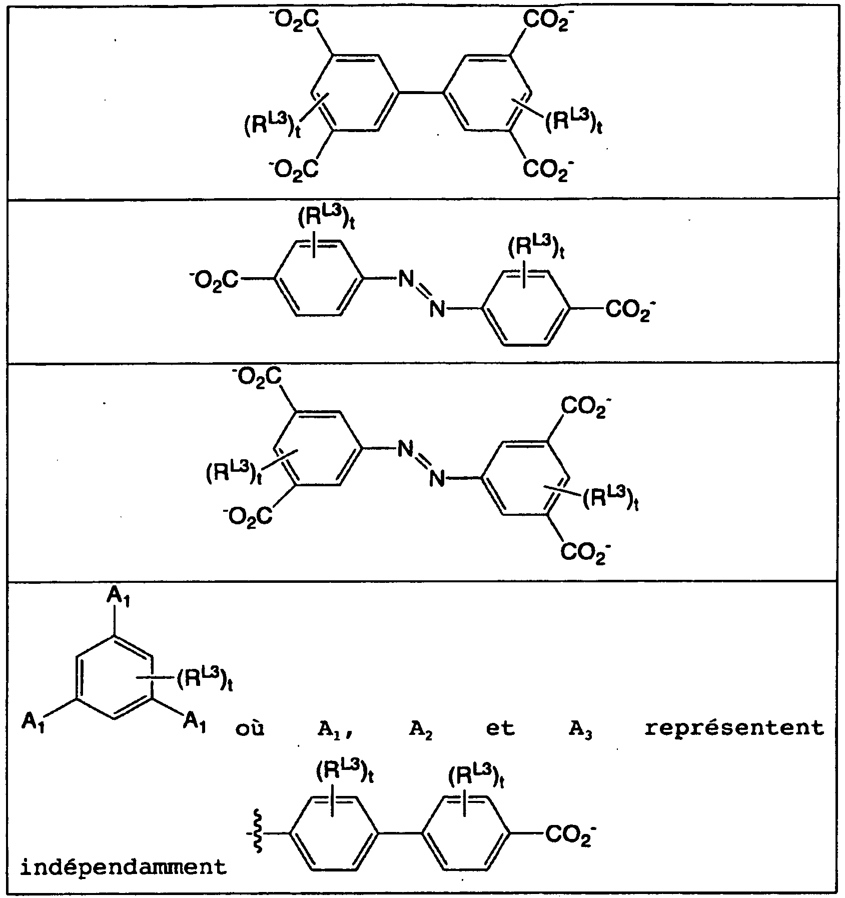

dans lesquels :
X₁ représente O ou S,
s représente un entier de 1 à 4,
chaque occurrence de t représente indépendamment un entier de 1 à 4,
u représente un entier de 1 à 6,
R^{L1} et R^{L2} représentent indépendamment H, un halogène ou un alkyle en C₁ à C₆ (de préférence méthyle ou éthyle), et
chaque occurrence de R^{L3} représente indépendamment H, un halogène (de préférence F, Cl ou Br), OH, NH₂, NO₂ ou un alkyle en C₁ à C₆ (de préférence méthyle ou éthyle).

En particulier, le ligand L du motif de formule (I) des solides MOF de la présente invention peut être un ligand di-, tri-, tétra- ou hexa-carboxylate choisi dans le groupe comprenant : C₂H₂(CO₂⁻)₂ (fumarate), C₂H₄(CO₂⁻)₂ (succinate), C₃H₆(CO₂⁻)₂ (glutarate), C₄H₄CO₂⁻)₂ (muconate), C₄H₈(CO₂⁻)₂ (adipate), C₅H₃S(CO₂⁻)₂ (2,5-thiophènedicarboxylate), C₆H₄(CO₂⁻)₂ (téréphtalate), C₆H₂N₂(CO₂⁻)₂ (2,5-pyrazine dicarboxylate), C₇H₁₆(CO₂⁻)₂ (azélate), C₁₀H₆(CO₂⁻)₂ (naphtalène-2,6-dicarboxylate), C₁₂H₈(CO₂⁻)₂ (biphényle-4,4'-dicarboxylate), C₁₂H₈N₂(CO₂⁻)₂ (azobenzènedicarboxylate), C₁₂H₆Cl₂N₂(CO₂⁻)₂ (dichloroazobenzènedicarboxylate), C₁₂H₆N₂(CO₂⁻)₄ (azobenzènetetracarboxylate), C₁₂H₆N₂(OH)₂(CO₂⁻)₂ (dihydroxoazobenzènedicarboxylate), C₆H₃(CO₂⁻)₃ (benzène-1,2,4-tricarboxylate), C₆H₃(CO₂⁻)₃ (benzène-1,3,5-tricarboxylate), C₂₄H₁₅(CO₂⁻)₃ (benzène-1,3,5-tribenzoate), C₄₂H₂₇(CO₂⁻)₃ (1,3,5-tris[4'-carboxy(1,1'-biphenyl-4-yl)benzene), C₆H₂(CO₂⁻)₄ (benzène-1,2,4,5-tétracarboxylate, C₁₀H₄(CO₂⁻)₄ (naphtalène-2,3,6,7-tétracarboxylate), C₁₀H₄(CO₂⁻)₄ (naphtalène-1,4,5,8-tétracarboxylate), C₁₂H₆(CO₂⁻)₄ (biphényl-3,5,3',5'-tétracarboxylate, et les analogues modifiés choisis dans le groupe comprenant le 2-aminotéréphtalate, le 2-nitrotéréphtalate, le 2-méthyltéréphtalate, le 2-chlorotéréphtalate, le 2-bromotéréphtalate, 2,5-perfluorotéréphtalate, le 2,5-dihydroxotéréphtalate, le tétrafluorotéréphtalate, le 2,5-dicarboxytéréphtalate, le tétraméthyl-4,4'-biphénydicarboxylate, le dicarboxy-4,4'-biphénydicarboxylate.

La plupart des ligands carboxylates listés ci-dessus sont commerciaux. Le lecteur pourra se référer à la partie Exemples pour la préparation des ligands carboxylates non commerciaux.

En particulier, l'anion X du motif de formule (I) de la présente invention peut être choisi dans le groupe comprenant OH⁻, Cl⁻, Br⁻, F⁻, R―(COO)ₙ⁻, PF₆⁻, NO₃⁻, SO₄²⁻, ClO₄⁻, avec R et n tels que définis précédemment. Dans un mode de réalisation particulier, l'anion X peut être choisi dans le groupe comprenant OH⁻, Cl⁻, F⁻ et R―(COO)ₙ⁻ où R représente -CH₃, -C₆H₃, -C₆H₄, -C₁₀H₄ ou -C₆(CH₃)₄.

En particulier, le solide MOF selon l'invention, peut comprendre un pourcentage massique de M en phase sèche de 5 à 50%, de préférence de 18 A 31%.

Le pourcentage massique (%m) est une unité de mesure utilisée en chimie et en métallurgie pour désigner la composition d'un mélange ou d'un alliage, c'est-à-dire les proportions de chaque composant dans le mélange.

1 %m d'un composant = 1g du composant pour 100 g de mélange ou encore 1 kg dudit composant pour 100 kg de mélange.

Les solides MOF de la présente invention présentent notamment l'avantage d'avoir une stabilité thermique jusqu'à une température de 350°C. Plus particulièrement, ces solides présentent une stabilité thermique de 120°C à 350°C.

Comme indiqué précédemment, dans les solides MOF de l'invention, au moins une partie de la ou des molécules contenant au moins une insaturation (base de Lewis) se coordine avec M. Avantageusement, au moins 1 à 5 mmol de ladite molécule par gramme de solide sec se coordine avec M.

La partie de la ou des molécules contenant au moins une insaturation qui ne se coordine pas avec M peut remplir avantageusement l'espace libre dans les pores.

Les inventeurs ont mis en évidence que les matériaux MOF comprenant une structure tridimensionnelle de motifs de formule (I) peuvent se présenter sous la forme d'une structure rigide ou flexible. En effet, un matériau MOF peut se gonfler et se dégonfler faisant varier l'ouverture des pores en fonction de la nature des molécules adsorbées qui peuvent être, par exemple, des solvants et/ou des gaz.

On entend par « structure rigide », au sens de la présente invention des structures qui se gonflent ou se contractent que très faiblement, c'est-à-dire avec une amplitude jusqu'à 10%.

On entend par « structure flexible », au sens de la présente invention des structures qui se gonflent ou se contractent avec une grande amplitude, notamment avec une amplitude supérieure à 10%, de préférence supérieure à 50%. En particulier, un matériau MOF de structure flexible peut se gonfler ou se contracter avec une amplitude de 10% à 300%, de préférence de 50 à 300%.

La présente invention peut être mise en oeuvre avec des matériaux MOF de structure rigide ou flexible.

Des résultats expérimentaux ont mis en évidence que des matériaux MOF à structure rigide était adaptés à la mise en oeuvre de la présente invention.

Avantageusement, le solide MOF de la présente invention se présente sous la forme d'une structure robuste, qui a une charpente rigide et ne se contracte que très peu lorsque les pores se vident de leur contenu qui peut être, par exemple, du solvant, de l'acide carboxylique non coordiné, etc. En particulier, le solide MOF selon l'invention, peut avoir une structure rigide qui se gonfle ou se contracte avec une amplitude allant de 0 à 10% en fonction de la nature des molécules adsorbées qui peuvent être, par exemple, des solvants et/ou des gaz.

Les structures des solides MOF peuvent par exemple être construites à base de chaînes ou de trimères d'octaèdres.

Selon un mode de réalisation de l'invention, le solide MOF de structure rigide peut avoir un pourcentage massique de M en phase sèche de 5 à 50%, de préférence de 18 à 31%. Avantageusement, M représentera ici le fer.

Le solide MOF de structure rigide selon l'invention peut avoir une taille de pores de 0,4 à 6 nm, en particulier de 0,5 à 5,2 nm, plus particulièrement de 0,5 à 3,4 nm.

Le solide MOF de structure rigide selon l'invention peut avoir un volume poreux de 0,5 à 4 cm³/g, en particulier de 0,05 à 2 cm³/g.

Dans le cadre de l'invention, le volume poreux signifie le volume accessible pour les molécules de gaz et/ou de liquide.

En particulier, le solide MOF selon l'invention peut avoir une surface spécifique (BET) de 5 à 6000 m²/g, de préférence de 5 à 4500 m²/g.

Dans un mode de réalisation particulier de l'invention, le solide MOF de structure flexible peut avoir un pourcentage massique de M en phase sèche de 5 à 40%, de préférence de 18 à 31%. Avantageusement, M représentera ici le fer.

Par exemple, dans le cadre de l'invention, le solide MOF de structure flexible peut avoir une taille de pores de 0,4 à 6 nm, en particulier de 0,5 à 5,2 nm, et plus particulièrement de 0,5 à 1,6 nm.

Par exemple, le solide MOF de structure flexible selon l'invention peut avoir un volume poreux de 0 à 3 cm³/g, en particulier de 0 à 2 cm³/g.

Le solide MOF de l'invention peut avoir un volume poreux de 0 à 4 cm²/g, en particulier de 0,5 à 4 cm²/g.

Le solide MOF de l'invention peut avoir une capacité de charge en molécules contenant au moins une insaturation de 0,5 à 50 mmol de molécule par gramme de solide sec.

Dans le cadre de la présente invention, la capacité de charge signifie la capacité de stockage de molécules ou la quantité de molécules adsorbé dans le matériau. La capacité de charge peut être exprimée en capacité massique (gramme/gramme) ou en capacité molaire (mol/mol) ou en d'autres ( mol/gramme, gramme/mol, etc.)

Différents matériaux MOF ont été élaborés par les inventeurs à l'Institut Lavoisier de Versailles nommés « MIL » (pour « Matériau Institut Lavoisier »). L'appellation « MIL » de ces structures est suivie d'un nombre arbitraire n donné par les inventeurs pour identifier les différents solides.

D'autres structures sont nommées « PIZA » (pour « Porphyrinic Illinois Zeolite Analogue ») ou « CPO » (pour « Coordination Polymer Oslo »).

Les nomenclatures MIL, PIZA et CPO sont des nomenclatures conventionnelles dans le domaine technique en question, qui sont bien connues de l'Homme du métier. Elles sont en effet couramment utilisées dans les publications scientifiques.

Un certain nombre de structures ont été mises en évidence par les inventeurs à ce jour. Il s'agit notamment des structures dites MIL-88A, MIL-89, MIL-100, MIL-101, MIL-102, MIL-88B_4CH3, MIL-88B_2OH, MIL-126, MIL-127, CPO-27 et PIZA-1.

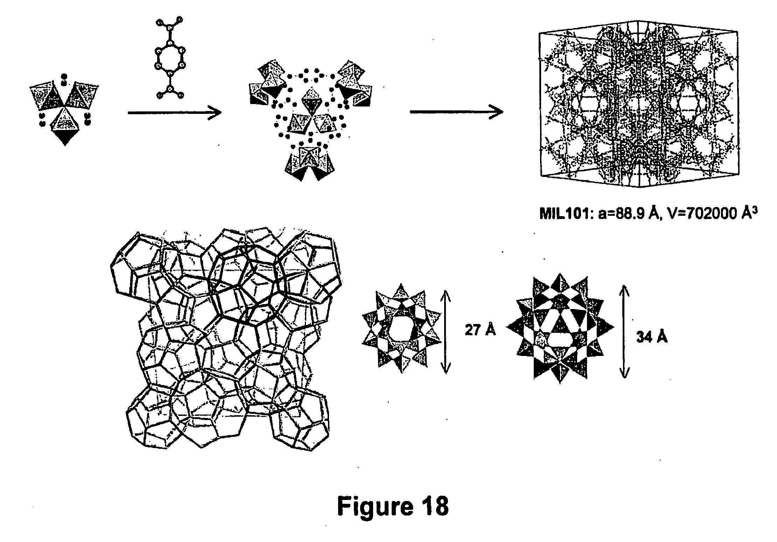

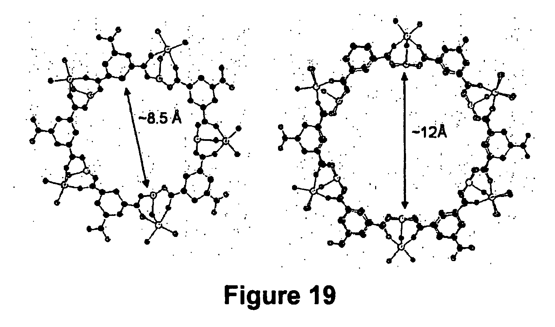

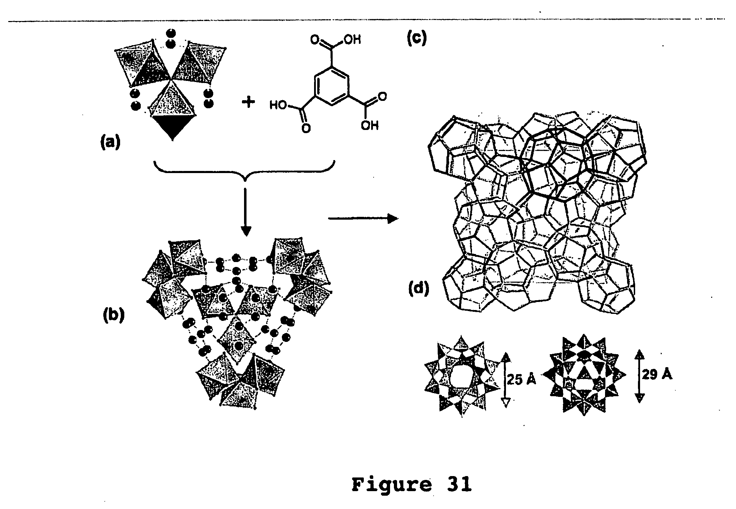

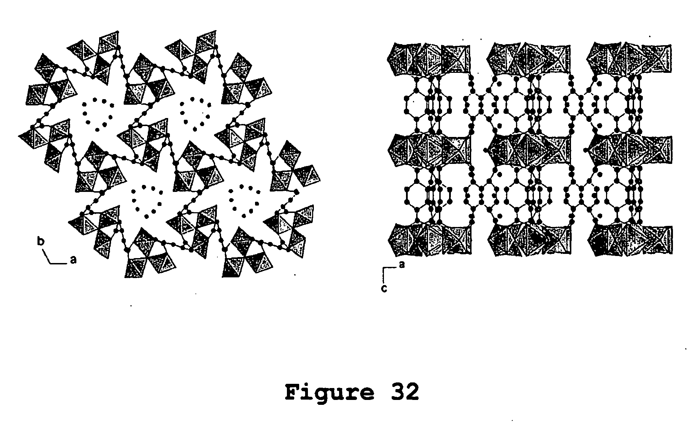

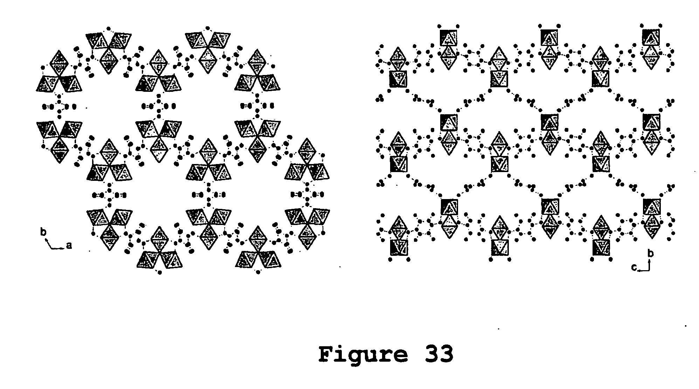

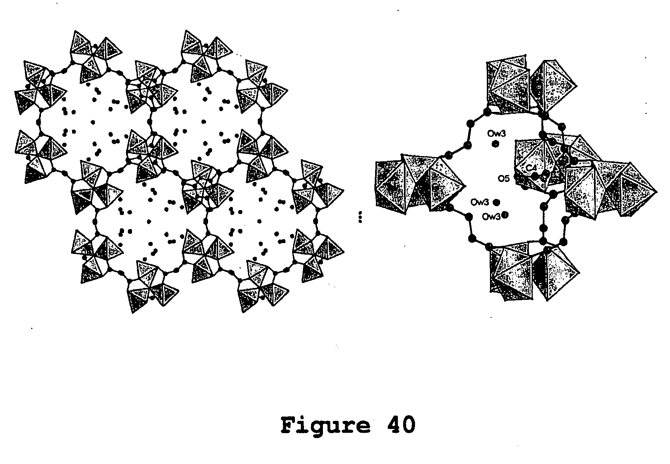

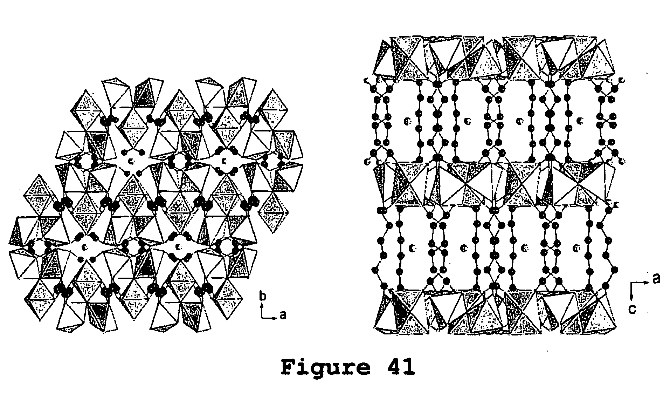

Les caractéristiques cristallographiques de ces structures sont connues, et ont fait l'objet de nombreux rapports. On citera par exemple :
**MIL-88A** : (a) Serre et al., « Role of solvent-host interactions that lead to very large swelling of hybrid frameworks », Science, 2007, Vol. 315, 1828-1831 [ref 33]; (b) Surblé et al., « A new isoreticular class of metal-organic frameworks with the MIL-88 topology », Chem. Comm., 2006, 284-286 [ref 47]; (c) Mellot-Draznieks et al., « Very large swelling in hybrid frameworks : a combined computational and power diffraction study », J. Am. Chem. Soc., 2005, Vol. 127, 16273-16278 [ref 48]. La structure d'un solide MIL-88A hydraté est représentée en figure 40, et les données cristallographiques sont répertoriées dans les publications qui précèdent.
**MIL-88B** à D : Serre et al., « Role of solvent-host interactions that lead to very large swelling of hybrid frameworks », Science, 2007, Vol. 315, 1828-1831 [ref 33].
**MIL-89** : C. Serre, F. Millange, S. Surblé, G. Férey Angew. Chem. Int. Ed. 2004, 43, 6286: A new route to the synthesis of trivalent transition metals porous carboxylates with trimeric SBU [ref 17]. La structure d'un solide MIL-89 est représentée en figure 41, et les données cristallographiques sont répertoriées dans la publication qui précède.
**MIL-100** : Horcajada et al., « Synthesis and catalytic properties of MIL-100(Fe), an iron(III) carboxylate with large pores », Chem. Comm., 2007, 2820-2822 [ref 28]. La structure d'un solide MIL-100 est représentée en figures 18 et 19, et les données cristallographiques sont répertoriées dans la publication qui précède.
**MIL-101 :** Férey et al., « A chromium terephthalate-based solid with unusally large pore volumes and surface area », Science, 2005, Vol. 309, 2040-2042 [ref 49]. La structure d'un solide MIL-101 est représentée en figure 31, et les données cristallographiques sont répertoriées dans la publication qui précède.
**MIL-102:** S. Surblé, F. Millange, C. Serre, T. Düren, M. Latroche, S. Bourrelly, P.L. Llewellyn and G. Férey « MIL-102: A Chromium Carboxylate Métal Organic Framework with Gas Sorption Analysis » J. Am. Chem. Soc. 128 (2006), 46, 14890 [ref 50]. La structure d'un solide MIL-102 est représentée en figure 32, et les données cristallographiques sont répertoriées dans la publication qui précède.
**MIL-88B_4CH3, MIL-88B-2OH:** Pour ce type structural, le lecteur pourra se référer aux publications concernant le type MIL-88 ci-dessus, à savoir, (a) Serre et al., « Role of solvent-host interactions that lead to very large swelling of hybrid frameworks », Science, 2007, Vol. 315, 1828-1831 [ref 33]; (b) Surblé et al., « A new isoreticular class of metal-organic frameworks with the MIL-88 topology », Chem. Comm., 2006, 284-286 [ref 47]; (c) Mellot-Draznieks et al., « Very large swelling in hybrid frameworks : a combined computational and power diffraction study », J. Am. Chem. Soc., 2005, Vol. 127, 16273-16278 [ref 48]. La structure d'un solide MIL-88B_4CH₃ est représentée en figure 33, et les données cristallographiques sont répertoriées dans les publications qui précèdent.
**MIL-127:** Y. Liu et al, Angew. Chem. Int. Ed. 2007, 46, 3278-3283 [ref 51]. Les données cristallographiques de ce matériau sont répertoriées dans la publication précitée.
**PIZA-1** : « A functional zeolite analogue assembled from metalloporphyrins », M.E. Kosal, J-H Chou, S. R. Wilson, K. S. Suslick, Nature, 2002, 1, 118-121 [ref 52]. Les données cristallographiques de ce matériau sont répertoriées dans la publication précitée.
**CPO-27:** « Hydrogen adsorption in a nickel based coordination polymer with open metal sites in the cylindrical cavities of the desolvated framework », P.D.C. Dietzel, B. Panella, M. Hirscher, R. Blom, H. Fjellvag, Chem. Commun., 2006, 959-961 [ref 53]. Les données cristallographiques de ce matériau sont répertoriées dans la publication précitée.

Ainsi, les inventeurs ont démontré que les matériaux MOF ayant une structure tridimensionnelle de motifs de formule (I) peuvent exister sous des phases différentes, selon leur composition et/ou leur mode de préparation. On citera par exemple, les matériaux MOF dont le motif de formule (I) répond à l'une des formules suivantes :
- M₃OX[C₆(CH₃)₄(CO₂)₂]₃, par exemple MIL-88B_4CH3
- M₃OX[C₆H₃(CO₂)₃]₂ par exemple MIL-100
- M₃OX[C₆H₄(CO₂)₂]₃ par exemple MIL-101
- M₆O₂X₂[C₁₀H₂(CO₂)₄]₃ par exemple MIL-102 ou MIL-126
- Fe₆O₂[C₁₂H₆N₂-(CO₂)₄]₃.X₂.nH₂O par exemple MIL-127
- [CoT(*p*-CO2)PPCol.5] par exemple PIZA-1
- Fe₂(₂OC-C₆H₂(OH)₂-CO₂)(H₂O).xH₂O par exemple CPO-27
dans lesquelles X est tel que défini précédemment.

En outre, les inventeurs ont mis en évidence que des matériaux MOF ayant des motifs de même formule générale (I), mais ayant des structures différentes, peuvent être obtenus à partir d'un même ligand acide carboxylique L et des mêmes bases de métal (trimères). C'est par exemple le cas des matériaux MOF MIL-88B_4CH₃ et MIL-101. En effet, la différence des solides MIL-88Btetra (i.e., MIL-88B_4CH₃) et MIL-101 réside dans le mode de connexions des ligands aux trimères d'octaèdre : dans le solide MIL-101, les ligands L s'assemblent sous forme de tétraèdres rigides, tandis que dans le solide MIL-88B, ils forment des bipyramides trigonales, rendant possible l'écartement entre les trimères.

Le mode d'assemblage de ces ligands peut être contrôlé lors de la synthèse par exemple par l'ajustement du pH. Par exemple, le solide MIL-88 est obtenu en milieu moins acide que le solide MIL-101 comme décrit dans la partie « Exemples » ci-dessous.

Avantageusement, un matériau MOF de structure rigide est utilisé, afin d'obtenir de meilleures propriétés de diffusivité dans le solide lors de la séparation.

Ainsi, dans un mode de réalisation particulier, le solide MOF comprend une succession de motifs de formule (I) suivantes :
- M₃OX[C₆H₃(CO₂)₃]2 (structure de type MIL-100)
- M₃OX[C₆H₄(CO)₂]₃ (structure de type MIL-101)
- M₃OX[C₆(CH₃)₄(CO₂)₂]₃ (structure de type MIL-88B_4CH3)
- M₆O₂X₂[C₁₀H₂(CO₂)₄]₃ (structure de type MIL-102)
- Fe₆O₂[C₁₂H₆N₂-(CO₂)₄]₃.X₂.nH₂O (structure de type MIL-127)

Dans un mode de réalisation particulier, dans les structures ci-dessus, M représente Fe.

Les méthodes de préparation de matériaux MOF sont bien connus de l'homme du métier, et ne seront pas développées dans le cadre de la présente demande. On notera simplement que, de façon générale, les matériaux MOF peuvent être préparés par un procédé comprenant au moins une étape réactionnelle (i) consistant à mélanger dans un solvant polaire :
- au moins une solution comprenant au moins un précurseur inorganique métallique se présentant sous la forme de métal M, d'un sel métallique de M ou d'un complexe de coordination comprenant un ion métallique de M
- au moins un ligand L' comprenant un radical R comportant q groupements *-C(=O)-R³, où
   ● M, q et R sont tels que définis ci-dessus,
   ● * désigne le point d'attachement du groupement avec le radical R,
R³ est choisi dans le groupe comprenant un OH, un OY, avec Y est un cation alcalin, un halogène, un radical -OR⁴, -O-C(=O)R⁴, -NR⁴R^{4'}, où R⁴ et R^{4'} sont des radicaux alkyles en C₁₋₁₂.

La préparation de matériaux MOF peut être de préférence réalisée en présence d'énergie qui peut être apportée par exemple par le chauffage, comme par exemple des conditions hydrothermales ou solvothermales, mais également par micro-ondes, par ultrasons, par broyage, par un procédé faisant intervenir un fluide supercritique, etc. Les protocoles correspondants sont ceux connus de l'homme du métier. Des exemples non limitatifs de protocoles utilisables pour les conditions hydrothermales ou solvothermales sont décrits par exemple dans [ref. 18]. Pour la synthèse par voie micro-ondes, des exemples non limitatifs de protocoles utilisables sont décrits par exemple dans [ref. 19, 20, 21, 22] Les conditions en présence d'un broyeur à cylindre peuvent par exemple se référer aux publications [ref. 23, 24, 25].

Les conditions hydrothermales ou solvothermales, dont les températures de réactions peuvent varier entre 0 et 220°C, sont généralement effectuées dans des récipients en verre (ou en plastique) lorsque la température est inférieure à la température d'ébullition du solvant. Lorsque la température est supérieure ou lorsque la réaction s'effectue en présence de fluor, des corps en téflon insérés dans des bombes métalliques sont employés [ref. 22].

Les solvants utilisés sont généralement polaires.

Notamment les solvants suivants peuvent être utilisés : l'eau, les alcools, le diméthylformamide, le diméthylsulfoxyde, l'acétonitrile, le tétrahydrofurane, le diéthylformamide, le chloroforme, le cyclohexane, l'acétone, le cyanobenzène, le dichlorométhane, le nitrobenzène, l'éthylèneglycol, le diméthylacétamide ou des mélanges de ces solvants.

Un ou plusieurs co-solvants peuvent également être ajoutés à n'importe quelle étape de la synthèse pour une meilleure solubilisation des composés du mélange. Il peut s'agir notamment d'acides monocarboxyliques, tels que l'acide acétique, l'acide formique, l'acide benzoïque, etc.

Un ou plusieurs additifs peuvent également être ajoutés au cours de la synthèse afin de moduler le pH du mélange. Ces additifs sont choisis parmi les acides minéraux ou organiques ou les bases minérales ou organiques. À titre d'exemple, l'additif peut être choisi dans le groupe comprenant : HF, HCl, HNO₃, H₂SO₄, NaOH, KOH, la lutidine, l'éthylamine, la méthylamine, l'ammoniac, l'urée, l'EDTA, la tripropylamine, la pyridine.

De préférence, l'étape réactionelle (i) de mélange peut être réalisée suivant au moins une des conditions réactionnelles suivantes :
- avec une température de réaction de 0°C à 220°C, de préférence de 50 à 150°C ;
- avec une vitesse d'agitation de 0 à 1000 rpm (ou rotations par minute), de préférence de 0 à 500 rpm ;
- avec un temps de réaction de 1 minute à 144 heures, de préférence de 1 minute à 15 heures ;
- avec un pH de 0 à 7, de préférence de 1 à 5 ;
- avec l'addition d'au moins un co-solvant au solvant, au précurseur, au ligand ou au mélange de ceux-ci, ledit co-solvant étant choisi dans le groupe comprenant l'acide acétique, l'acide formique, l'acide benzoïque ;
- en présence d'un solvant, qui est choisi dans le groupe comprenant l'eau, les alcools R^{s}-OH où R^{s} est un radical alkyle en C₁ à C₆ linéaire ou ramifié, le diméthylformamide, diméthylsulfoxyde, l'acétonitrile, le tétrahydrofurane, le diéthylformamide, le chloroforme, le cyclohexane, l'acétone; le cyanobenzène, le dichlorométhane, le nitrobenzène, l'éthylèneglycol, le diméthylacétamide ou des mélanges de ces solvants, miscibles ou non ;
- dans un milieu supercritique, par exemple dans le CO₂ supercritique ;
- sous micro-ondes et/ou sous ultrasons ;
- dans des conditions d'électrolyse électrochimique ;
- dans des conditions utilisant un broyeur à cylindres ;
- dans un flux gazeux.

Comme indiqué précédemment, selon un aspect, l'invention se rapporte à un procédé pour séparer un mélange de molécules ayant des degrés et/ou un nombre d'insaturations différents par adsorption préférentielle des molécules à degré et/ou nombre d'insaturations supérieur sur un solide MOF cristallin poreux tel que défini ci-dessus, ledit procédé comprenant au moins une étape réactionnelle consistant
(i) à mélanger dans un solvant polaire :
   - au moins une solution comprenant au moins un précurseur inorganique métallique se présentant sous la forme de métal M, d'un sel métallique de M ou d'un complexe de coordination comprenant un ion métallique de M ;
   - au moins un ligand L' comprenant un radical R comportant q groupements *-C(=O)-R³, où
      ● M, q et R sont tels que définis ci-dessus,
      ● * désigne le point d'attachement du groupement avec le radical R,
      ● R³ est choisi dans le groupe comprenant un OH, un OY, avec Y est un cation alcalin, un halogène, un radical -OR⁴, -O-C(=O)R⁴, -NR⁴R^{4'}, où R⁴ et R^{4'} sont des radicaux alkyles en C₁₋₁₂, pour obtenir un matériau MOF ;
(ii) à activer le matériau MOF obtenu en (i) ; et
(iii) à mettre en contact le matériau MOF obtenu à l'étape (ii) avec un mélange de molécules ayant des degrés et/ou un nombre d'insaturations différents.

Ainsi, préalablement à la mise en contact du matériau MOF avec le mélange de molécules ayant des degrés et/ou un nombre d'insaturations différents dans l'étape (iii), il est nécessaire que le matériau issu de l'étape (i) soit activé dans l'étape (ii).

Dans un mode de réalisation particulier, l'étape d'activation (ii) peut avoir deux effets:
- Elle peut permettre de vider les pores du matériau MOF et de les rendre accessibles pour la coordination de la ou des molécules à séparer.
- Elle peut permettre la réduction de centres métalliques M²⁺ du matériau MOF obtenu en (ii) en ions M^{(z-1)+} dans lequel z représente 3 ou 4.

Le vidage peut se faire, par exemple, par le départ des molécules d'eau et/ou des solvants présents dans le milieu réactionnel soit par activation sous vide primaire ou secondaire ou sous flux gazeux (hélium, azote, air...), avec ou non le chauffage du solide à une température de 25 à 300°C, en particulier de 50 à 250°C, et plus particulièrement de 100 à 250°C. Le chauffage peut être effectué pendant une période de temps entre 1 heure et 96 heures, typiquement entre 3 et 15 heures. L'activation est un phénomène cinétique : plus la température d'activation est élevée, plus on déshydrate vite et plus la réduction est rapide. Sans vouloir être lié en aucune façon à une théorie particulière, il semble pouvoir être avancé que le taux de réduction maximal à une température donnée est sans doute contrôlé par la thermodynamique.

L'étape d'activation (ii) permet en particulier de réduire une partie des ions M^{z+} du matériau MOF obtenu en (ii) en ions M^{(z-1)+}. Cette étape d'activation a pour effet de rendre le matériau MOF plus sélectif vis-à-vis des - molécules comportant au moins une insaturation. Comme indiqué précédemment, l'ion métallique réduit M^{(z-1)+} possède un électron d supplémentaire par rapport à M^{z+}, ce qui renforce la liaison de coordination avec les molécules comprenant au moins insaturation et accroit ainsi la stabilité du complexe formé.

Il est entendu que tous les sites métalliques M^{z+} du matériau MOF ne sont pas réduits dans cette étape d'activation (ii). Seulement les sites métalliques accessibles sont susceptibles d'être réduits.

Par « site métallique accessible », on entend un site métallique M^{z+} ou M^{(z-1)+} qui peut se coordinner avec des molécules.

Selon les conditions d'activation (ii), les centres métalliques accessibles du matériau MOF peuvent être partiellement voire totalement réduits. Par « partiellement réduits », on entend la réduction de moins de 100% des sites métalliques accessibles M^{z+} en ions correspondants M^{(z-1)+}. Selon un mode de réalisation particulier, la fraction d'ions réduits M^{(z-1)+} par rapport aux ions métalliques (y=M^{(z-1)+}/M^{z+}) est strictement supérieure à zéro.

Dans un mode de réalisation, le procédé selon l'invention peut comprendre en outre une étape (i') de traitement du matériau MOF obtenu à l'étape (i) avec une source d'ions F⁻, comme par exemple, KF, CsF, LiF, MgF₂, CaF₂, ou un sel fluoré d'amine comme NH₄F. Avantageusement, la source d'ions F⁻ est NH₄F, par exemple en solution aqueuse. Le matériau ainsi obtenu peut ensuite être soumis aux étapes (ii) et (iii) telles que définies précédemment. Dans ce cas, la température utilisée dans l'étape d'activation (ii) sera généralement inférieure à celle qui est utilisée lorque le matériau n'est pas soumis au traitement avec une source d'ions F⁻.

Selon un mode de réalisation, M peut représenter Fe et pas plus d'un tiers des sites métalliques Fe³⁺ sont réduits en ions Fe²⁺. Il s'agit généralement de solides MOF ne comprenant pas de ligands redox (dont l'oxydation est susceptible de conduire à une compensation de charge permettant d'obtenir des valeurs de x supérieures à 1/3). Dans ce cas de figure, y=1/3 correspond au maximum théorique d'activation du matériau MOF par réduction des sites Fe³⁺ par le phénomène d'auto-réduction (i.e., dans un premier temps, une température <150°C, voire <100°C, permet de retirer les molécules d'eau et de solvant coordinées sur les sites M^{z+}. Au-dessus de 100°C, en particuler au-dessu de 150°C, les sites M^{z+} commencent à être réduits. La réduction peut s'accompagner de l'élimination d'un ligand chargé négativement (-1) pour préserver l'électroneutralité du solide MOF). En d'autres termes, selon un mode particulier de l'invention, M=Fe et le rapport y=M^{(z-1)+}/M^{z+} peut être inférieur ou égal à 1/3. Ainsi, dans un mode de réalisation particulier, M peut représenter Fe et le rapport y=M^{(z-1)+}/M^{z+} peut être compris entre 0<y≤1/3.

Selon un autre mode de réalisation, M peut représenter Fe, le solide MOF poreux contient au moins un ligand redox susceptible d'être oxydé et y peut prendre toute valeur comprise entre 1/3 et 1. Par exemple, il peut s'agir d'un ligand de type hydroquinone (forme réduite) ou quinone (forme oxydée). On pourra citer par exemple le ligand acide 2,5-dihydroxotéréphthalique. Un exemple de solide MOF de ce type est le carboxylate de fer MIL-88B-2OH(Fe), Fe₃O[C₆H₂ (OH)₂-(CO₂)₂]₃.X.nH2O (X=F, Cl, OH). Voir l'exemple 31 pour un exemple de réalisation.

Les centres métalliques d'un même matériau MOF peuvent provenir de métaux identiques ou différents, par exemple uniquement le fer, ou de plusieurs métaux, tels que le fer en présence de manganèse et/ou de cobalt.

Lorsque les centres métalliques accessibles sont en partie réduits, en particulier si une partie du fer est au degré d'oxydation +II (z=3), une partie du manganèse au degré d'oxydation +II (z=3) et/ou une partie du cobalt au degré d'oxydation +II (z=3) et/ou une partie du vanadium IV a été réduit au degré d'oxydation +III (z=4), une partie des molécules du mélange peuvent alors se coordiner plus fortement avec le métal par effet de rétrodonation.

Par rétrodonation, on entend le transfert de la densité électronique du métal M dans une orbitale antiliante de la molécule comprenant a moins un insaturation. Cela conduit à une augmentation du nombre de molécules comprenant au moins une insaturation coordinées par centre métallique. Le solide MOF final comportera alors une plus grande quantité de molécules coordinées aux ions métalliques réduits. Les solides MOF résultants possèderont alors une plus grande sélectivité dans la séparation de mélanges de molécules ayant des degrés et/ou un nombre d'insaturations différents. La présence des ions réduits M^{(z-1)+} permet notamment d'augmenter l'affinité du solide MOF par rapport aux molécules à degré et/ou nombre d'insaturations supérieur dans un mélange donné (par exemple, des molécules comprenant deux double liaisons seront préférentiellement adsorbées sur le solide MOF que celles ayant une double liaison).

L'étape de réduction (ii) permet ainsi d'obtenir un matériau MOF susceptible d'être utilisé pour la séparation différentielle de molécules ayant des degrés et/ou un nombre d'insaturations différents, les molécules ayant comparativement des degrés et/ou un nombre d'insaturations supérieurs séjourneront plus longtemps dans le matériau (dû à une affinité plus grande pour les ions métalliques réduits M^{(z-1)+} pour les molécules comportant des insaturations),

Par ailleurs, le taux de centres métalliques réduits a une influence sur la durée du séjour des molécules du mélange dans le solide MOF, celle-ci augmentant de manière conséquente. Plus la teneur du solide MOF en ions réduits est grande, plus la durée de séjour dans le solide MOF des molécules à degré et/ou nombre d'insaturations supérieur sera grand.

L'étape d'activation (ii) peut être effectuée par tout moyen permettant de déhydrater le matériau MOF (vidage des molécules d'eau coordinnées sur les sites M^{z+}) et de conduire à la réduction d'au moins une partie des sites accessibles M^{z+} en ions M^{(z-1)+}.

Dans un mode de réalisation, l'étape d'activation (ii) peut être effectuée à une température élevée sous atmosphère neutre ou réductrice, ou sous pression réduite.

Dans un mode de réalisation, l'étape d'activation (ii) peut être effectuée à une température élevée sous flux de gaz inerte, tel que l'hélium, l'argon, l'azote, ou un mélange de ceux-ci. Par exemple, l'étape d'activation (ii) peut se faire entre 25 et 400°C, plus particulièrement entre 100 et 300°C, tout particulièrement entre 150 et 280°C, sous flux d'hélium.

Dans un mode de réalisation, l'étape d'activation (ii) peut être effectuée à une température élevée sous atmosphère réductrice, telle que H₂, CO ou NO. Par exemple, l'étape d'activation (ii) peut se faire entre 25 et 400°C, plus particulièrement entre 100 et 300°C, tout particulièrement entre 150 et 280°C, sous atmosphère d'hydrogène.

Dans un mode de réalisation particulier, l'étape d'activation (ii) peut être réalisée à une température élevée et sous pression réduite. Par pression réduite, on entend une pression allant de 1 à 10⁻⁵ Pa, avantageusement de 1 à 10⁻² Pa voire de 10⁻³ à 10⁻⁵ Pa.

Par exemple, l'étape d'activation (ii) peut se faire à 50-250°C sous une pression de 1 à 10⁻² Pa, ou de 10⁻³ à 10⁻⁵ Pa.

Il est entendu que les conditions de températures employées dépendront du matériau MOF utilisé. Celles-ci pourront également dépendre du fait qu'un éventuel prétraitement du matériau avec avec une source d'ions F⁻ (telle que KF, CsF, LiF, MgF₂, CaF₂, ou un sel fluoré d'amine comme NH₄F) aura été effectué ou pas. Par exemple, il peut s'agir d'un traitement du matériau MOF avec une solution aqueuse de NH₄F à 70°C pendant 24 heures.

En général, les conditions de température et de pression mentionnés ci-dessus peuvent également être utilisées lorsque le solide MOF poreux a été soumis au préalable à un traitement avec une source d'ions F- (telle que KF, CsF, LiF, MgF₂, CaF₂, ou un sel fluoré d'amine comme NH₄F). Ainsi, pour un MOF préalablement traité avec une source d'ions F⁻, l'étape d'activation (ii) peut se faire à 50-250°C sous une pression de 1 à 10⁻² Pa, ou de 10⁻³ à 10⁻⁵ Pa.

Avantageusement, la température dans l'étape d'activation (ii) n'excédera pas la température de dégradation du solide MOF. Dans un mode de réalisation particulier, l'étape (ii) d'activation est réalisée à une température de 25 à 300°C, avantageusement de 50 à 250°C. Du point de vue industriel le choix des conditions d'activation pourra se faire selon des considérations pratiques (coût, disponibilité du gaz choisi etc...).

Une fois l'étape d'activation (ii) effectuée, le solide MOF partiellement réduit peut être stocké sous vide ou sous atmosphère inerte pour le protéger d'une réoxydaton éventuelle. Alternativement, le solide MOF partiellement réduit obtenu à l'issue de l'étape (ii) peut être utilisé directement pour l'étape (iii) du procédé.

Dans l'étape (iii) du procédé de l'invention, le matériau MOF activé dans l'étape (ii) est mis en contact avec un mélange de molécules ayant des degrés et/ou un nombre d'insaturations différents. Le mélange peut être sous forme gazeuse ou en phase liquide.

Lorsque le mélange est sous forme gazeuse, celui-ci peut être à séparer en tant que tel, ou en mélange avec un gaz inerte (le gaz inerte pouvant servir de gaz véhicule (« carrier gas »).

Lorque le mélange est en phase liquide, le mélange peut être solvaté dans un solvant ou mélange de solvants. De préférence, le(s) solvant(s) ne contiennent pas d'insaturations au sens de la présente invention afin que le contact avec le solvant ne conduise pas à un empoisonnement des sites insaturés du solide MOF. Par exemple, il peut s'agir d'hydrocarbures saturés.

La mise en contact du solide MOF avec le mélange de molécules dans l'étape (iii) peut être réalisée à une température allant de -150 à 200 °C.

Par ailleurs, l'étape (iii) peut être réalisée à une pression allant de 10⁴ à 10⁷ Pa.

L'homme du métier saura sélectionner les gammes de température et de pression pour effectuer la séparation d'un mélange donné, en fonction de la composition du mélange, de la nature du solide MOF (solide MOF à base de Fe, Mn, Co, Ni, V ou mixte), de la teneur en ions M^{(z-1)+}, des conditions de séparation (phase gazeuse ou liquide, séparation thermodynamique ou sous flux dynamique), et du profile de séparation souhaité (temps de séparation, résolution). En général, le choix des conditions de température et de pression dépendra du procédé de séparation utilisé (par exemple, Fractionnement d'air par adsorption modulée pression (« Pressure Swing Adsorption ») ou lit mobile simulé (« Simulated Moving Bed ») et des coûts de mise en oeuvre du procédé. Dans le choix des conditions de température et de pression, l'homme du métier sera guidé par des considérations relevant des compromis performances-coûts énergétiques.

A titre d'exemple, des conditions de température et de pression sont données ci-dessous pour quelques mélanges particuliers.

Dans un mode de réalisation particulier, le mélange de molécules est un mélange d'au moins un hydrocarbure saturé et au moins un hydrocarbure insaturé. Il peut s'agir par exemple, d'un mélange d'au moins une paraffine (alcane) et au moins une oléfine (alcène). Il peut s'agir également d'un mélange d'au moins une oléfine et au moins un alcyne. Par exemple, il peut s'agir de mélanges d'hydrocarbures en C2, C3, C4 ou C5. A titre d'exemple, on peut citer:
● propane/propylène,
● n-butane/isobutène,
● n-butane/mélange de butènes,
● acétylène/éthylène

### propane/propylène

La séparation de mélange propane-propylène est d'une importance industrielle considérable dans l'industrie chimique et pétrochimique. Depuis près de 60 ans, la séparation propane/propylène est réalisée par un procédé très couteux sur le plan énergétique de distillation cryogénique à -30°C (243 K) et 30 psig (0.308 MPa) dans une colonne contenant près de 100 plateaux. La raison de ces conditions extrêmes vient des points d'ébullition très proches de ces deux hydrocarbures.

Le propylène est l'un des précurseurs de base d'un grand nombre de polymères. Sa récupération dans les flux issus des raffineries est utilisée de façon intensive pour répondre aux besoins des fabricants industriels de polymères à base de propylène. Pour répondre aux spécifications correspondantes, le flux industriel doit contenir au minimum 99.5 % de propylène et être libre de traces de di-oléfines et d'acétylène.

### butane/isobutène

L'isobutène est un réactant pour la polymérisation. L'isobutène doit avoir un niveau de pureté supérieur à 99% et ne plus contenir que des traces de butène-1 et de butènes-2 (quelques centaines de parties par million en poids, ppm). En effet, si le taux d'impureté dans l'isobutène est trop élevé, les polymères obtenus sont de moins bonne qualité et le rendement de la polymérisation est plus faible. Il est donc nécessaire d'éliminer d'une coupe d'hydrocarbures contenant de l'isobutène les autres hydrocarbures oléfiniques.

La difficulté actuelle est que le butène- 1 et l'isobutène ayant des points d'ébullition très voisins, il n'est pas possible de les séparer par distillation economiquement.

Le principal problème qui se pose pour produire de l'isobutène de haute pureté est donc la séparation du butène-1 de l'isobutène (Voir WO 98/006684 [ref. 45]).

### acétylène/éthylène

L'acétylène est un produit secondaire obtenu lors de la production de l'éthylène, et agit comme un poison pour les catalyseurs utilisés pour fabriquer le polyéthylène à partir de l'éthylène. Par conséquent, le polymère à base d'éthylène ne doit pas contenir plus de 5 ppm d'acétylène. Retirer ces traces d'acétylène est donc un véritable défi pour les fabriquants d'éthylène et les fournisseurs de catalyseurs à cause des faibles concentrations en acétylène dans les réacteurs et leurs conversion presque totales dans ces conditions.

De plus, l'acétylène peut former des acétylures métalliques qui sont des contaminants explosifs. Il est donc impératif de réduire la concentration en acétylène dans le flux d'éthylène à un degré acceptable. (Pour des informations complémentaires, le lecteur pourra se référer à http://kolmetz.com/Article-039.htm)

Une utilisation particulièrement importante des matériaux MOF de l'invention concerne la séparation des oléfines et des paraffines. Comme évoqué précédemment, le recouvrement des oléfines à partir de mélanges d'oléfines et de paraffines constitue l'une des problématiques les plus importantes dans le domaine de la séparation. Citons par exemple l'éthylène et le propylène qui sont des précurseurs fondamentaux pour la synthèse de nombreux polymères tels que le polyéthylène ou le polypropylène. Les technologies de séparation actuelles sont très coûteuses et possèdent de nombreux inconvénients. Compte tenu de leur facilité de mise en oeuvre, de leur coût relativement bas, et des propriétés structurales et électroniqes exposés dans la présente, les matériaux MOF de l'invention constituent une alternative appréciable pour la séparation oléfines/paraffines à l'échelle industrielle.

Ainsi, dans un mode de réalisation particulier, le mélange à séparer est un mélange de propane et de propène. Dans ce mode de réalisation, l'étape (iii) peut être réalisée à une température de 0° C à +150°C. Dans ce mode de réalisation, l'étape (iii) peut être réalisée à une pression de 10⁴ à 10⁶ Pa. Dans ce mode de réalisation, le matériau MOF obtenu à l'étape (ii) a avantageusement une capacité d'adsorption du propane et/ou du propène comprise entre 0.5 et 20 mmol/g.

Dans un mode de réalisation particulier, le mélange à séparer est un mélange d'éthane et d'éthylène. Dans ce mode de réalisation, l'étape (iii) peut être réalisée à une température de 0° C à +150°C. Dans ce mode de réalisation, l'étape (iii) peut être réalisée à une pression de 10⁴ à 10⁶ Pa. Dans ce mode de réalisation, le matériau MOF obtenu à l'étape (ii) a avantageusement une capacité d'adsorption de l'éthane et/ou de l'éthylène comprise entre 0.5 et 20 mmol/g.

Dans un mode de réalisation particulier, le mélange à séparer est un mélange d'isobutane et d'isobutène. Dans ce mode de réalisation, l'étape (iii) peut être réalisée à une température de 0° C à +150°C. Dans ce mode de réalisation, l'étape (iii) peut être réalisée à une pression de 10⁴ à 10⁶ Pa. Dans ce mode de réalisation, le matériau MOF obtenu à l'étape (ii) a avantageusement une capacité d'adsorption de l'isobutane et/ou de l'isobutène comprise entre 0.5 et 20 mmol/g.

Dans un mode de réalisation particulier, le mélange de molécules est un mélange d'au moins un composé comprenant un ou plusieurs doublet(s) libre(s) et d'au moins un composé ne comprenant pas de doublet libre. Dans un mode de réalisation particulier, le mélange de molécules est un mélange d'au moins un hydrocarbure saturé et au moins un hydrocarbure insaturé ou de dioxyde de carbone. Par exemple, il peut s'agir d'un mélange d'acétylène et de dioxyde de carbone, ou encore d'un mélange d'hydrogène et de monoxyde et/ou dioxyde de carbone.

Il peut s'agir également d'un mélange de propane et de propène, un mélange de n-butane et d'isobutène, un mélange de n-butane et de butènes, un mélange d'acétylène et d'éthylène, un mélange d'acétylène et de dioxide de carbone, un mélange de CO et d'hydrogène, un mélange de CO₂ et d'hydrogène, un mélange contenant de l'hydrogène et au moins un hydrocarbure insaturé et/ou d'au moins un composé comprenant un ou plusieurs doublet(s) libre(s), un mélange d'hydrogène et d'autres gaz. (oléfines, alcynes, monoxyde de carbone, dioxyde de carbone, ...).

Dans un mode de réalisation particulier, le mélange de molécules est choisi parmi :
- un mélange d'au moins un hydrocarbure saturé et au moins un hydrocarbure insaturé,
- un mélange d'au moins un composé comprenant un ou plusieurs doublet(s) libre(s) et d'au moins un composé ne comprenant pas de doublet libre, et
- un mélange d'hydrogène et de monoxyde et/ou dioxyde de carbone.

Dans un mode de réalisation particulier, le mélange de molécules est choisi parmi :
- un mélange d'au moins une paraffine et au moins une oléfine.
- un mélange d'au mois une oléfine et un alcyne.
- un mélange d'acétylène et de dioxyde de carbone.
- un mélange d'hydrogène et de monoxyde et/ou dioxyde de carbone.

En effet, une utilisation particulièrement intéressante des matériaux MOF de la présente invention est la purification de l'hydrogène par élimination des traces de CO et/ou CO₂. Actuellement, la demande en hydrogène est très importante pour des applications à pile à combustible, cette technologie étant considérée comme étant l'une des plus prometteuses pour répondre aux besoins énergétiques du futur. [ref. 26] L'hydrogène est généralement produit par vaporéformage à partir d'un gaz naturel ou d'autres combustibles fossiles. Le reformage est un procédé thermochimique, par lequel les combustibles contenant de l'hydrogène sont transformés en un mélange gazeux riche en H₂.

La réaction de vaporéformage s'effectue généralement à haute temperature de 400 à 700°C sur des catalyseurs à base de nickel déposé sur un support d'alumine, et s'accompagne de la formation de monoxyde de carbone. Généralement, le vaporéformage est suivi d'une reaction en présence d'eau (réaction de gaz à eau) afin d'oxyder le monoxyde de carbone en dioxyde de carbone.

Pour les besoins de pile à combustible, il est nécessaire de purifier l'hydrogène produit industriellement par les procédés précités. Les méthodes de purification actuellement utilisées reposent sur des membranes à mousse de platine, qui sont extrêmement coûteuses et de plus en plus rares. Les matériaux MOF de la présente invention peuvent être utilisés à cet effet, à savoir pour séparer les traces de CO et CO₂ de l'hydrogène. En effet, CO et CO₂ peuvent être séparés de l'hydrogène, qui ne contient aucune insaturation, par le procédé de l'invention.

Ainsi, la présente invention s'étend à l'utilisation des solides MOF et du procédé selon l'invention à la purification de l'hydrogène. Selon un aspect, la présente concerne donc un procédé pour purifier l'hydrogène. Ainsi, dans un mode de réalisation, le mélange de molécules à l'étape (iii) du procédé de l'invention peut être un mélange de H₂, CO et CO₂.

Une autre utilisation- des matériaux MOF de la présente invention présentant un intérêt industriel important est la séparation de l'acétylène du dioxyde de carbone. L'acétylène est un gaz d'instrumentation employé en tant que gaz de combustion dans la spectrométrie d'absorption atomique pour l'analyse élémentaire et dans la spectrométrie optique.

Il s'agit d'un gaz synthétique issu de la réaction entre carbure de calcium et eau :

CaC₂ + 2 H₂O → C₂H₂ + Ca(OH)₂

Il peut également être obtenu par combustion partielle du méthane :

3 CH₄ + 3 O₂ → C₂H₂ + CO + 5 H₂O

L'acétylène et le dioxyde de carbone, qui sont présents le plus souvent dans les procédés de récupération du CO₂, ont des caractéristiques très similaires (taille), leur séparation est donc difficile par distillation (cryogénique ou non). Pour plus de détails sur la problématique de récupération de CO₂, le lecteur pourra se référer par exemple au brevet US 6,024,781 [ref. 44].

Ainsi, la présente invention s'étend à l'utilisation des solides MOF et du procédé selon l'invention à la séparation de l'acétylène du dioxyde de carbone. Selon un aspect, la présente concerne donc un procédé pour séparer l'acétylène du dioxyde de carbone. Ainsi, dans un mode de réalisation, le mélange de molécules à l'étape (iii) du procédé de l'invention peut être un mélange d'acétylène et de dioxyde de carbone.

L'invention concerne également l'utilisation des matériaux MOF tels que définis précédemment pour :
- la purification de l'air (notamment pour réduire le taux de, voire éliminer les traces de, CO₂, H₂O, N₂O et d'huile dans les flux d'air),
- la séparation de mélanges de CO₂ et d'hydrocarbures (par exemple des mélanges CO₂/ethylène),
- piéger les traces d'eau dans les mélanges gazeux (utilisation en tant qu'agent dessiccant).

Dans un mode de réalisation particulier, le procédé de l'invention peut comprendre en outre une étape (iv) de désorption des molécules à degré et/ou nombre d'insaturations supérieur sur le solide MOF cristallin poreux de l'invention. La désorption peut se faire par tout moyen connu dans le domaine des séparations sur phase solide. Par exemple, la désorption peut être effectuée par une technique de désorption choisie parmi le déplacement avec un autre gaz, un changement de pression, un changement de température, ou une combinaison de celles-ci. Par exemple, la désorption peut s'opérer par déplacement avec un gaz pur ou essentiellement pur (par exemple au minimum 98% pur) à basse pression, entre 1 et 8 bars par exemple. La désorption peut également être effectuée par la combinaison d'un changement de température et un changement de pression. L'homme du métier saura sélectionner la combinaison température/pression en fonction du procédé utilisé et du mélange à séparer.

Ainsi, dans un aspect, la présente invention repose sur l'activation de matériaux MOF carboxylates métalliques poreux possédant des centres métalliques réductibles (Fe, Mn, Ni, V et/ou Co) insaturés afin d'améliorer leur propriétés de séparation de molécules possédant au moins une insaturation (par exemple les mélanges paraffine/oléfine).

Il s'agit en pratique de réduire partiellement le centre métallique au préalable par activation sous vide (ou sous atmosphère réductible ou neutre) afin d'augmenter l'affinité du métal, par effet de rétrodonation, pour les molécules d'un mélange donné ayant un degré et/ou nombre d'insaturations supérieur (par exemple les alcènes (ou alcynes)) par rapport aux molécules du même mélange ayant un degré et/ou nombre d'insaturations inférieur (par exemple les alcanes).

Dans un mode de réalisation particulier, les matériaux MOF en question sont des carboxylates de fer(III) poreux, de structure rigide, à base de trimères d'octaèdres qui possèdent une quantité importante de centres métalliques insaturés (1-3 mmol.g⁻¹). L'activation permet d'accéder à une réduction jusqu'à 33 % du fer initialement présent sous forme de fer(III).

Les matériaux MOF de la présente invention permettent ainsi la séparation desdits mélanges de molécules ayant des degrés et/ou nombres d'insaturations différents (par exemple, des mélanges alcènes/alcanes (ou alcynes/alcènes)) sous flux dynamique en pression. La séparation est basée sur les différences d'affinité entre les molécules du mélange à séparer et le matériau MOF, notamment les sites métalliques insaturés M^{(z-1)+} qui ont une forte affinité pour les insaturations.

Une propriété unique des matériaux MOF de l'invention est que la modulation du taux d'ions réduits M^{(z-1)+} dans le matériau MOF par une activation préalable soigneusement contrôlée (par exemple, temps et/ou température d'activation données) permet également d'optimiser et/ou d'adapter les performances du matériau MOF (sélectivité, régénération, diffusion) en fonction du procédé industriel choisi, notamment en fonction du mélange de molécules à séparer.

Un avantage des matériaux MOF de la présente invention est qu'ils peuvent être obtenus sous forme cristalline pure, et homogène, en un nombre réduit d'étapes et avec des rendements élevés. Ceci réduit la durée de synthèse et les coûts de fabrication.

D'autre part, le matériaux MOF de la présente invention se distinguent par le fait fait que la structure du matériau MOF demeure stable après réduction d'ions métalliques M^{z+} du matériau en ions M^{(z-1)+}. Ceci n'est pas le cas des matériaux MOF à base de Cu(II) étudiés à ce jour dans le contexte de la purification des oléfines [ref. 14 et 16]. En effet, dans ces MOF de l'art antérieur, la réduction du Cu(II) en Cu(I) entraînerait la destruction de la structure (framework) du complexe. A l'inverse, les inventeur ont démontré les matériaux MOF de l'invention conservent leur structure intègre lorsque tout ou partie des ions accessibles M^{z+} du matériau MOF sont réduits en ions M^{(z-1)+} correspondants. Cette propriété est mise en évidence dans la partie « Exemples » et procure aux matériaux MOF de l'invention des propriétés spécifiques particulièrement adaptées à la séparation de mélanges de molécules ayant des degrés et/ou un nombre d'insaturations différents.

Par ailleurs, les inventeurs ont mis en évidence que la teneur en ions réduits M^{(z-1)+} pouvait être modulée en fonction des conditions de température et/ou temps d'activation utilisées dans l'étape d'activation (ii) du procédé de l'invention (voir la partie « Exemples », notamment l'exemple 5). Ainsi, des teneurs plus élevées en ions réduits M^{(z-1)+} peuvent être obtenues à des gammes de température plus élevées et/ou des temps d'activation plus longs.

Les inventeurs ont également démontré que cette teneur en ions réduits M^{(z-1)+} influençait la sélectivité du matériau MOF dans la séparation de mélanges contenant des molécules possédant un caractère donneur d'électrons (base de Lewis). Plus la teneur en ions réduits M^{(z-1)+} est importante, plus le matériau MOF a d'affinité pour les bases de Lewis, et par conséquent plus l'adsorption de molécules à insaturations est importante.

Ainsi, un avantage important des matériaux MOF de l'invention est que la teneur en ions réduits M^{(z-1)+} peut facilement être modulée/ajustée en fonction du mélange de molécules à séparer. En particulier, celle-ci est un paramètre utile et facilement exploitable pour moduler la sélectivité du matériau MOF dans la séparation de mélanges de molécules ayant des degrés et/ou un nombre d'insaturations différents.

En outre, les sites métalliques réduits M^{(z-1)+} faisant partie intégrale de la charpente du matériau MOF, il n'y a pas l'effet de lavage ou de « lixiviation » qui peut être observé pour certains matériaux utilisés dans les méthodes de séparations des oléfines (par exemple les zéolithes à constituant d'argent ou de cuivre (« Agzeolithes » ou « Cu-zeolithes ») obtenues par impregnation). Le terme « lixiviation » est généralement utilisé pour désigner un lavage de dépôt solides pour en extraire les composés solubles. Dans contexte de la présente invention, on entend par « lixiviation » le phénomène de lavage, ou de perte de constituants du matériau de séparation, qui affecte parfois les procédés de séparation.

Ainsi, non seulement la structure (charpente) du matériau MOF de l'invention demeure intègre après réduction de sites métalliques accessibles M^{z+}, mais aussi les sites métalliques réduits M^{(z-1)+} ne sont pas perdus/lavés lorsque le procédé de séparation est mis en oeuvre. Cela évite donc les coûts économiques dus à la déterioration des matériaux et prévient aussi des éventuels phénomènes de pollution ambiante.

D'autre part, comme indiqué précédemment, la température requise pour activer les matériaux MOF selon l'étape (ii) du procédé de l'invention est relativement basse (de 25 à 300°C, plus particulièrement de 100 à 250°C) par rapport à la température d'activation des zéolithes utilisées dans ce domaine (autour de 400°C). Le procédé de l'invention présente donc un avantage économique (coûts de mise en oeuvre moins élévés).

Un autre avantage des matériaux MOF de l'invention est également la possibilité de mettre en forme directement des monolithes d'adsorbants, étape nécessaire avant d'envisager la formation de membranes, afin d'éviter les problèmes classiques liés à la mise en forme : endommagement de la structure poreuse, résistance de transfert de masse intracristallin. [ref. 27]

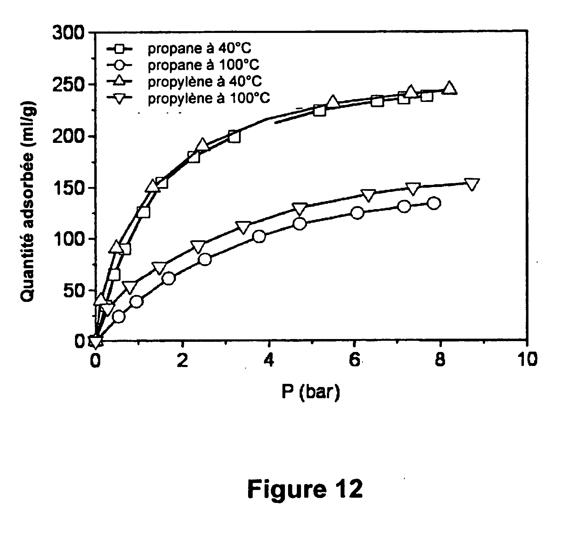

Les inventeurs ont par ailleurs mis en évidence que les matériaux MOF de l'invention avaient une capacité d'adsorption supérieure aux zéolithes. On notera par exemple que la capacité d'adsorption du matériau MOF MIL-100(Fe) de formule Fe₃O[C₆H₃-(CO₂)₃]₂.X.nH₂O (X=F, Cl, OH) pour le propane et/ou propylène est d'environ 11 mmol/g (soit près de 172 cm³/cm³ STP (« Standard Temperature and Pressure »)) ou de solide sec (figure 12). Ceci est très supérieur à la capacité d'adsorption d'une zéolithe qui est de 1 à 3 mmol/g.

D'autre part, un désavantage notoire des zéolithes est que celles-ci jouissent d'une faible diffusivité des hydrocarbures dans leur pore, due en partie à la taille des pores trop réduite des zéolithes. Au contraire, il est attendu que la diffusivité des hydrocarbures dans les matériaux MOF de la présente invention sera bien meilleure. Il est bien connu que les zéolithes possèdent des problèmes de diffusivité car les pores sont plus petits [réf. 11].

Citons par exemple, le cas du solide MIL-100(Fe). [ref. 28] Celui-ci est constitué de trimères d'octaèdres de fer relié par des acides trimésiques qui s'associent pour former des supertétraèdres hybrides. Le tout conduit ainsi à une structure mésoporeuse cristallisée, dont les cages de dimensions libres 25 et 29 Å, sont accessibles par des fenêtres microporeuses (figure 18). Le volume poreux résultant est très important, proche de 1.2 g.cm⁻³ pour une surface spécifique BET de 2200 m².g⁻¹. Ainsi, l'évacuation des molécules d'eau ou de fluor coordinnées sur le fer, donne lieu à des tailles de fenêtres d'accès aux cages mésoporeuses de grande dimension (25/29 Å): 8.5 Å pour les fenêtres pentagonales et 12 Å pour les fenêtres hexagonales (figure 19), soit une taille supérieure à celle de la zéolithe de grande taille Faujasite (fenêtres de 7 Å et cages de 12 Å). Cela, combiné à la présence de cages mésoporeuses, la meilleure diffusion des hydrocarbures (notamment le propane et le propylène) dans les pores du MIL-100(Fe) par rapport à ceux de petite taille des zéolithes. La séparation de ces oléfines dans un solide à si grande taille de pores s'explique sans doute par la présence des centres métalliques insaturés M^{(z-1)+} dans les fenêtres, ces derniers « filtrant » les molécules avant qu'elles ne rentrent dans les mésopores. Ainsi, les matériaux MOF décrits dans la présente sont particulièrement adaptés aux séparations en flux, pour lesquelles les propriétés de diffusivité sont importantes.

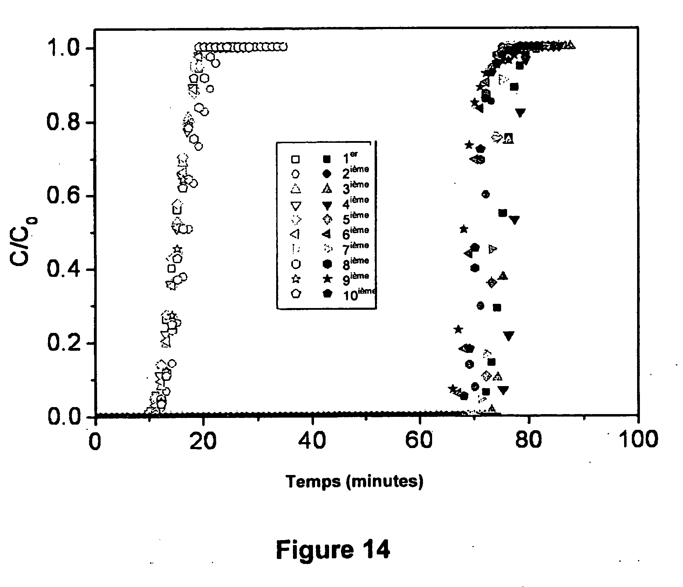

D'autre part, il a été démontré que l'intégrité des matériaux MOF selon l'invention était maintenue après plusieurs cycles de séparation (voir Exemple 10, et Figure 14). Les matériaux MOF peuvent donc être utilisés plusieurs fois après réactivation, sans que leurs propriétés structurales et/ou chimiques soient affectées. Cela est un aspect important vis-à-vis de leur applicabilité dans des processus de séparation à l'échelle industrielle.

Enfin, les inventeurs ont mis en évidence que bien que les matériaux MOF de l'invention possèdent des centres métalliques insaturés M^{(z-1)+}, qui sont des centres acides de Lewis, même si ceux-ci peuvent générer une acidité de Brønsted par coordination de molécules donneur de proton telles que l'eau, l'acidité mesurée par adsorption de CO n'est pas très significative. Ainsi, dans le cas de l'utilisation des matériaux MOF selon l'invention pour la séparation de mélanges contenant des hydrocarbures insaturés, l'acidité de Brønsted du matériau MOF n'est pas suffisante pour déclencher la polymérisation de l'hydrocarbure insaturé.

Ainsi, les solides MOF de la présente invention représentent une alternative viable aux matériaux conventionnellement utilisés dans les procédés de séparation, en pariculier pour la séparation/purification des oléfines.

D'autres avantages pourront apparaître à l'homme du métier à la lecture des exemples ci-dessous, illustrés par les figures annexées, donnés à titre illustratifs.

### Brève description des figures

- La figure 1 représente un diffractogramme RX du solide MIL-100(Fe)
- La figure 2 représente un isotherme d'adsorption d'azote à 77 K du solide MIL-100(Fe) (Po=1 atm)
- La figure 3 représente une analyse thermogravimétrique (sous air, avec une vitesse de chauffe de 2°C/minute) du composé MIL-100(Fe).
- La figure 4 représente le diffractogramme RX du solide MIL-101(Fe) (λ_{Cu}=1.5406 Å)
- La figure 5 représente une analyse thermogravimétrique (sous air, avec une vitesse de chauffe de 2°C/minute) du composé MIL-101(Fe).
- La figure 6 représente des diffractogrammes RX des solides MIL-102(Fe) brut (courbe (a)) et référence MIL-102(Cr) (courbe (b))
- La figure 7 représente une analyse thermogravimétrique (sous air) du composé MIL-102 (Fe) brut de synthèse.
- La figure 8 représente des diffractogrammes RX du solide MIL-88B-4CH₃(Fe) brut (courbe (b) en bas) et hydraté (courbe (a) en haut)
- La figure 9 représente une analyse thermogravimétrique (sous air, avec une,vitesse de chauffe de 2°C/minute) du composé MIL-88B-4CH₃(Fe) hydraté.
- La figure 10A représente des isothermes d'adsorption à 303 K propane/propylène avec le MIL-100(Fe) activé à 120°C (fer(III)).
- La figure 10B représente des isothermes d'adsorption à 303 K propane/propylène avec le MIL-100(Fe) activé à 260°C (Fer(II)/Fer(III)).
- La figure 11 représente des courbes de perçages à 2.5, 5, 10, 15 et 20 kPa de mélanges 50/50 de propane et de propylène à 40°C avec le solide MIL-100(Fe) prétraité avec du NH₄F, et activé à 250°C sous helium (3 heures) : effet de la pression partielle sur la séparation propane/propylène (vide : propylène; plein : propane).
- La figure 12 représente des isothermes d'adsorption à 40°C et 100°C de propane et de propylène dans le solide MIL-100(Fe) activé à 250°C sous vide.
- La figure 13 représente des courbes de perçages à 5 kPa de mélanges 50/50 de propane et de propylène à différentes températures avec le solide MIL-100(Fe) prétraité avec du NH₄F, et activé à 250°C sous helium (3 heures) : séparation propane/propylène en fonction de la température (mélange équimolaire propane/propylène P=5 kPa, dans He) sur la séparation propane/propylène (vide : propylène; plein : propane).
- La figure 14 représente des tests de cyclabilité des courbes de perçages à 1 bar d'un mélange 50/50 de propane et de propylène à 298 K avec le solide MIL-100(Fe) activé à 280°C sous flux d'hélium (vide : propane; plein : propylène).
- La figure 15 représente des courbes de perçages à 5kPa d'un mélange 50/50 de propane et de propylène à 40°C avec le solide MIL-100(Fe) prétraité avec du NH₄F, et activé à différentes températures sous hélium: effet de la température d'activation sous helium (3 heures) sur la séparation propane/propylène à 40°C (Pression partielle totale propane + propylène (mélange équimolaire) = 5kPa) (vide : propane; plein : propylène).
- La figure 16 représente la thermodésorption de propane/propylène dans le solide MIL-100(Fe) prétraité avec du NH₄F, et a) activé à 250°C sous helium (3 heures) ou b) sous vide secondaire pendant 12 heures (pression partielle mélange équimolaire propane/propylène dans He = 5kPa)
- La figure 17 représente la séparation de pulses de mélanges 50/50 d'alcanes et d'alcènes (1 % en poids dans un flux d'azote) à 298 K sur MIL-100(Fe) activé à 280°C. Figure 17A : mélange propane/Propylène. Figure 17B : mélange n-butane/iso-butène.
- La figure 18 représente la structure du solide MIL-100(Fe). (a) : trimère d'octaèdre et ligand trimésique ; (b) : supertétraèdre ; (c) : structure 3d schématique ; (d) : les deux types de cages mésoporeuses.
- La figure 19 représente les fenêtres pentagonales et hexagonales du solide MIL-100(Fe) après activation sous vide.
- La figure 20 représente un schéma réactionnel pour l'obtention de l'acide 3,5,3',5'-tétraméthylbiphényl 4,4'-dicarboxylique.
- La figure 21 représente un schéma de réaction pour l'obtention de l'acide 3,3'-dimethylbiphenyl 4,4'-dicarboxylique.
- La figure 22 représente une quantité de sites Fer insaturés présents dans le MIL-100(Fe) fluoré (X=F) activé sous vide à différentes températures.
- La figure 23 représente une vue schématique du phénomène de respiration (gonflement et contraction) dans les solides MIL-88A, MIL-88B, MIL-88C, MIL-88D et MIL-89. L'amplitude de gonflement entre formes sèches (en haut) et formes ouvertes (en bas) est représentée en % en bas de la figure.
- La figure 24 représente, en haut, une étude de la réversibilité du gonflement - du solide MIL-88A par diffraction des RX ((∼1.79Å), en bas, des diffractogrammes RX du solide MIL-88A en présence de solvants ((∼1.5406Å).
- La figure 25 représente un schéma explicatif de la flexibilité dans les phases hybrides MIL-53 (a) et MIL-88 (b et c).
- La figure 26 représente un schéma récapitulatif de l'activation du solide MIL-100(Fe).
- La figure 27A représente un graphique montrant l'évolution du taux de fer(II) dans le MIL-100(Fe) en fonction de la température d'activation sous vide par spectroscopie Mossbauer.
- La figure 27B représente un diffractogramme RX (thermodiffraction RX sous vide) du solide MIL-100(Fe) (λ_{Cu}=1.5406 Â).
- La figure 28 représente un graphe de la force acide de Brønsted des groupements OH de différentes espèces greffées sur un échantillon MIL-100(Cr) analysé par IR après adsorption de CO : corrélation entre le déplacement v(OH), les valeurs de Hº et la position v(CO).
- La figure 29 représente un schéma récapitulatif du montage expérimental utilisé pour les tests de séparation sur colonne des Exemples 6 à 13.
- La figure 30 représente un diagramme schématique du système manométrique d'introduction de doses de gaz utilisé pour les Exemples 6 et 8.
- Figure 31: Haut : construction du solide MIL-101 à partir de trimères d'octaèdres de fer, d'acide 1,4 Benzenedicarboxylique pour former un supertétraèdre hybride et finalement une structure zéolithique hybride à grande taille de pores. Bas : vue schématique de la charpente poreuse et représentation des deux types de cages mésoporeuses, avec leur dimensions libres. Les octaèdres de fer, les atomes de carbone sont en vert et noir, respectivement.
- La figure 32 représente la structure du solide MOF MIL-102(Fe). Gauche : vue selon l'axe des tunnels (axe c) ; droite : vue selon l'axe perpendiculaire aux tunnels (axe b, vue similaire selon l'axe a). Les atomes de fer, de carbone et les molécules d'eau sont en vert, noir et rouge, respectivement.
- La figure 33 représente la structure du solide MOF MIL-88B_4CH₃(Fe). Gauche : vue selon l'axe des tunnels (axe c) ; droite : vue selon l'axe des cages (axes a et b équivalent). Les octaèdres de fer et les atomes de carbone sont en orange et noir, respectivement.
- La figure 34 représente les courbes de perçages à 1 bar et 40°C d'un mélanges 50/50 d'acétylène et d'éthylène avec le solide MIL-100(Fe) activé à 270°C sous flux d'hélium.
- La figure 35 représente une courbe de thermodésorption d'un mélange acétylène/éthylène à partir du solide MIL-100(Fe) activé à 270° sous flux d'hélium et soumis à 1 bar d'acétylène et d'éthylène en proportion 50/50 à 40°C.
- La figure 36 représente une séparation par pulses de mélanges d'isomères C4 (40°C).
- La figure 37 représente des isothermes d'adsorption à 303 K CO/CO₂ avec le MIL-100(Fe) activé à 100°C (fer(III)) et le MIL-100(Fe) activé à 260°C (Fer(II)/Fer(III). (CO₂ : deux courbes du haut; CO : deux courbes du bas).
- La figure 38 représente la sélectivité propylène/propane en fonction de la température d'activation sous hélium du solide MIL-100(Fe) prétraité avec du NH₄F: effet de la température d'activation sous hélium sur la séparation propane/propylène à 40°C (Pression partielle totale propane + propylène (mélange équimolaire) = 5kPa).

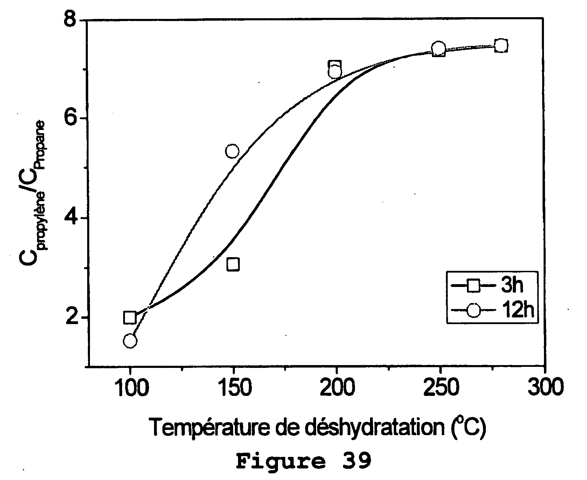

- La figure 39 représente 1' effet du temps d'activation à température variable (100-250°C) sous helium sur la séparation propane/propylène à 40°C. sur le solide MIL-100(Fe) prétraité avec NH₄F: (Pression partielle totale propane + propylène (mélange équimolaire) = 5kPa)
- La figure 40 représente la structure du carboxylate de fer MIL-88A (hydraté). Gauche : vue selon l'axe des tunnels (axe c) ; droite : vue selon l'axe perpendiculaire aux tunnels (axe b, vue similaire selon l'axe a). Les octaèdres de fer, les atomes de carbone et les molécules d'eau sont en vert, noir et rouge, respectivement.
- La figure 41 représente la structure du carboxylate de fer MIL-89(Fe). Gauche : vue selon l'axe des tunnels (axe c) ; droite : vue selon l'axe perpendiculaire aux tunnels (axe b, vue similaire selon l'axe a). Les atomes de fer, de carbone et les molécules d'eau sont en gris, noir et blanc, respectivement.
- La figure 42 représente un isotherme d'adsorption d'azote à 77K du solide MIL-88B_4CH3 (dégazé sous vide primaire à 100°C pendant 16 heures). P0=1 atmosphère.
- La figure 43A représente la structure cristallographique du MIL-126(Fe). Les polyèdres FeO₆ sont représentés en orange ou en vert, chaque couleur représentant un réseau MIL-88D. Les atomes de carbone sont en noir.
- La figure 43B représente les données cristallographiques du solide MOF MIL-126(Fe).
- La figure 44 représente un diffractogramme de rayons X du MIL-126(Fe) (λ_{Cu}=1.5406Å).
- La figure 45 représente une analyse thermogravimétrique du MIL-126(Fe) sous air (vitesse de chauffe de 2°C/min).
- La figure 46 représente des isothermes d'adsorption d'azote du MIL-126(Fe) (P₀=1 atmosphère).
- La figure 47 représente un diffractogramme RX du solide 3,3',5,5'-azobenzenetetracarboxylate de fer(III) brut de synthèse.
- La figure 48 représente les résultats de l'analyse thermogravimétrique du composé 3,3',5,5'-azobenzenetetracarboxylate de fer brut de synthèse sous air, à une vitesse de chauffe de 2°C/minute (perte de masse Pm en fonction de la température T).
- La figure 49 représente un isotherme d'adsorption d'azote du 3,3',5,5'-azobenzenetetracarboxylate de fer (P₀=1atmosphère).
- La figure 50 représente un diffractogramme RX du solide 2,5-dihydroxotéréphthalate de fer brut (CPO-27(Fe)).
- La figure 51 représente un diffractogramme RX du solide MIL-100 synthétisé a partir de différents proportions de Fe :Cr. (De haut vers le bas : 1 :0, 0.7 :0.3, 0.5 :0.5, 0.3 :0.7, 0 :1)
- La figure 52 représente un isotherme d'adsorption d'azote du composé MIL-100 Fe/Cr synthétisé a partir du rapport molar Fe :Cr=0.5 :0.5 (P₀=1atmosphère).
- La figure 53 représente des isothermes d'adsorption de propane et de propylène à l'équilibre thermodynamique à 40°C à partir de 1 g de solide CPO-27(Ni) préalablement activé à 250°C sous vide primaire (P=1 Pa pendant 16 heures.
- La figure 54 représente des isothermes d'adsorption de propane et de propylène à l'équilibre thermodynamique à 40°C à partir de 1 g de solide CPO-27(Fe) préalablement activé à 150°C sous vide primaire (P=1 Pa pendant 16 heures).
- La figure 55 représente des isothermes d'adsorption de propane et de propylène à l'équilibre thermodynamique à 40°C à partir de 1 g de solide CPO-27(Fe) préalablement activé à 250°C sous vide primaire (P=1 Pa pendant 16 heures).
- La figure 56 représente une comparaison de la teneur en ions Fe²⁺ (exprimée en % de site Fer(II)par rapport au nombre de site total Fer) obtenue pour le MIL-100(Fe) non fluoré (X=OH,Cl), le MIL-100(Fe) fluoré (X=F) prétraité avec NH₄F, et le MIL-127(Fe).
- La figure 57 représente des isothermes d'adsorption propane/propylène à 40°C à partir du solide MIL-100(Fe) prétraité avec NH₄F (S_{BET}=2340 m²/g)
- La figure 58 représente l'effet de la température sur les isothermes d'adsorption propane/propylène sur une solide MIL-100(Fe) prétraité avec NH₄F, activé à 250°C sous vide secondaire (12 heures).
- La figure 59 représente une étude de la sélectivité de la séparation propane/propylène en fonction de la température, mélange équimolaire propane/propylène, dans He, sur la séparation propane/propylène à P(partielle)=5 kPa sur le solide MIL-100(Fe) prétraité avec NH₄F, activé à 250°C sous hélium (3 heures).
- La figure 60 représente des calculs de chaleurs d'adsorption propane/propylène (déduites des isothermes d'adsorption corps pur (cf figure 59)) sur le solide MIL-100(Fe) prétraité avec NH₄F, activé à 250°C sous vide secondaire (12 heures)
- La figure 61 représente l'effet de la présence d'hydrogène dans le mélange propane/propylène sur la séparation propane/propylène dans le MIL-100(Fe) prétraité avec NH₄F, activé à 250°C sous helium (3 heures) (pression partielle mélange équimolaire propane/propylène dans He = 5 kPa)
- La figure 62 représente des isothermes d'adsorption C3 avec des granulats de MIL-100(Fe).
- La figure 63 représente des tests de séparation avec des granulats de MIL-100(Fe). Température d'adsorption: 40°C. Pression partielle variable.
- La figure 64 représente la désorption isotherme à 40°C sous flux d'hélium (30cc/min (gauche) et 100 CC/min (droite) après test de perçage(mélange équimolaire Propane/propylène) à 40°C et Pression partielle = 5kPa sur le solide MIL-100(Fe) prétraité avec NH₄F
- La figure 65 représente des courbes de perçages répétitives avec régénération sous hélium du solide MIL-100(Fe) prétraité avec NH4F. Test de perçage à 40°C et 5 kPa. Régénération: 30 ml/min He à 200°C pendant 1 h (gauche) ou 100 ml/min He à 40°C pendant 2 heures.
- La figure 66A représente la désorption isotherme à 80°C sous flux d'hélium (100 CC/min après test de perçage (mélange équimolaire Propane/propylène) à 80°C et Pression partielle = 5kPa sur le solide MIL-100(Fe) prétraité avec NH₄F.
- La figure 66B représente l'effet de la régénération du solide MIL-100(Fe) prétraité avec NH4F. Test de perçage à 80°C et 5 kPa. Régénération: avec 100 ml/min He à 80°C pendant 2 heures.
- La figure 67 représente l'effet de la pression à température variable (40 ou 80°C) sur la séparation propane/propylène sous hélium sur le solide MIL-100(Fe) prétraité avec NH4F puis dégazé à 250°C pendant 3 heures sous flux d'hélium (mélange équimolaire propane + propylène).
- La figure 68 schématise un appareil utilisé pour les expériences de microcalorimétrie de l'Exemple 25.
- La figure 69 représente des isothermes d'adsorption et chaleurs d'adsorption de propane et propylène à 303K dans le solide MIL-100(Fe) après activation à 250°C sous vide secondaire pendant 12 heures.
- La figure 70 représente des isothermes d'adsorption et chaleurs d'adsorption du propylène à 303K dans le solide MIL-100(Fe) après le traitement a 100 ou 250°C sous vide secondaire pendant 12 heures.
- La figure 71 représente des isothermes d'adsorption et chaleurs d'adsorption de CO et de CO2 à 303K dans le solide MIL-100(Fe) après activation à 100°C sous vide secondaire pendant 12 heures.
- La figure 72 représente les bandes infrarouges de la vibration d'élongation des molécules NO en interaction avec les sites coordinativement insaturés Fe(II) du matériau MIL-102(Fe) activé sous vide à 225°C pendant 12 heures (spectre a), du materiau MIL-100(Fe) prétraité avec NH₄F et activé sous vide à 250°C pendant 12 heures (spectre b), du matériau MIL-127(Fer) activé sous vide à 150°C pendant 12 heures (spectre c). Les spectres infrarouges sont enregistrés à température ambiante sous une pression de NO égale à 1333 Pa.
- La figure 73 représente un spectre infrarouge du solide MIL-127(Fer) sous un flux de H₂/CO/CO₂ à 30°C après chauffage sous un flux d'argon à 150°C (spectre a), 250°C (b).
- La figure 74 représente les isothermes d'adsorption à 25°C de C3H4 sur le MIL-100(Fe) prétraité avec NH₄F et activé sous vide secondaire à 150°C et 250°C. Les quantités sont qualitativement déduites de l'intensité. de la bande d'élongation du groupement méthyl à 2931-cm-1.
- La figure 75 représente les isothermes d'adsorption à 25°C de C3H4 sur le MIL=100(Fe) prétraité avec NH₄F et activé sous vide secondaire à 150°C et 250°C dans la gamme de pression 0-0,3 torr. A gauche: Les quantités adsorbées sont déduites de l'intensité de la bande d'élongation du groupement méthyl à 2931 cm-1. A droite: les quantités adsorbées données en *µ*mol par gramme de solide deshydraté sont déduites par méthode volumétrique.
- La figure 76 représente l'évolution des bandes infrarouge ν(C≡C) de C3H4 adsorbé sur les sites FeII et FeIII du MIL-100(Fe) prétraité avec NH₄F et activé à 250°C en fonction de la pression de C3H4 en phase gaz (gamme 0-2,5 torr).
- La figure 77 représente les isothermes d'adsorption de C3H6 sur le MIL-100(Fe) fluoré (X=F) activé à 150°C et 250°C dans la gamme de pression 0-60 torr. les quantités adsorbées de C3H6 sur les sites Fe(II) sont qualitativement représentées par l'aire de la bande à 2996 cm-1.

### EXEMPLES

### Exemple 1 Synthèse et données sur des matériaux MOF carboxylate de fer de la présente invention

Cet exemple décrive la synthèse de divers carboxylates de fer. Les solides obtenus ont ensuite été caractérisés selon les méthodes décrites ci-dessous.

L'analyse de la structure cristalline des solides carboxylate de fer a été effectuée par diffraction des rayons X (RX) à l'aide d'un diffractomètre Siemens D5000 (radiation CuKα λ_{Cu}=1,5406 Å, mode θ-2θ), à temperature ambiante à l'air. Les diagrammes sont representés soit en distances angulaires (2θ, en degrés °) soit en distances inter-reticulaire (d, en Å ou Angström).

La Caractérisation de la porosité (surface spécifique Langmuir et volume de pore) des solides a été mesurée par l'adsorption d'azote à 77 K avec un appareil Micromeretics ASAP-2010. Les solides ont été préalablement dehydratés à 150°C sous vide primaire pendant une nuit. L'isotherme d'adsorption d'azote par les solides est donnée par une courbe représentant le volume d'azote adsorbé V (en cm³/g) en fonction du rapport de la pression P sur la pression de référence P₀=1 atm.

L'analyse thermogravimétrique a été effectuée sous atmosphère d'air à l'aide d'un appareil Modèle TA-instrument 2050. La vitesse de chauffe était de 2°C/minute. La courbe résultant de l'analyse thermogravimétrique des solides représente la perte de masse Pm (en %) en fonction de la température T (en °C).

L'analyse élémentaire des solides a été effectuée par le Service Central d'Analyse du CNRS de Vernaison :

### Analyse organique :

Microanalyses C,H,N,O,S dans les produits pharmaceutiques, les polymères et d'une façon générale les produits de synthèse, par détections coulométrique, catharométrique ou cellule infra-rouge.

### Analyse inorganique:

Les principales techniques mises en oeuvre :
- ICP-AES (« Inductive Coupled Plasma - Atomic Emission Spectroscopy » ou « Spectrométrie d'émission atomique par plasma à couplage inductif ») avec différents types de détecteurs
- ICP-MS (« Inductively Coupled Plasma-Mass Spectrometry » ou « Spectrométrie de masse par Plasma à couplage inductif ») avec des spectromètres de masse quadripôlaires ou à secteur magnétique
- CVAAS (« Cold-Vapor Atomic Absorption Spectroscopy » ou « Spectroscopie d'absorption atomique à vapeur froide »)
- Couplage ICP / MS / HPLC (« Inductively Coupled Plasma /Mass Spectrometry / High Performance Liquid Chromatography » ou « Chromatographie liquide Haute Performance / Spectrométrie de Masse par Plasma à Couplage Inductif »)
- Fluorescence X
- Traitements d'échantillons par voie humide, par voie sèche ou micro-ondes.

### a) MIL-100(Fe) ou Fe₃O[C₆H₃-(CO₂)₃]₂.x.nH₂O (X=F, Cl, OH)

Le carboxylate de fer MIL-100(Fe) a été synthétisé selon deux conditions: avec et sans acide fluorhydrique.

### Conditions de synthèse avec de l'acide fluorhydrique

56 mg de fer métallique en poudre (1 mmol, commercialisé par la société Riedel de Haen, 99%), 140 mg d'acide 1,3,5-benzenetricarboxylique (0,6 mmol, 1,3,5-BTC ; commercialisé par la société Aldrich, 99%) sont dispersés dans 5 mL d'eau distillée avec 0,6 mL d'acide nitrique 2M (commercialisé par la socité VWR, 50%) et 0,4 mL d'acide fluorhydrique 5M (commercialisé par la société SDS, 50%). Le tout est placé dans un corps en Téflon de 23 ml mis dans une bombe métallique de la société PAAR et laissé pendant 6 jours à 150°C avec un palier de monté en température de 12 heures et un palier de descente en température de 24 heures. Le solide est récupéré par filtration.

Puis, le solide (200 mg) est suspendu dans 100 mL d'eau distillée à reflux sous agitation pendant 3h pour éliminer l'acide trimésique restant dans les pores. Le solide est ensuite récupéré par filtration à chaud.

### Conditions de synthèse sans acide fluorhydrique :

0,27 g de FeCl₃.6H₂O (1 mmol, commercialisé par la société Alfa Aesar; 98%), 140 mg (0,6 mmol) d'acide 1,3,5-benzenetricarboxylique (1,3,5-BTC ; commercialisé par la société Aldrich, 99%) sont dispersés dans 5 mL de l'eau distillée. Le tout est laissé dans un corps en Téflon de 23 mL mis dans une Bombe métallique PAAR pendant 3 jours à 130°C. Le solide est ensuite filtré et lavé avec de l'acétone

Le solide (200 mg) est ensuite suspendu dans 100 mL d'eau distillée à reflux sous agitation pendant 3h pour éliminer l'acide trimésique restant dans les pores. Le solide est ensuite récupéré par filtration à chaud.

### Données caractéristiques du solide carboxylate de fer MIL-100(Fe) :

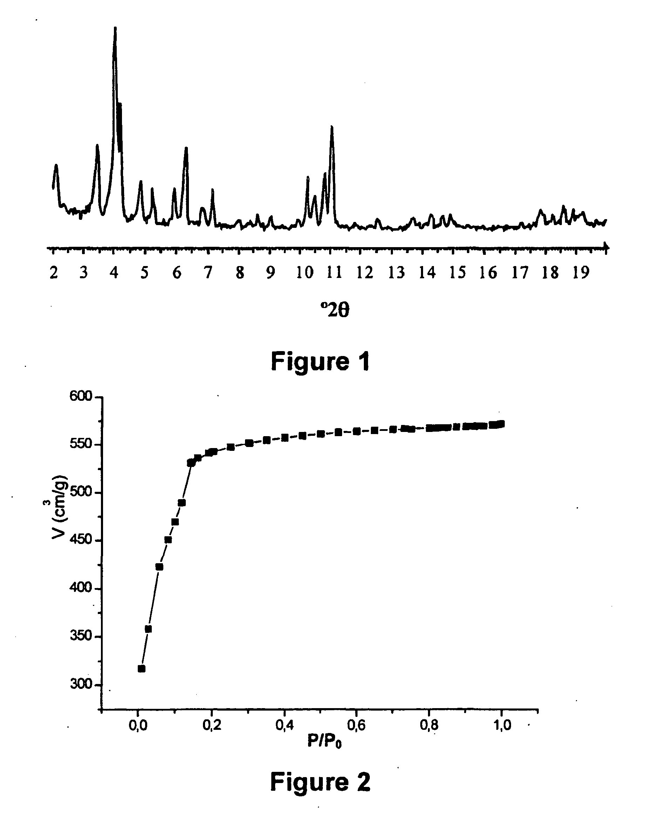

L'analyse de la structure cristalline du solide MIL-100(Fe) par diffraction des rayons X donne le diffractogramme RX représenté en figure 1.

Les caractéristiques de la structure cristalline sont les suivantes :
- le groupe d'espace est Fd-3m (n°227).
- les paramètres de maille sont : a=73,1 Å ; volume de la maille V=393000 Å³.

L'isotherme d'absorption d'azote à 77 K du solide MIL-100(Fe) (à la pression P₀=1 atm) est donnée en figure 2. La surface (Langmuir) spécifique de ce solide est proche de 2900 m². g⁻¹.

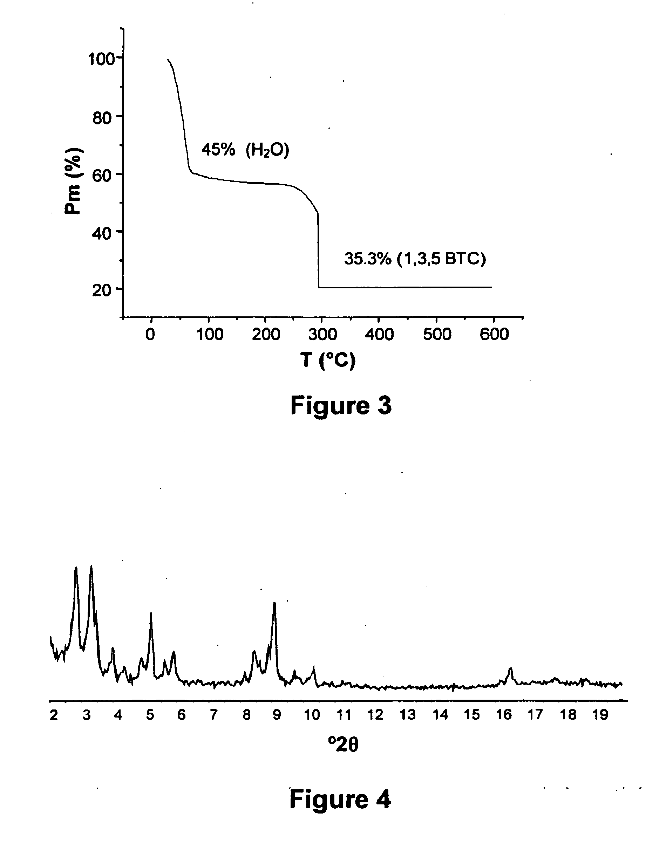

La courbe résultant de l'analyse thermogravimétrique du composé MIL-100(Fe) est donné en figure 3. Ce diagramme représente la perte de masse Pm (en %) en fonction de la température T (en °C).

Le tableau ci-dessous donne l'analyse élémentaire du solide MIL-100(Fe) ou Fe₃O[C₆H₃-(CO)₃]₂.X.nH₂O dans le cas où X=F.

**Tableau 1.**

| Elément (% massique) | % Fer | % Carbone | % Fluor |
|---|---|---|---|
| MIL-100(Fe) | 13,8 | 23,5 | 1,3 |

### b) MIL-101(Fe) ou Fe₃O[C₆H₄-(CO₂)₂]₃.x.nH₂O (X=F, Cl, OH)

### Synthèse du solide MIL-101(Fe) :

0,27 g (1 mmol) de FeCl₃.6H₂O, 249 mg (1,5 mmol) d'acide 1,4-benzenedicarboxylique (1,4-BDC, commercialisé par la société Aldrich, 99%) sont dispersés dans 10 mL de diméthylformamide (DMF, commercialisé par la société Fluka, 98%). Le mélange est laissé 12 heures à 100°C dans un corps en Téflon de 23 ml mis dans une Bombe métallique PAAR. Le solide est ensuite filtré et lavé avec de l'acétone.

### Données caractéristiques du solide MIL-101(Fe) :

Le diffractogramme RX du solide MIL-101(Fe) est représenté dans la figure 4.

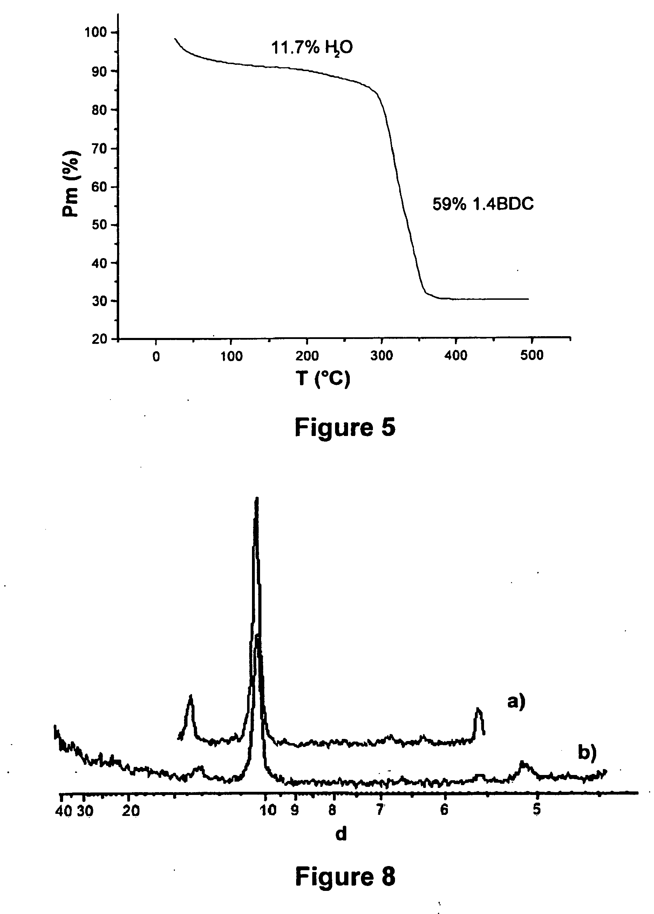

L'analyse thermogravimétrique (sous air, à une vitesse de chauffe de 2°C/min) du solide MIL-101(Fe) brut est représentée en figure 5.

Les caractéristiques de la structure cristalline sont les suivantes :
- le groupe d'espace est Fd-3m (n°227).
- les paramètres de maille du solide MIL-101(Fe) à 298 K sont : a=89,0 Å ; volume de la maille V=707000 Å³.

La composition élémentaire théorique du solide sec (avec X=F) est la suivante : Fe 24,2 % ; C 41,4 % ; F 2,7 % ; H 1,7 %.

### c) MIL-102(Fe) ou Fe₆O₂X₂[C₁₀H₂-(CO₂)₄]₃.nH₂O (X=F, Cl...)

### Synthèse du solide MIL-102(Fe) non fluoré :

270 mg (1 mmol) de FeCl₃.6H₂O (Alfa Aesar, 98%) et 268 mg (1 mmol) d'acide 1,4,5,8-naphtalènetétracarboxylique sont dispersés dans 5 ml d'eau distillée. Le mélange est laissé dans un corps en Téflon de 23 ml mis dans une Bombe métallique PAAR pendant 15 heures à 100°C. Le solide est récupéré par filtration.

### Données caractéristiques du solide MIL-102(Fe) :

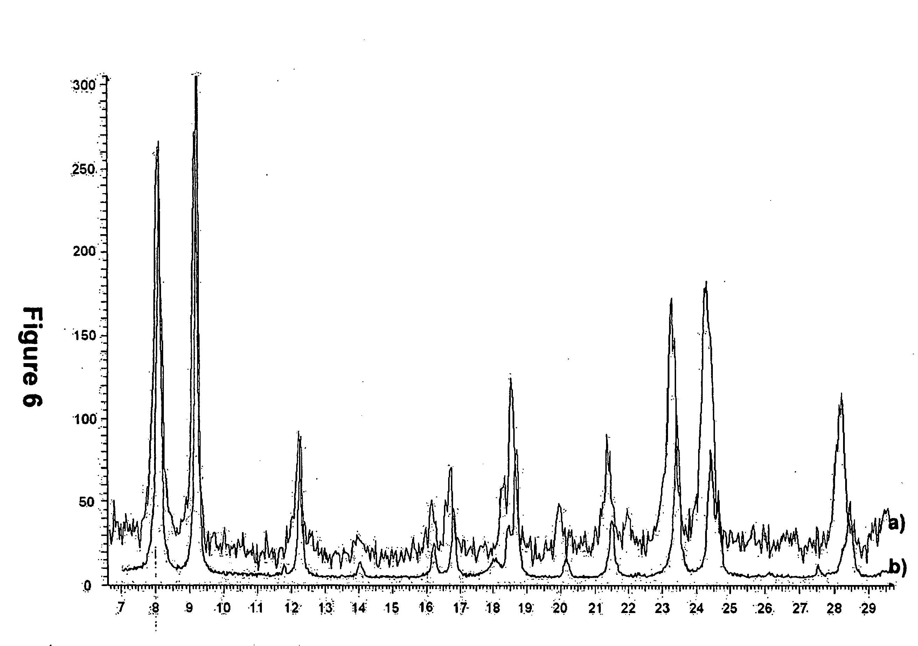

La figure 6 représente les diffractogrammes RX du solide MIL-102(Fe) brut (courbe (a)) et du solide MIL-102(Cr) (courbe (b)).

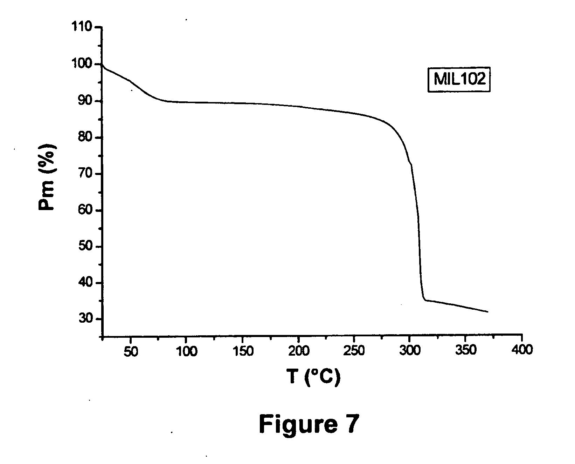

L'analyse thermogravimétrique (sous air, à une vitesse de chauffe de 2°C/min) du solide MIL-102(Fe) brut est représentée en figure 7.

Ce composé présente une surface spécifique faible (surface de Langmuir : 101 m²/g) à l'azote à 77 K.

### Obtention du solide MIL-102(Fe) fluoré :

0.2 g de solide MIL-102(Fe) non fluoré de formule Fe₆O₂Cl₂[C₁₀H₄(CO₂)₄]₃.nH₂O obtenu selon le mode opératoire décrit précédemment est mis en présence de 1 g de fluorure de sodium NaF dans 100 ml d'eau distillée. Le mélange est laissé sous agitation dans un corps en Téflon de 125 ml pendant 15 heures à température ambiante. Le solide est récupéré par filtration et lavé à cinq reprises dans de l'eau distillée pour évacuer les traces de NaF. L'analyse semi-quantitative par EDX indique une teneur en fluor de 0.17 fluor par fer. Le solide ainsi traité possède une formule approximative de type Fe₆O₂F(OH)[C₁₀H₄(CO₂)₄]₃.nH₂O.

### d) MIL-88B-4CH₃ (Fe) ou Fe₃O[C₆_(CH₃)₄-(CO₂)₂]₃.x.nH₂O (X=F, Cl, OH)

### Synthèse du solide MIL-88B-4CH₃ (Fe) :

0,27 g (1 mmol) de FeCl₃.6H₂O (Alfa Aesar, 98%), 222 mg (1 mmol) d'acide 1,4-tétraméthyltéréphthalique (Chem Service, 95%) sont dispersés dans 10 ml de DMF (Fluka, 98%) avec 0,4 mL de soude 2M (Alfa Aesar, 98%). Le mélange est laissé dans un corps en Téflon de 23 ml mis dans une Bombe métallique PAAR pendant 12 heures à 100°C. Le solide est récupéré par filtration.

200 mg du solide sont mis en suspension dans 100mL d'eau sous agitation à temperature ambiante pendant 12 heures pour éliminer l'acide restant dans les pores. Le solide est ensuite récupéré par filtration.

### Données caractéristiques du solide MIL-88B-4CH₃ (Fe) :

La figure 8 représente le diffractogramme RX du solide brut (courbe (b) en bas) et du solide hydraté (courbe (a) en haut).

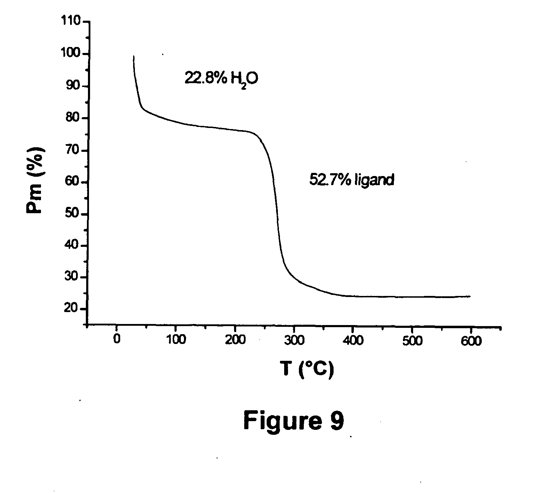

L'analyse thermogravimétrique (sous air, à une vitesse de chauffe de 2°C/minute) du solide MIL-88B-4CH₃(Fe) hydraté est représentée en figure 9.

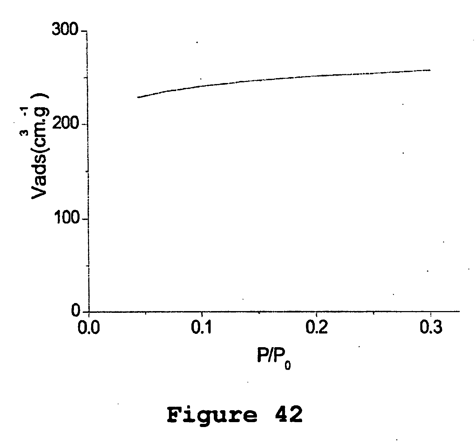

Ce composé présente une surface accessible de l'ordre de 1200 m²/g (modèle de Langmuir) à l'azote à 77 K, puisque la structure sèche possède une taille de pores suffisante (6-7 Å) pour incorporer de l'azote N₂ (figure 42).

### d) MIL-88A(Fe) ou Fe₃O[C₂H₂-(CO₂₎₂]₃.x.nH₂O (X=F, Cl, OH)

### Synthèse du solide MIL-88A(Fe) :

0,27 g (1 mmol) de FeCl₃.6H₂O (commercialisé par la société Alfa Aesar, 98%) et 116 mg (1 mmol) d'acide fumarique (Aldrich, 99%) sont dispersés dans 5 ml de diméthylformamide (DMF, Fluka, 98%) avec 0,4 mL NaOH 2M (Alfa Aesar, 98%). Le mélange est laissé dans un corps en Téflon de 23 ml mis dans une Bombe métallique PAAR pendant 12 heures à 100°C. Le solide est ensuite filtré et lavé avec de l'acétone.

Puis, le solide (200 mg) est mis en suspension dans 100 mL d'eau distillée sous agitation pendant 12h pour éliminer le solvant restant dans les pores. Le solide est ensuite récupéré par filtration.

### Données caractéristiques du solide MIL-88A(Fe) :

L'analyse de la structure cristalline du solide donne les caractéristiques répertoriées dans le tableau suivant :

**Tableau 2 : paramètres de maille du solide MIL-88A, sec et hydraté.**

| Phase | a (Å) | c (Å) | Volume de maille (Å³) | Taille de pore (Å) | Groupe d'espace |
|---|---|---|---|---|---|
| MIL-88A sec | 9,25 | 15,30 | 1135 | - | P-62c |
| MIL-88A hydrate (H₂O) | 13,9 | 12,66 | 2110 | 6-7 | P-62c |

Une analyse thermogravimétrique du composé MIL-88A hydraté (sous air, à une vitesse de chauffe de 2°C/minute) a été effectuée (résultats non présentés).

Le composé MIL-88A ne présente pas de surface accessible (supérieure à 20 m²/g) à l'azote à 77 K, puisque la structure sèche possède une taille de pores trop faible pour incorporer de l'azote N₂.

L'analyse élémentaire est donnée dans le tableau ci-dessous :

**Tableau 3 :**

| Élément (% massique) | % Fer | % Carbone |
|---|---|---|
| MIL-88A (brut) | 21,8 | 24,0 |

### e) MIL-88B(Fe) ou Fe₃O[C₆H₄-(CO₂)]₃.x.nH₂O (X=F, Cl, OH)

### Synthèse du solide MIL-88B(Fe) :

0,27 g (1 mmol) de FeCl₃.6H₂O (Alfa Aesar, 98%) et 116 mg (1 mmol) d'acide 1,4-benzenedicarboxylique (Aldrich, 98%) sont dispersés dans 5 mL de diméthylformamide (DMF, Fluka, 98%) avec 0,4 mL de soude 2M (Alfa Aesar, 98%). Le mélange est laissé dans un corps en Téflon de 23 ml mis dans une Bombe métallique PAAR pendant 12 heures à 100°C. Le solide est ensuite filtré et lavé avec de l'acétone.

200 mg du solide sont mis en suspension dans 100mL d'eau distillée sous agitation pendant 12h pour éliminer le solvant restant dans les pores. Puis, le solide est récupéré par filtration.

### Données caractéristiques du solide MIL-88B(Fe) :

L'analyse de la structure cristalline du solide donne les caractéristiques répertoriées dans le tableau suivant :

**Tableau 4 : paramètres de maille du solide MIL-88B, sec et hydraté.**

| Phase | a (Å) | c (Å) | Volume de maille (Å³) | Taille de pore (Å) | Groupe d'espace |
|---|---|---|---|---|---|
| MIL-88B sec | 9,6 | 19,1 | 1500 | <3 | P-62c |
| MIL-88B hydrate (EtOH) | 15,7 | 14,0 | 3100 | 9 | P-62c |

Une analyse thermogravimétrique du composé MIL-88B hydraté (sous air, à une vitesse de chauffe de 2°C/minute) a été effectuée (résultats non présentés).

Le composé MIL-88B ne présente pas de surface accessible (supérieure à 20 m²/g) à l'azote à 77 K, puisque la structure sèche possède une taille de pores trop faible pour incorporer de l'azote N₂.

### f) MIL-89(Fe) ou Fe₃O[C₄H₄-(CO₂₎₂]₃.x.nH₂O (X=F, Cl, OH)

### Synthèse du solide MIL-89(Fe) :

172 mg (1 mmol) d'acétate de fer (préparé suivant la synthèse décrite par Dziobkowski et al., *Inorg. Chem.* 1982, 20, 671 [ref. 29]) et 150 mg (1 mmol) d'acide muconique (Fluka, 97%) sont dispersés dans 10 ml de méthanol (Fluka, 98%) avec 0,35 mL de soude 2M (Alfa Aesar, 98%). Le mélange est laissé dans un corps en Téflon de 23 ml mis dans une bombe métallique PAAR pendant 3 jours à 100°C. Le solide est ensuite filtré et lavé avec de l'acétone.

200 mg du solide sont mis en suspension dans 100mL d'eau distillée sous agitation pendant 12h pour éliminer le solvant restant dans les pores. Le solide est ensuite récupéré par filtration.

### Données caractéristiques du solide MIL-89(Fe) :

Une analyse du solide MIL-89(Fe) sec, du solide MIL-89(Fe) solvaté avec le DMF et du solide MIL-89(Fe) hydraté par diffraction RX a été effectuée (résultats non présentés).

Le composé MIL-89(Fe) ne présente pas de surface accessible (supérieure à 20 m²/g) à l'azote à 77 K, puisque la structure sèche possède une taille de pores trop faible pour incorporer de l'azote N₂.

### g) MIL-88C(Fe) ou Fe₃O[C₁₀H₆-(CO₂)₂]₃.x.nH₂O (X=F, Cl, OH)

### Préparation de l'acétate de fer(III)

L'acétate de fer(III), utilisé dans les exemples ci-dessous pour la synthèse des matériaux MOF selon l'invention, est synthétisé selon le protocole suivant. Cette synthèse peut se référer à la publication de Dziobkowski et al., Inorg. Chem., 1982, 21, 671 [ref. 14].

6,72 g de poudre de fer métallique (Riedel-de Haën, 99%), 64 mL d'eau déionisée et 33,6 mL d'acide perchlorique à 70 % dans l'eau (Riedel-de Haën) sont mélangés sous agitation magnétique et chauffés à 50°C pendant 3 heures. Après arrêt du chauffage, la solution est agitée pendant 12 heures. Le fer métallique résiduel est éliminé par décantation suivie d'un changement de récipient. 20,6 mL de solution de peroxyde d'hydrogène dans l'eau (commercialisé par la société Alfa Aesar, 35%) sont ajoutés goutte à goutte sous agitation, le tout étant maintenu dans un bain de glace à 0°C. On ajoute 19,7 g d'acétate de sodium (Aldrich, 99%) à la solution bleue sous agitation en maintenant la solution à 0-5°C. On laisse la solution s'évaporer pendant 3 jours sous la hotte dans un cristallisoir (Volume=0,5 1) en verre. Finalement, les cristaux rouges d'acétate de fer sont récupérés par filtration et lavés très rapidement avec de

l'eau déionisée glacée. Les cristaux sont ensuite séchés sous atmosphère d'air.

### Synthèse du solide MIL-88C(Fe) :

172 mg (1 mmol) d'acétate de fer (synthétisé selon le protocole ci-dessus) et 140 mg (1 mmol) d'acide 2,6-naphtalenedicarboxylique (Aldrich, 95%) sont dispersés dans 5 ml de diméthylformamide (DMF, Fluka, 98%). Le mélange est laissé dans un corps en Téflon de 23 ml mis dans une bombe métallique PAAR pendant 3 jours à 150°C avec un palier de montée de 12 heures et un palier de descente de 24 heures. Le solide est récupéré par filtration. Le solide est séché à 150°C sous air pendant 15 heures.

### Données caractéristiques du solide MIL-88C(Fe) :

L'analyse de la structure cristalline du solide donne les caractéristiques répertoriées dans le tableau suivant :

**Tableau 5 : paramètres de maille du solide MIL-88C, sec et solvaté.**

| Phase | a (Å) | c (Å) | volume de maille (Å³) | Taille de pore (Å) | Groupe d'espace |
|---|---|---|---|---|---|
| MIL-88C sec | 9,9 | 23,8 | 2020 | 3 | P-62c |
| MIL-88C solvaté (Pyridine) | 18,7 | 18,8 | 5600 | 13 | P-62c |

Une analyse thermogravimétrique du composé MIL-88C brut de synthèse (sous air, à une vitesse de chauffe de 2°C/minute) a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### h) MIL-88D(Fe) ou Fe₃O[C₁₂H₈-(CO₂)₂]₃.x.nH₂O (X=F, Cl, OH)

### Synthèse du solide MIL-88D(Fe) :

270 mg (1 mmol) de FeCl₃.6H₂O (Alfa Aesar, 98%) et 140 mg (0,6 mmol) d'acide 4,4'-biphenyldicarboxylique (Fluka, 95%) sont dispersés dans 5 ml de Diméthylformamide (DMF, Aldrich, 99%). Le mélange est laissé dans un corps en Téflon de 23 ml mis dans une Bombe métallique PAAR pendant 12 heures à 100°C avec un palier de montée d'une heure et un palier de descente d'une heure. Le solide est récupéré par filtration.

Le solide est ensuite séché à 150°C sous air pendant 15 heures.

### Données caractéristiques du solide MIL-88D(Fe) :

L'analyse de la structure cristalline du solide donne les caractéristiques répertoriées dans le tableau suivant :

**Tableau 6 : paramètres de maille du solide MIL-88D, sec et solvaté (pyridine).**

| Phase | a (Å) | c (Å) | Volume de maille (Å³) | Taille de pore (Å) | Groupe d'espace |
|---|---|---|---|---|---|
| MIL-88D sec | 10,1 | 27,8 | 2480 | <3 | P-62c |
| MIL-88D solvaté (pyridine) | 20,5 | 22,4 | 8100 | 16 | P-62c |

Une analyse par diffraction RX du solide MIL-88D brut et hydraté a été effectuée (résultats non présentés).

Une analyse thermogravimétrique du composé MIL-88D(Fe) hydraté (sous air, à une vitesse de chauffe de 2°C/minute) a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### i) MIL-88B-NO₂ (Fe) ou Fe₃O[C₆H₃NO₂-(CO₂)₂]₃.x.nH₂O (X=F, Cl, OH)

### Synthèse du solide MIL-88H-NO₂(Fe) :

0,27 g (1 mmol) de FeCl₃.6H₂O (Alfa Aesar, 98%) et 211 mg (1 mmol) d'acide 2-nitrotéréphthalique (Acros, 99%) sont dispersés dans 5 ml d'eau distillée. Le mélange est laissé dans un corps en Téflon de 23 ml mis dans une bombe métallique PAAR pendant 12 heures à 100°C. Le solide est récupéré par filtration.

200 mg du solide sont mis en suspension dans 10mL d'éthanol absolu dans un corps en Téflon de 23 ml mis dans une bombe métallique PAAR pendant 12 heures à 100°C pour éliminer l'acide restant dans les pores. Le solide est ensuite récupéré par filtration et séché à 100°C.

### Données caractéristiques du solide MIL-88B-NO₂ (Fe) :

Une analyse par diffraction RX du solide MIL-88B-NO₂ brut et hydraté a été effectuée (résultats non présentés).

Une analyse thermogravimétrique (sous air, à une vitesse de chauffe de 2°C/minute) du composé MIL-88B-NO₂(Fe), après lavage et séchage, a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

L'analyse élémentaire est donnée dans le tableau ci-dessous :

**Tableau 7 :**

| Élément (% massique) | % Fer | % Carbone | % Azote |
|---|---|---|---|
| MIL-88B-NO₂ | 20,6 | 39,3 | 4,6 |

### j) MIL-88B-2OH(Fe) ou Fe₃O[C₆H₂ (OH)₂-(CO₂)₂]₃.x.nH₂O (X=F, Cl, OH)

### Synthèse du solide MIL-88B-2OH(Fe) :

354 mg (1 mmol) de Fe(ClO₄)₃.xH₂O (Aldrich, 99%) et 198 mg (1 mmol) d'acide 2,5-dihydroxotéréphthalique (obtenu par hydrolyse de l'ester diéthylique correspondant, Aldrich 97%) sont dispersés dans 5 ml de DMF (Fluka, 98%). Le mélange est laissé dans un corps en Téflon de 23 ml mis dans une Bombe métallique PAAR pendant 12 heures à 85°C. Le solide est récupéré par filtration.

Pour éliminer l'acide restant dans les pores, le produit est calciné à 150°C sous vide pendant 15 heures.

### Données caractéristiques du solide MIL-88H-2OH(Fe) :

Une analyse par diffraction RX du solide MIL-88B-2OH brut, hydraté et séché sous vide a été effectuée (résultats non présentés).

Une analyse thermogravimétrique (sous air, à une vitesse de chauffe de 2°C/minute) du composé MIL-88B-2OH(Fe), après lavage et séchage, a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

L'analyse élémentaire est donnée dans le tableau ci-dessous :

**Tableau 8 :**

| Élément (% massique) | % Fer | % Carbone |
|---|---|---|
| MIL-88B-2OH | 15,4 | 36,5 |

k) MIL-88B-NH₂ (Fe) ou Fe₃O[C₆H₃NH₂-(CO₂)₂]₃.x.nH₂O (X=F, Cl, OH)

### Synthèse du solide MIL-88B-NH₂(Fe) :

0.27 g (1 mmol) de FeCl₃·6H₂O (Alfa Aesar, 98%) et 180 mg (1 mmol) d'acide 2-aminotéréphthalique (Fluka, 98%) sont dispersés dans 5 ml de l'éthanol absolu. Le mélange est laissé dans un corps en Téflon de 23 ml mis dans une Bombe métallique PAAR pendant 3 jours à 100°C. Le solide est récupéré par filtration.

Pour éliminer l'acide restant dans les pores, le solide est calciné à 200°C pendant 2 jours.

### Données caractéristiques du solide MIL-88B-NH₂(Fe) :

Une analyse par diffraction RX du solide MIL-88B-NH₂ brut, et séché sous vide a été effectuée (résultats non présentés).

Une analyse thermogravimétrique (sous air, à une vitesse de chauffe de 2°C/minute) du solide MIL-88B-NH₂(Fe) hydraté a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### 1) MIL-88B-CH₃ (Fe) ou Fe₃O[C₆H₃CH₃-(CO₂)₂]₃.x.nH₂O (X=F, Cl, OH)

### Synthèse du solide MIL-88B-CH₃(Fe)

354 mg (1 mmol) de Fe(ClO₄)_{3·}xH₂O (Aldrich, 99%) et 180 mg (1 mmol) d'acide 2-méthyltéréphthalique (préparé suivant la synthèse décrite par Anzalone et al., J. Org. Chem. 1985, 50, 2128 [ref. 30]) sont dispersés dans 5 ml de méthanol (Fluka, 99%). Le mélange est laissé dans un corps en Téflon de 23 ml mis dans une Bombe métallique PAAR pendant 3 jours à 100°C. Le solide est récupéré par filtration.

200 mg du solide sont mis en suspension dans 10mL de DMF sous agitation à température ambiante pour échanger l'acide présent dans les pores par le DMF, puis le DMF est éliminé par chauffage à 150°C sous vide pendant 12 heures.

### Données caractéristiques du solide MIL-88B-CH₃ (Fe) :

Une analyse par diffraction RX du solide MIL-88B-CH₃ brut, hydraté et solvaté avec le DMF a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### m) MIL-88B-Cl (Fe) ou Fe₃O[C₆H₃Cl-(CO₂)₂]₃.x.nH₂O (X=F, Cl, OH)

### Synthèse du solide MIL-88B-Cl(Fe) :

354 mg (1 mmol) de Fe(ClO₄)₃.xH₂O (Aldrich, 99%) et 200 mg (1 mmol) d'acide 2-chlorotéréphthalique (synthétisé selon la synthèse A de l'exemple 2) sont dispersés dans 10 ml de DMF avec 0,1 mL de HF 5M (SDS, 50%) et 0,1 mL de HCl 1M (Aldrich, 37%). Le mélange est laissé dans un corps en Téflon de 23 ml mis dans une Bombe métallique PAAR pendant 5 jours à 100°C. Le solide est récupéré par filtration.

Le solide obtenu est calciné à 150°C sous vide.

### Données caractéristiques du solide MIL-88B-Cl (Fe) :

Une analyse par diffraction RX du solide MIL-88B-Cl brut, hydraté et solvaté avec le DMF a été effectuée (résultats non présentés).

L'analyse thermogravimétrique (sous air, à une vitesse de chauffe de 2°C/minute) du solide MIL-88B-Cl(Fe) hydraté a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### n) MIL-88B-4F (Fe) ou Fe₃O[C₆F₄-(CO₂)₂]₃.x.nH₂O (X=F, Cl, OH)

### Synthèse du solide MIL-88B-4F (Fe) :

270 mg (1 mmol) de FeCl₃.6H₂O (Alfa Aesar, 98%) et 230 mg (1 mmol) d'acide tétrafluorotéréphthalique (Aldrich, 98%) sont dispersés dans 10 ml d'eau distillée. Le mélange est laissé dans un corps en Téflon de 23 ml mis dans une Bombe métallique PAAR pendant 12 heures à 85°C. Le solide est récupéré par filtration.

200 mg du solide sont mis en suspension dans 20mL de l'eau sous agitation à température ambiante pendant 2 heures pour éliminer l'acide restant dans les pores. Le solide est ensuite récupéré par filtration.

### Données caractéristiques du solide MIL-88B-4F (Fe) :

Une analyse par diffraction RX du solide brut, du solide hydraté et du solide solvaté avec l'éthanol a été effectuée (résultats non présentés).

L'analyse thermogravimétrique (sous air, à une vitesse de chauffe de 2°C/minute) du solide MIL-88B-4F(Fe) hydraté a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### o) MIL-88B-Br (Fe) ou Fe₃O[C₆H₃Br-(CO₂)₂]₃.x.nH₂O (X=F, Cl, OH)

### Synthèse du solide MIL-88B-Br (Fe) :

270 mg (1 mmol) de FeCl₃.6H₂O (Alfa Aesar, 98%) et 250 mg (1 mmol) d'acide 2-bromotéréphthalique (Fluka, 95%) sont dispersés dans 10 ml de DMF (Fluka, 98%) avec 0,2 mL d'acide fluorhydrique 5M (SDS, 50%). Le mélange est laissé dans un corps en Téflon de 23 ml mis dans une Bombe métallique PAAR pendant 12 heures à 150°C. Le solide est récupéré par filtration.

Pour éliminer l'acide restant dans les pores, le solide est calciné à 150°C sous vide pendant 15 heures.

### Données caractéristiques du solide MIL-88B-Br (Fe) :

Une analyse par diffraction RX du solide brut et du solide hydraté a été effectuée (résultats non présentés).

L'analyse thermogravimétrique (sous air, à une vitesse de chauffe de 2°C/minute) du solide MIL-88B-Br(Fe) hydraté a été effectuée (résultats non présentés).

Ce composé ne présente pas de surface accessible (supérieure à 20 m²/g) à l'azote à 77 K, puisque la structuré sèche possède une taille de pores trop faible pour incorporer de l'azote N₂.

### p) MIL-88E (Pyr) (Fe) ou Fe₃O[C₄H₃N₂-(CO₂)₂]₃.x.nH₂O (X=F, Cl, OH)

### Synthèse du solide MIL-88E (Fe) :

270 mg (1 mmol) de FeCl₃.6H₂O (Alfa Aesar, 98%) et 204 mg (1 mmol) d'acide 2,5-pyrazinedicarboxylique (Aldrich, 98%) sont dispersés dans 5 ml de DMF (Fluka, 98%) avec 0,05 mL de HF 5M (SDS, 50%). Le mélange est laissé dans un corps en Téflon de 23 ml mis dans une Bombe métallique PAAR pendant 3 jours à 100°C. Le solide est récupéré par filtration.

### Données caractéristiques du solide MIL-88E (Fe) :

Une analyse par diffraction RX du solide MIL-88E(Fe) brut de synthèse a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### q) MIL-88P (Thio) (Fe) ou Fe₃O[C₄H₂S-(CO₂)₂]₃.x.nH₂O (X=F, Cl, OH)

### Synthèse du solide MIL-88F(Fe) :

354 mg (1 mmol) de Fe(ClO₄)₃.xH₂O (Aldrich, 99%) et 258 mg (1 mmol) d'acide 2,5-tiophenedicarboxylique (Aldrich, 99%) sont dispersés dans 2,5 ml de DMF (Fluka, 98%) avec 0,1 mL de HF 5M (SDS, 50%). Le mélange est laissé dans un corps en Téflon de 23 ml mis dans une Bombe métallique PAAR pendant 3 jours à 100°C. Le solide est récupéré par filtration.

200 mg du solide sont mis en suspension dans 100mL d'eau sous agitation à température ambiante pendant 12 heures pour éliminer l'acide restant dans les pores. Le solide est ensuite récupéré par filtration.

### Données caractéristiques du solide MIL-88F(Fe) :

Une analyse par diffraction RX du solide brut et du solide hydraté a été effectuée (résultats non présentés).

L'analyse thermogravimétrique (sous air, à une vitesse de chauffe de 2°C/minute) du solide MIL-88F(Fe) hydraté a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### r) MIL-88D-4CH₃ (Fe) ou Fe₃O[C₁₂H₄(CH₃)₄-(CO₂)₂]₃.x.nH₂O (X=F, Cl, OH)

### Synthèse du solide MIL-88D-4CH₃ (Fe) :

354 mg (1 mmol) de Fe(ClO₄)₃.xH₂O (Aldrich, 99%) et 298 mg (1 mmol) d'acide tétraméthylbiphényl-4,4'-dicarboxylique (synthétisé selon la synthèse B décrite dans l'exemple 2) sont dispersés dans 5 ml de DMF (Fluka, 98%) avec 0,2 mL de soude 2M (Alfa Aesar, 98%). Le mélange est laissé dans un corps en Téflon de 23 ml mis dans une Bombe métallique PAAR pendant 12 heures à 100°C. Le solide est récupéré par filtration.

200 mg du solide sont mis en suspension dans 10mL de DMF sous agitation à température ambiante pendant 2 heures pour échanger l'acide restant dans les pores. Le solide est ensuite récupéré par filtration, et le DMF présent dans les pores éliminé par calcination à 150°C sous vide pendant 15 heures.

### Données caractéristiques du solide MIL-88D-4CH₃(Fe) :

L'analyse par diffraction RX du solide brut et du solide hydraté a été effectuée (résultats non présentés).

L'analyse thermogravimétrique (sous air, à une vitesse de chauffe de 2°C/minute) du solide MIL-88D-4CH₃(Fe) hydraté a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### s) MIL-88D-2CH₃(Fe) ou Fe₃O[C₁₂H₆(CH₃)₂-(CO₂)₂]₃.x.nH₂O (X=F, Cl, OH)

### Synthèse du solide MIL-88D-2CH₃(Fe) :

270 mg (1 mmol) de FeCl₃.6H₂O (Alfa Aesar, 98%) et 268 mg (1 mmol) d'acide diméthylbiphényl-4,4'-dicarboxylique (synthétisé selon la synthèse C décrite dans l'exemple 2) sont dispersés dans 5 ml de DMF (Fluka, 98%) avec 0,25 mL de HF 5M (SDS, 50%). Le mélange est laissé dans un corps en Téflon de 23 ml mis dans une Bombe métallique PAAR pendant 12 heures à 150°C. Le solide est récupéré par filtration.

Pour éliminer l'acide restant dans les pores, le solide est calciné à 150°C sous vide pendant 15 heures.

### Données caractéristiques du solide MIL-88D-2CH₃(Fe)

L'analyse par diffraction RX du solide brut, du solide hydraté MIL-88D-2CH₃(H₂O) et du solide en suspension dans l'eau MIL-88D-2CH₃(goutte H₂O) a été effectuée (résultats non présentés).

L'analyse thermogravimétrique (sous air, à une vitesse de chauffe de 2°C/minute) du solide MIL-88D-2CH₃(Fe) brut a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### t) MIL-88G (AzBz) (Fe) ou Fe₃O[C₁₂H₈N₂-(CO₂)₂]₃.X.nH₂O (X=F, Cl, OH)

### Synthèse du solide MIL-88G(Fe)

118 mg (0,33 mmol) de Fe(ClO₄)₃.xH₂O (Aldrich, 99%) et 90 mg (0,33 mmol) d'acide 4,4'-azobenzènedicarboxylique (synthétisé selon la méthode décrite par Ameerunisha et al., J. Chem. Soc. Perkin Trans.2 1995, 1679 [ref. 31]) sont dispersés dans 15 ml de DMF (Fluka, 98%). Le mélange est laissé dans un corps en Téflon de 23 ml mis dans une Bombe métallique PAAR pendant 3 jours à 150°C. Le solide est récupéré par filtration.

200 mg du solide sont mis en suspension dans 10 mL de DMF sous agitation à température ambiante pendant 2 heures pour échanger l'acide restant dans les pores. Le solide est ensuite récupéré par filtration et le DMF restant dans les pores éliminé par calcination à 150°C sous vide pendant 15 heures.

### Données caractéristiques du solide MIL-88G(Fe) :

L'analyse par diffraction RX du solide MIL-88G brut, du solide solvaté avec le DMF et du solide solvaté avec la pyridine a été effectuée (résultats non présentés).

L'analyse thermogravimétrique (sous air, à une vitesse de chauffe de 2°C/minute) du solide MIL-88G(Fe) brut a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### u) MIL-88G-2Cl (AzBz-2Cl) (Fe) ou Fe₃O[C₁₂N₂Cl₂-(CO₂)₂]₃.X.nH₂O (X=F, Cl, OH)

### Synthèse du solide MIL-88G-2Cl(Fe)

177 mg (0,5 mmol) de Fe(ClO₄)₃.xH₂O (Aldrich, 99%) et 169 mg (0,5 mmol) d'acide dichloro-4,4'-azobenzenedicarboxylique (synthétisé selon la synthèse D décrite dans l'exemple 2) sont dispersés dans 15 ml de DMF (Fluka, 98%). Le mélange est laissé dans un corps en Téflon de 23 ml mis dans une Bombe métallique PAAR pendant 12 heures à 150°C. Le solide est récupéré par filtration.

200 mg du solide sont mis en suspension dans 10mL de DMF sous agitation à température ambiante pendant 2 heures pour échanger l'acide restant dans les pores. Le solide est ensuite récupéré par filtration, et le DMF restant dans les pores est éliminé par calcination à 150°C sous vide pendant 15 heures.

### Données caractéristiques du solide MIL-88G-2C1(Fe)

L'analyse par diffraction RX du solide MIL-88G-2Cl brut et du solide MIL-88G-2Cl sec a été effectuée (résultats non présentés).

L'analyse thermogravimétrique (sous air, à une vitesse de chauffe de 2°C/minute) du solide MIL-88G-2Cl(Fe) brut a été effectuée (résultats non présentés).

Ce composé ne présente pas de surface accessible (supérieure à 20 m²/g) à l'azote à 77 K, puisque la structure sèche possède une taille de pores trop faible pour incorporer de l'azote N₂.

### v) MIL-126(Fe) ou Fe₆O₂X₂[C₁₀H₂-(CO₂)₄]₃.nH₂O (X=F, Cl...)

### Synthèse du solide MIL-126(Fe)

270 mg (1 mmol) de FeCl₃.6H₂O (Alfa Aesar, 98%) et 140 mg (0,6 mmol) d'acide 4,4'-biphenyldicarboxylique (Fluka, 95%) sont dispersés dans 5 ml de Diméthylformamide (DMF, Aldrich, 99%). Le mélange est laissé dans un corps en Téflon de 23 ml mis dans une Bombe métallique PAAR pendant 12 heures à 150°C avec un palier de montée de 1 heure et un palier de descende de 1 heure. Le solide est récupéré par filtration.

Le solide est ensuite séché à 150°C sous vide primaire pendant 15 heures.

### Données caractéristiques du solide MIL-126(Fe) :

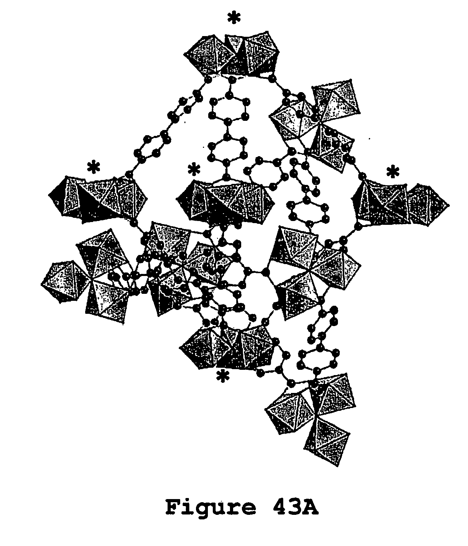

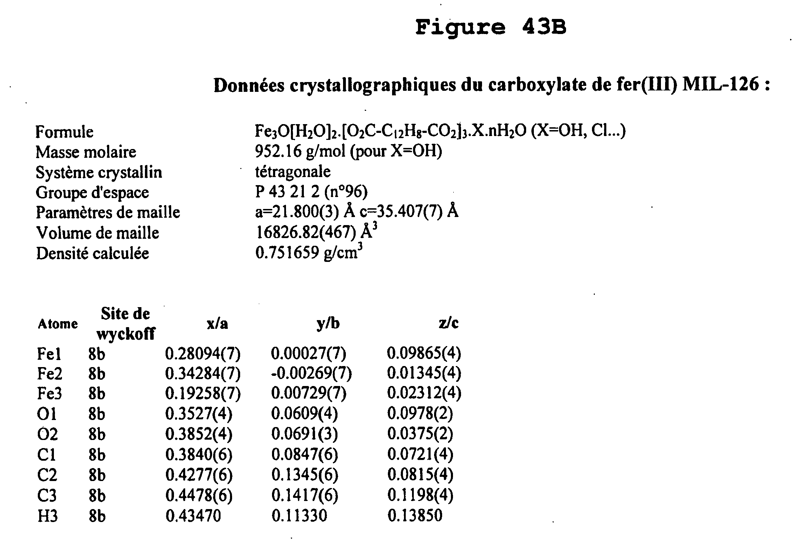

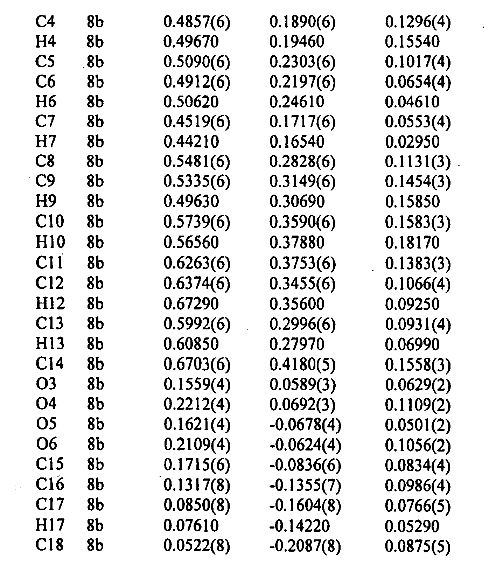

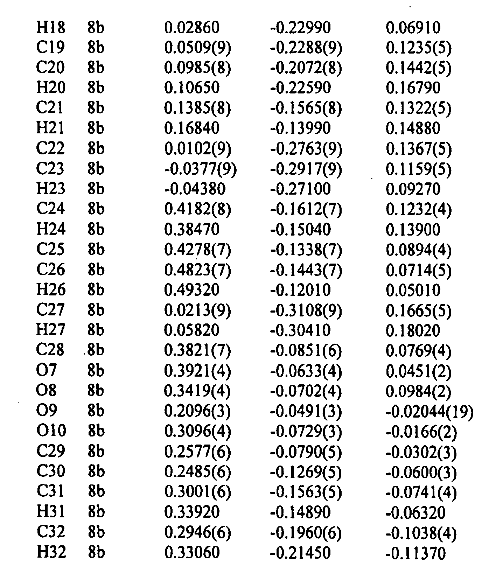

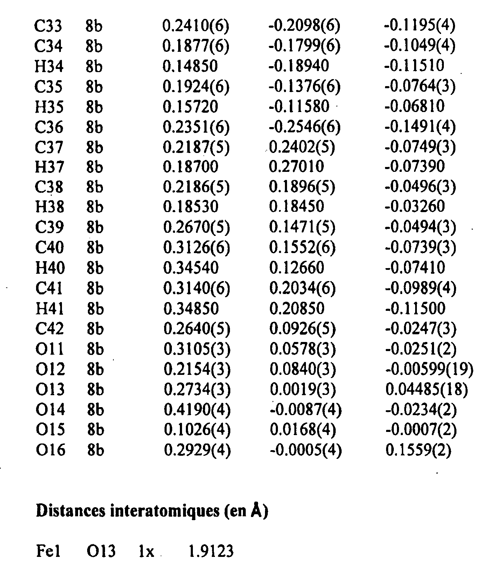

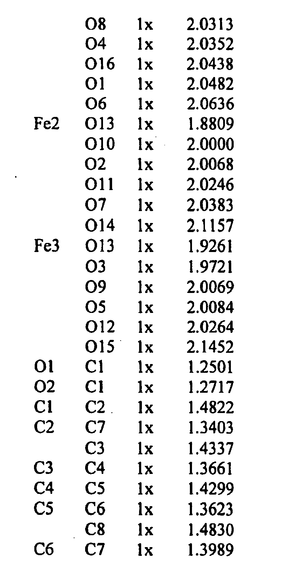

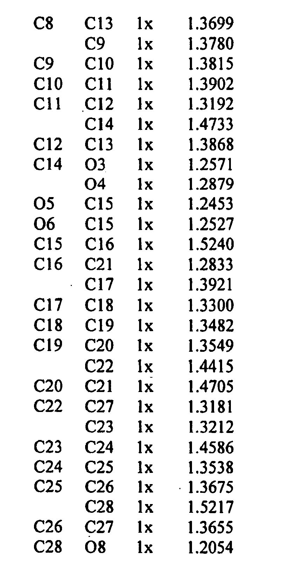

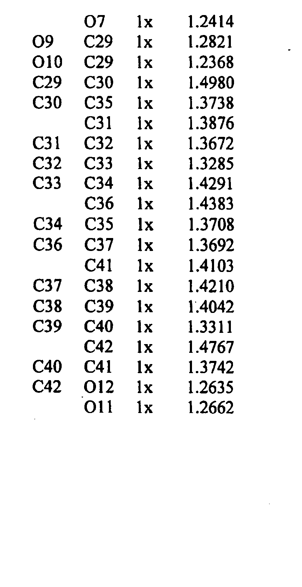

La structure cristallographique du solide MIL-126(Fe) est une forme interpénétrée de la structure MIL-88D(Fe), c'est-à-dire qu'elle possède deux sous-réseaux cristallins de type MIL-88D enchevêtrés (figure 43).

L'analyse de la structure cristalline du solide donne les caractéristiques répertoriées dans le tableau suivant :

**Tableau 9 : paramètres de maille du solide MIL-126, sec et solvaté (dimethylformamide).**

| Phase | a (Å) | c (Å) | Volume de maille (Å³) | Taille de pore (Å) | Groupe d'espace |
|---|---|---|---|---|---|
| MIL-126 sec | 19,5 | 35.3 | 13500 | 4 à 10 | P 41 21 2 |
| MIL-126 solvaté (DMF) | 21.8 | 36,1 | 17200 | 5 à 11 | P 41 21 2 |

Les données cristallographiques complètes sont données en figure 43B.

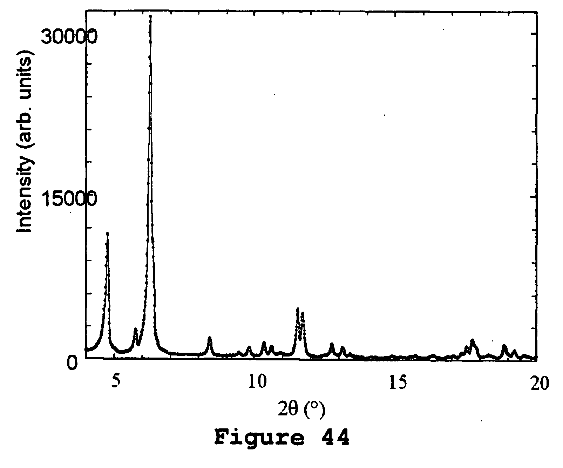

La figure 44 représente le diffractogramme RX du solide MIL-126 brut.

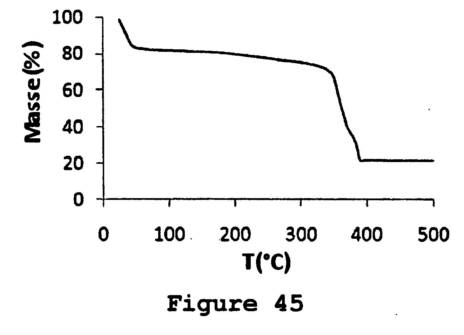

Les résultats de l'analyse thermogravimétrique du composé MIL-126(Fe) brut de synthèse (sous air, à une vitesse de chauffe de 2°C/minute) sont représentés en figure 45 (perte de masse Pm en fonction de la température T).

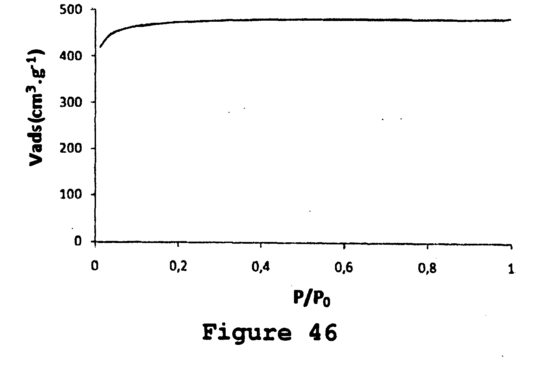

Ce composé présente une surface accessible importante(Langmuir) (supérieure à 2100 m²/g) à l'azote à 77 K (figure 46).

### w) MIL-127(Fe) ou Fe₆O₂[C₁₂H₆N₂-(CO₂)₄]₃.X₂.nH₂O (X=F, Cl, OH)

Dans cet exemple, on fabrique la phase isotype de celle à l'indium publiée par Y. Liu et al, Angew. Chem. Int. Ed. 2007, 46, 3278-3283 [ref 51].

### Synthèse du solide MIL-127(Fe) :

Le solide est récupéré par filtration et séché sous vide à 90°C.

118 mg (0,3 mmol) de Fe(ClO₄)₃.nH₂O (Aldrich, 98%) et 119 mg (0,6 mmol) d'acide 3,3',5,5'-azobenzènetetracarboxylique (synthétisé selon le protocole de synthèse E décrit dans l'exemple 3 ci-dessous) sont dispersés dans 5 mL de Diméthylformamide (DMF, Aldrich, 99%) avec l'adition de 0.1 mL d'acide fluorhydrique 5M (HF, SDS 50%). Le mélange est laissé dans un corps en Téflon de 23 mL mis dans une Bombe métallique PAAR pendant 3 jours à 150°C avec un palier de montée de 1 heure. Le solide est récupéré par filtration.

Le solide est ensuite séché à 200°C sous vide primaire pendant 15 heures.

### Données caractéristiques du solide MIL-127(Fe) : 3,3',5,5'-azobenzènetetracarboxylate de fer

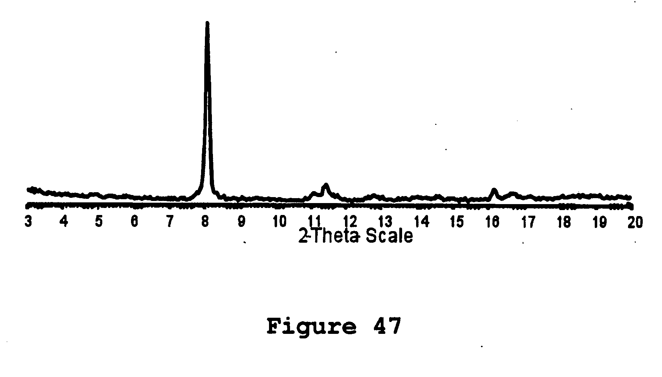

La figure 47 représente le diffractogramme RX du solide 3,3',5,5'-azobenzenetetracarboxylate de fer(III) brut de synthèse.

La phase, de symétrie cubique, est isostructurale de celle publiée par le groupe du Prof. Eddaoudi [ref 51] à l'indium (groupe d'espace Pa3).

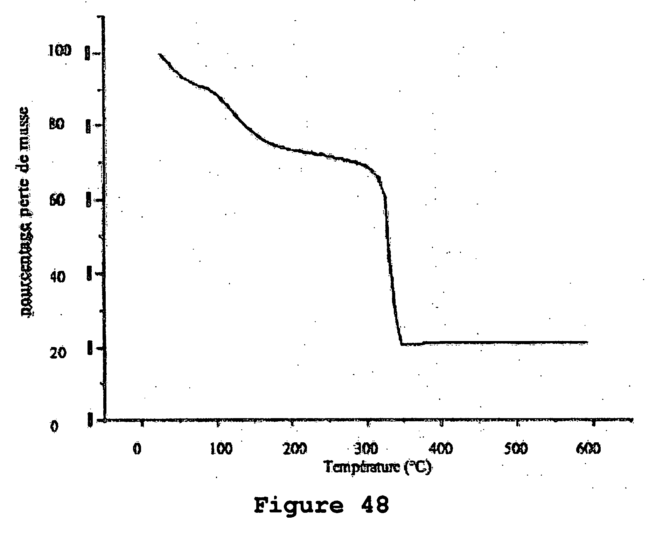

Les résultats de l'analyse thermogravimétrique du composé 3,3',5,5'-azobenzenetetracarboxylate de fer brut de synthèse (sous air, à une vitesse de chauffe de 2°C/minute) sont représentés en figure 48 (perte de masse Pm en fonction de la température T).

Les pertes de masse observées à des températures inférieures à 250°C correspondent au solvant (eau, diméthylformamide) présent dans les pores.

La décomposition du produit intervient vers 300°C pour conduire à de l'oxyde de fer(III).

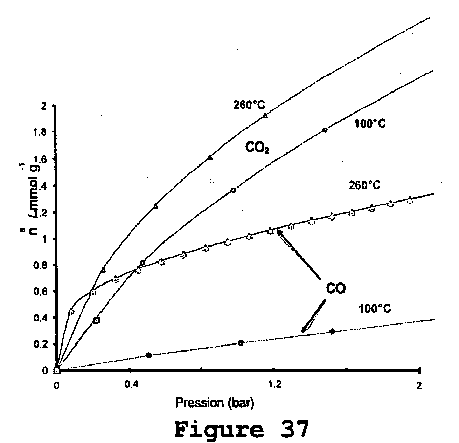

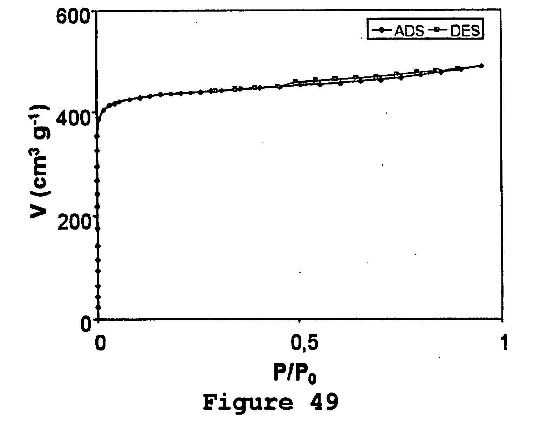

Ce composé présente une surface accessible importante (Langmuir) (supérieure à 2000 m²/g) à l'azote à 77 K (figure 37). (Porosimétrie à l'azote appareil Microméritics ASAP 2010). La figure 49 représente un isotherme d'adsorption d'azote du 3,3',5,5'-azobenzenetetracarboxylate de fer (P₀=1atmosphère).

### x) CPO-27(Fe) ou 2,5-dihydroxoterephthalate de fer ou Fe₂(₂OC-C₆H₂(OH)₂-CO₂)(H₂O).xH₂O

### Synthèse du solide 2,5-dihydroxotéréphthalate de fer:

270 mg (1 mmol) de FeClO₃.6H₂O (Alfa Aesar, 98%) et 200 mg (1 mmol) d'acide 2,5-dihydroxotéréphthalique (obtenu par hydrolyse de l'ester diéthylique correspondant, Aldrich 97%) sont dispersés dans 5 ml de diméthylformamide (DMF, Aldrich, 99%). Le mélange est laissé dans un corps en Téflon de 23 ml mis dans une bombe métallique PAAR pendant 3 jours à 150°C avec un palier de montée de 12 heures et un palier de refroidissement de 24 heures. Le solide est récupéré par filtration.

Le solide est ensuite séché à 150°C sous vide primaire pendant 15 heures.

### Données caractéristiques du solide 2,5-dihydroxoterephthalate de fer :

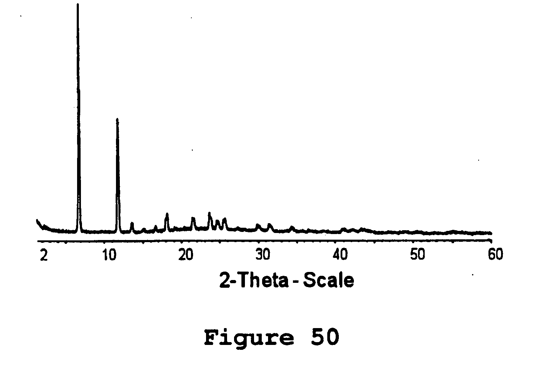

La figure 50 représente le diffractogramme RX du solide 2,5-dihydroxotéréphthalate de fer brut. La phase, de symétrie trigonal, est un isotype de celle publiée par Dietzel et al. au cobalt et nickel (groupe de espace R3) [P.D.C. Dietzel, B. Panella, M. Hirscher, R. Blom, H. Fjellvag, Chem. Commun., 2006, 956-961 [ref 53] et P.D.C. Dietzel, R.E. Johnsen, R. Blom, H. Fjellvag, P.Chem. Eur. J., 2008, 14, 2389-2397 [ref 54]].

### y) Exemple de synthèse d'un solide hybride poreux mixte MIL-100 Cr/Fe

Le solide hybride MIL-100 bimétallique, contenant dans son structure deux métaux différents, le chrome et le fer, a été obtenu en utilisant la synthèse microondes (CEM u Waves, Mars 5) à 200°C pendant 1 h en partant du rapport molaire suivant :Fe :Cr :acide 1,3,5-benzene tricarboxylique :HF :HNO₃ :H₂O=X :Y :0.67 :2 :0.6 :278 (X+Y=1).

Le solide est récupéré par filtration. Puis, le solide (200 mg) est suspendu dans 100 mL d'eau distillée à reflux sous agitation pendant 3h pour éliminer l'acide trimésique restant dans les pores. Le solide est ensuite récupéré par filtration à chaud.

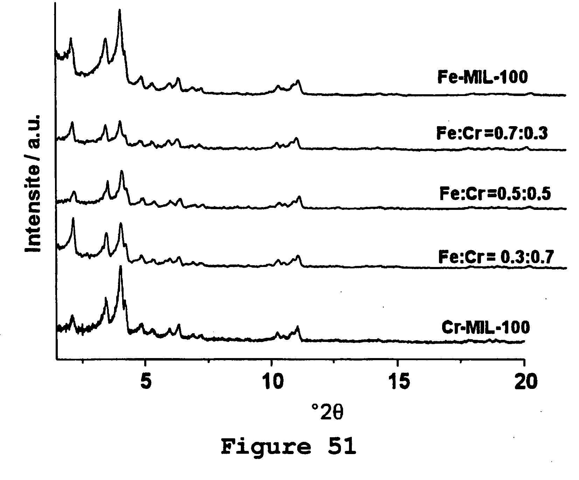

La figure 51 représente le diffractogramme RX du solide MIL-100 synthétisé a partir de différents proportions de Fe :Cr. (De haut vers le bas : 1 :0, 0.7 :0.3, 0.5 :0.5, 0.3 :0.7, 0 :1)

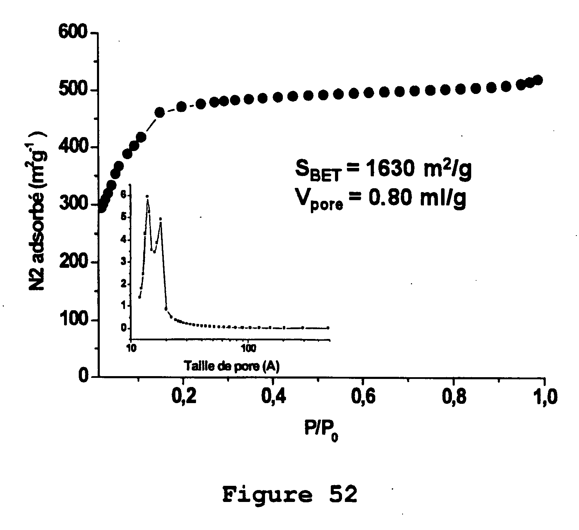

Le composé MIL-100 Fe/Cr synthétisé a partir du rapport molar Fe :Cr=0.5 :0.5 présente une surface accessible importante (BET) (supérieure à 1600 m²/g) à l'azote à 77 K (figure 52). (Porosimétrie à l'azote appareil Microméritics ASAP 2010). La figure 52 représente un isotherme d'adsorption d'azote du composé MIL-100 Fe/Cr synthétisé a partir du rapport molaire Fe :Cr=0.5 :0.5 (P₀=1atmosphère).

### Exemple 2 : Synthèse des ligands

### a) Synthèse A : synthèse de l'acide chlorotéréphtalique

6 g (0,043 mol) de chloroxylène (commercialisé par la société Aldrich, > 99%), 16 mL d'acide nitrique (commercialisé par la société VWR, 70%) et 60 mL d'eau distillée sont introduits dans un corps en téflon de 120 mL. Celui-ci est placé dans une bombe métallique PAAR, chauffé à 170°C pendant 12h. Le produit est récupéré par filtration, puis lavé abondamment à l'eau distillée. Un rendement de 75% est obtenu.

RMN ¹H (300 MHz, d6-DMSO) : δ (ppm) : 7,86 (d, J = 7,8 Hz), 7,93 (dd, J = 7,8; 1,2 Hz), 7,96 (d, J = 1,2 Hz)

### b) Synthèse B synthèse de l'acide 3,5,3ʹ,5ʹ-tétraméthylbiphényl-4,4'-dicarboxylique

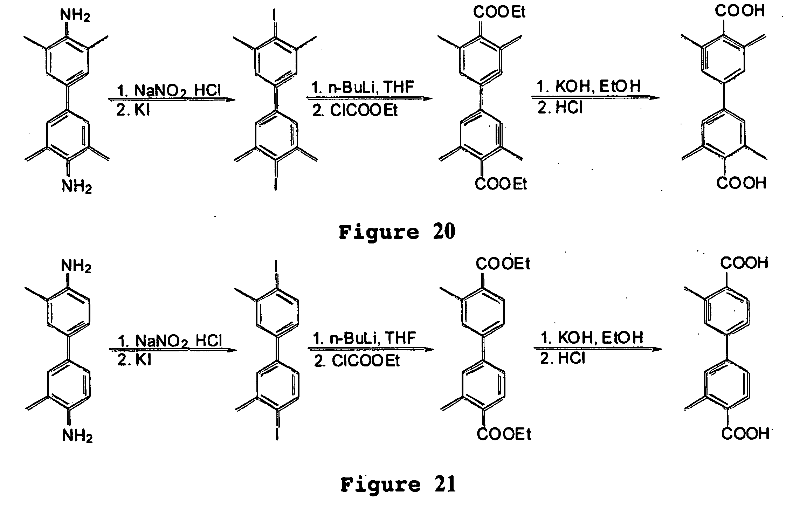

Le schéma réactionnel de cette synthèse est représenté en figure 20.

### 1^{ère} étape :

10,2 g de tétraméthylbenzidine (98%, Alfa Aesar) sont mis en suspension dans 39 mL d'acide chlorhydrique concentré (37%, commercialisé par la société Aldrich) à 0°C. La diazotation a été effectuée par addition d'une solution du nitrite de sodium (6 g dans 50 mL d'eau). Après 15 min d'agitation à 0°C, une solution d'iodure de potassium (70 g dans 200 mL d'eau) a été ajoutée lentement à la solution violette résultante. Une fois l'addition terminée, le mélange est agité pendant 2 heures à température ambiante. La suspension noire résultante est filtrée pour récupérer un précipité noir, lavé à l'eau. Le solide est mis en suspension dans le dichlorométhane (DCM, 98%, commercialisé par la société SDS) et une solution saturée de thiosulfate de sodium est ajoutée, causant la décoloration. Après 1 heure d'agitation, la phase organique est décantée et la phase aqueuse est extraite au DCM. La phase organique est séchée sur sulfate de sodium, puis évaporée pour donner l'intermédiaire diiodé sous forme d'un solide grisâtre. L'élution avec du pentane pur sur colonne de silice (commercialisé par la société SDS) permet d'obtenir le mélangé des composés monoiodé et diiodé. Le mélange de ces derniers a été directement employé dans l'étape suivante.

### 2^{e} étape :

7,2 g du composé iodé brut est solubilisé dans 100 mL de tétrahydrofurane (THF, distillé sur sodium). Après refroidissement à -78°C, 35 mL de n-butyllithium dans le cyclohexane (2,5 M, commercialisé par la société Aldrich) sont ajoutés. La solution est laissée revenir à température ambiante, une suspension blanche apparaît après 2 heures. Elle est à nouveau refroidie à -78°C et 12 mL d'éthylchloroformate sont ajoutés. Le mélange est laissé à température ambiante, une solution jaune limpide est obtenu après 1 heure. Une partition entre l'eau et le dichlorométhane, suivie d'une extraction au dichlorométhane donne le diester brut. Celui-ci est purifié par chromatographie sur gel de silice, avec pour éluant un mélange Et₂O/Pentane : 1/9, (rapport frontal : R_{f} = 0,3). 6,3 g de diester sont obtenus sous la forme d'un solide incolore (rendement de 42 % à partir de la benzidine).

Caractérisation du diester obtenu : RMN ¹H (300 MHz, CDCl₃): δ (ppm) : 1,29 (t, *J* = 7,2 Hz, 6H), 2,29 (s, 13H); 4,31 (q, *J* = 7,2 Hz, 4H); 7,12 (s, 4H). RMN ¹³C (75 MHz, CDCl₃): δ (ppm) : 14,3 (CH₃), 19,9 (CH₃), 61,0 (CH₂), 126,5 (CH), 133,2 (Cq), 135,5 (Cq), 141,4 (Cq), 169,8 (Cq).

### 3^{e} étape :

Finalement, le diester est saponifié avec 9,7 g d'hydroxyde de potassium (commercialisé par la société VWR) dans 100 mL d'éthanol 95% (commercialisé par la société SDS), au reflux pendant 5 jours. La solution est concentrée sous vide et le produit est solubilisé dans l'eau. De l'acide chlorhydrique concentré est ajouté jusqu'à pH 1, et un précipité blanc est formé. Il est récupéré par filtration, lavé à l'eau et séché. 5,3 g de diacide sont ainsi obtenus sous forme de solide blanc (rendement quantitatif).

### c) Synthèse C : synthèse de l'acide 3,3ʹ-dimethylbiphenyl 4,4'-dicarboxylique

Le schéma réactionnel de cette synthèse est représenté en figure 21.

La même procédure que celle décrite pour la synthèse B a été utilisée en partant de 12,1 g de diméthylbenzidine. A l'issue de la 1^{ère} étape, 18,4 g de 3,3'-diméthyl-4,4'-diiodo-biphényle sont obtenus (rendement: 74%).

Caractérisation du composé diiodé obtenu :

RMN ¹H (300 MHz, CDCl₃): δ (ppm) : 2,54 (s, 6H), 7,10 (dd, *J* = 2,2 et 8,1 Hz, 2H), 7,46 (d, *J* = 2,2 Hz, 2H), 7,90 (d, *J =,* 8,1 Hz, 2H). RMN ¹³C (75 MHz, CDCl₃): δ (ppm) : 28,3 (CH₃), 100,3 (Cq), 126,0 (CH), 128,3 (CH), 139,4 (CH), 140,4 (Cq), 141,9 (Cq).

A l'issue des 2^{e} et 3^{e} étapes, 6, 9 g d'acide 3,3'-dimethyl-biphenyl-4,4'-dicarboxylique sont obtenus à partir des 18,4 g de composé diiodé.

Caractérisation des composés obtenus :

Le diester obtenu à l'issue de la 2^{e} étape et le diacide obtenu à l'issue de la 3^{e} étape ont des signatures spectroscopiques identiques à celles décrites dans la littérature (Shiotani Akinori, *Z. Naturforsch.* **1994,** 49, 12, 1731-1736 [ref. 32]).

### d) Synthèse D : synthèse de l'acide 3,3ʹ-dichloro4,4ʹ-azobenzenedicarboxylique

15g d'acide o-chlorobenzoïque (commercialisé par la société Aldrich, 98%) et 50g de soude sont placés dans 225 mL d'eau distillée, et chauffés à 50°C sous agitation. 100g de glucose (Aldrich, 96%) dissout dans 150 mL d'eau sont ajoutés. Le mélange est agité 15 minutes, puis un bullage d'air est effectué pendant 3 heures, à température ambiante. Le sel de disodium est récupéré par filtration, lavé à l'éthanol, puis dissout à nouveau dans 120 mL d'eau. De l'acide chlorhydrique (commercialisé par la société Aldrich VWR, 37%) est ajouté jusqu'à obtenir un pH égal à 1. Le solide est récupéré par filtration et séché sous vide à 90°C.

### e) Synthèse E Synthèse de l'acide 3,3',5,5'-azobenzenetetracarboxylique

19g d'acide nitroisophthalique (commercialisé par la société Aldrich, 98%) et 50g de soude sont placés dans 225 mL d'eau distillée, et chauffés à 50°C sous agitation. 100g de glucose (Aldrich, 96%) dissout dans 150 mL d'eau sont ajoutés. Le mélange est agité 15 minutes, puis un bullage d'air est effectué pendant 3 heures, à température ambiante. Le sel de tetrasodium est récupéré par filtration, puis dissout à nouveau dans 300 mL d'eau. De l'acide chlorhydrique (commercialisé par la société Aldrich VWR, 37%) est ajouté jusqu'à obtenir un pH égal à 1. Le solide est récupéré par centrifugation (4000 rpm/10min) lavé a l'eau et séché sous vide à 90°C.

### Exemple 3 : Détermination du caractère flexible des matériaux MOF

Le caractère flexible des matériaux MOF carboxylates de Fe, Cr, V, Mn et/ou Co peut être déterminé par diffraction RX. Les paragraphes qui suivent indiquent une méthode qui peut être utilisée pour mettre en évidence la flexibilité éventuelle des matériaux MOF. En particulier, les inventeurs ont mis en évidence le caractère flexible de certains matériaux MOF dits « MIL-88 ».

A la lecture de ce qui suit, le lecteur sera en mesure d'adapter la méthode pour s'assurer lesquels des matériaux MOF comprenant une structure tridimensionnelle de motifs de formule (I) ont une structure rigide, et sont donc susceptibles d'être utilisés dans le procédé de l'invention.

La catégorie de solides hybrides flexibles à base de trimères de métaux de transition trivalents (Fe, Cr, V, Mn ...) est dénommée MIL-88. Ces composés sont typiquement construits à partir de trimères d'octaèdres de fer, c'est-à-dire trois atomes de fer connectés par un oxygène central et par six fonctions carboxylates connectant deux à deux les atomes de fer ; une molécule d'eau terminale, coordinée à chaque atome de fer vient ensuite compléter la coordinence octaédrique du métal. Ces trimères sont ensuite reliés entre eux par des acides dicarboxyliques aliphatiques ou aromatiques pour former les solides MIL-88A, B, C, D et MIL-89 (-A pour acide fumarique, -B pour acide téréphthalique, -C pour acide 2,6-naphtalenedicarboxylique, -D pour acide 4,4'-biphényidicarboxylique et MIL-89 pour l'acide trans, trans muconique), comme décrit dans le document de Serre et al., Angew. Chem. Int. Ed. 2004, 43, 6286 [ref. 17]. D'autres analogues avec d'autres diacides carboxyliques ont également été synthétisés et sont nommés MIL-88E, F, G...

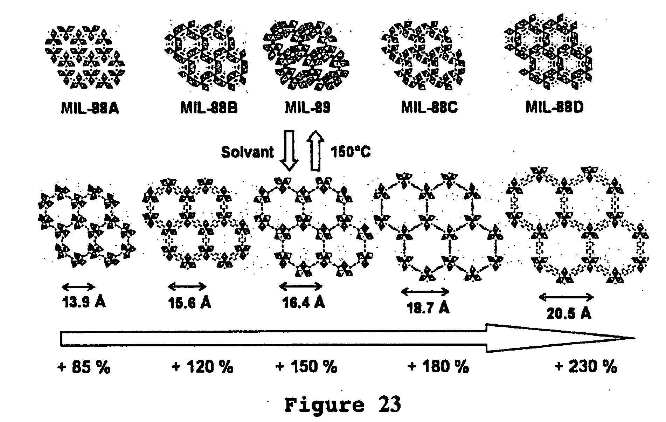

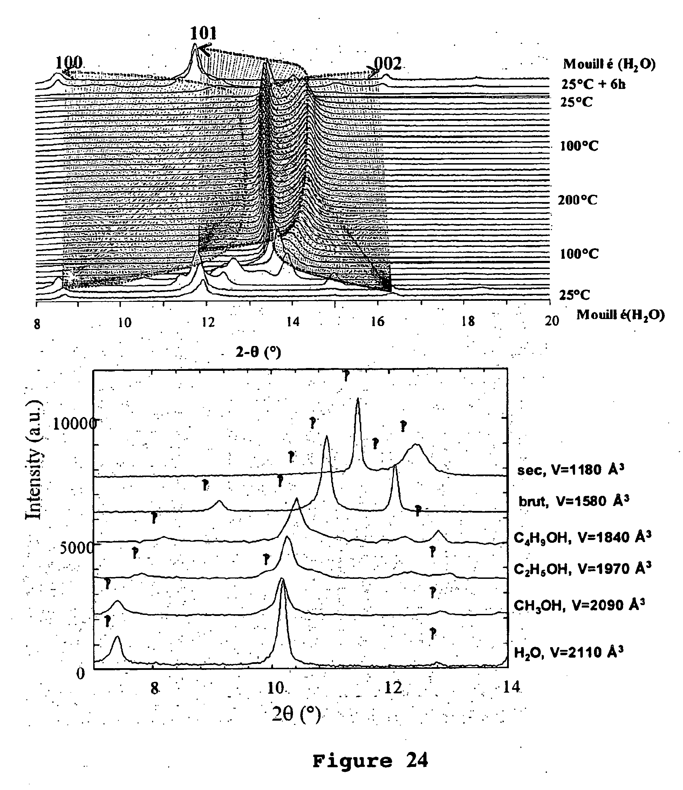

L'étude du comportement de ces solides par diffraction des RX, a permis d'établir que ces composés sont flexibles avec des amplitudes de « respiration » (c'est-à-dire de gonflement ou de contraction) considérables entre leur forme sèche et leur forme solvatée. Il en résulte des variations de volume de maille entre 85 et 230 % selon la nature de l'espaceur organique (figure 23), comme décrit dans le document Serre et al., Science, 2007, 315, 1828 [ref. 33]. Les inventeurs ont noté que les formes sèches ne sont pas poreuses avec une taille de pores (tunnels) à peu près identique quel que soit le ligand carboxylique utilisé. Par contre, le gonflement du solide hybride en phase liquide est fonction de la longueur de l'espaceur organique. Ainsi, la distance entre trimères dans la forme gonflée passe de 13,8 Å avec l'acide fumarique (MIL-88A) à 20,5 Å avec le ligand biphényle (MIL-88D). La taille des pores des formes gonflées varie ainsi entre 7 Å (MIL-88A) à 16 Å (MIL-88D). Le gonflement est réversible, comme le montre l'exemple du solide MIL-88A en présence d'eau dans la figure 24) et dépend également de la nature du solvant utilisé comme décrit dans le document Serre et al. J. Am. Chem. Soc., 2005, 127, 16273-16278 [ref. 34]. La « respiration » s'effectue de manière continue, sans rupture apparente de liaisons durant la respiration. Par ailleurs, de retour à température ambiante, le solide gonfle à nouveau par resolvatation, confirmant le caractère réversible de la respiration.

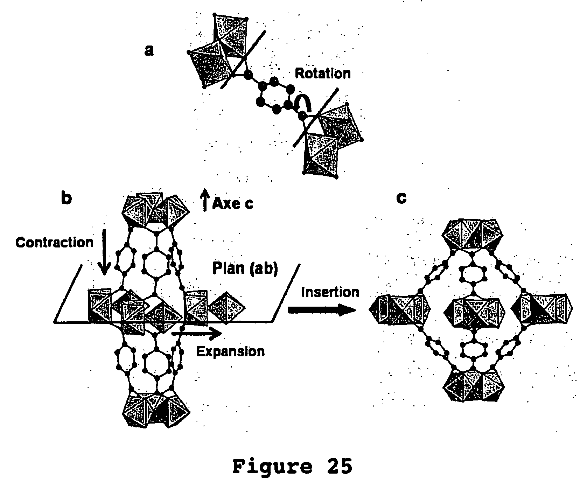

Si l'on regarde de près l'arrangement entre les trimères constitutifs de la structure, chaque trimère est relié à six autres trimères, trois en dessous et trois au dessus, par les dicarboxylates ce qui conduit à la formation de cages bipyramidales de trimères. Au sein de ces cages, la connexion entre trimères s'effectue uniquement selon l'axe c et l'absence de tout lien dans le plan (ab) est à l'origine de la flexibilité (figure 25).

**Tableau 10 :**

| **Solide** | **Condition** | **a(**Å**)** | **c(**Å**)** | **v(**Å**³)** | **Expansion de la maille** | **Taille de pore estimée** | **Solvant** |
|---|---|---|---|---|---|---|---|
| **MIL-88A** | 100°C | 9,6 | 14,8 | 1180 | > 80% | environ 6Å | Eau |
| | **25°C** | **11,1** | **14,5** | **1480** | | | |
| | Forme ouverte | 13,8 | 12,5 | 2100 | | | |
| **MIL-88B** | 100°C | 9,6 | 19,1 | 1500 | > 100% | environ 9Å | Ethanol |
| | **25°C** | **11,0** | **19,0** | **2000** | | | |
| | Forme ouverte | 15,7 | 14,0 | 3100 | | | |
| **MIL-88C** | 100°C | 9,9 | 23,8 | 2020 | > 170% | environ 13Å | Pyridine |
| | **25°C** | **10,2** | **23,6** | **2100** | | | |
| | Forme ouverte | 18.7 | 18,8 | 5600 | | | |
| **MIL-89** | 100°C | 9,15 | 20 | 1470 | > 160 % | Environ 11Å | Pyridine |
| | 25°C | — | — | — | | | |
| | Forme ouverte | 17,0 | 15,7 | 3900 | | | |
| **MIL-88D** | 100°C | 10,1 | 27,8 | 2480 | >220% | environ 16Å | Ethanol |
| | **25°C** | **12,1** | **27,5** | **3500** | | | |
| | Forme ouverte | 20,5 | 22,4 | 8100 | | | |

En effet, lorsque l'on insère un solvant dans le matériau, la cage se déforme avec un rapprochement des trimères selon l'axe c et un éloignement dans les directions a et b, ce qui provoque une augmentation globale du volume de la cage (figure 25). Finalement, la flexibilité de ces solides hybrides est remarquable, mais cependant comparable à celle de certains polymères. La principale différence concerne la cristallinité des solides hybrides, les polymères étant amorphes. Enfin, contrairement aux polymères, le gonflement se produit dans les solides hybrides de manière anisotrope.

**Tableau 11: Structures « MIL » de quelques carboxylates de fer(III).**

| **Solide MIL-n** | **Fraction organique** | **Formule** |
|---|---|---|
| **MIL-88A** | Acide fumarique | Fe₃OX[O₂C-C₂H₂-CO₂]₃.nH₂O |
| **MIL-88B_4CH3** | Acide téréphthalique | Fe₃OX[O₂C-C₆(CH₃)₄-CO₂]₃.nH₂O |
| **MIL-89** | Acide muconique | Fe₃OCl[O₂C-C₄H₄-CO₂]₃.nH₂O |
| **MIL-100** | Acide 1,3,5-Benzene tricarboxylique (Acide 1,4-BTC) | Fe₃OX[C₆H₃-[CO₂]₃].nH₂O |
| **MIL-101** | Acide téréphthalique | Fe₃OX[O₂C-C₆H₄.CO₂]₃.nH₂O |
| **MIL-102** | Acide 1,4,5,8 naphthalenetetracarboxylique | Fe₆O₂X₂[C₁₀H₂(CO₂)₄]₃ |

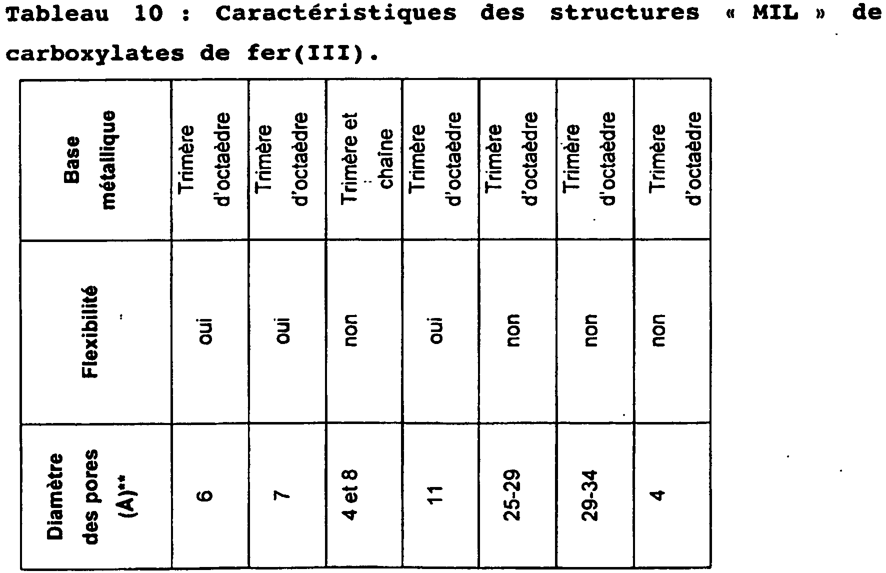

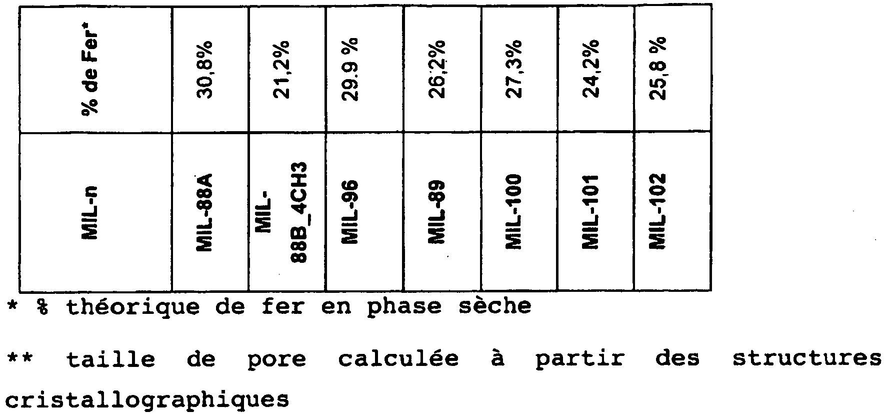

### Exemple 4 MIL-100(Fe).

Le matériau MOF carboxylate de fer MIL-100(Fe) est constitué de trimères d'octaèdres de chrome ou de fer relié par des acides trimésiques qui s'associent pour former des supertétraèdres hybrides. [ Horcajada et al., ref. 28] Le tout conduit ainsi à une structure mésoporeuse cristallisée, dont les cages de dimensions libres 25 et 29 Å, sont accessibles par des fenêtres microporeuses (figure 19). Le volume poreux résultant est très important, proche de 1.2 g.cm⁻³ pour une surface spécifique BET de 2200 m².g⁻¹.

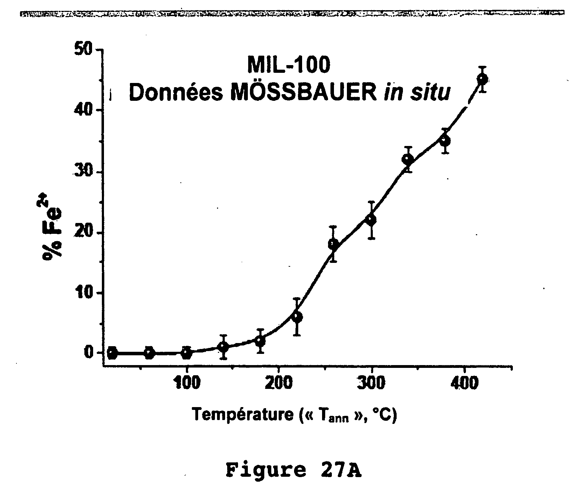

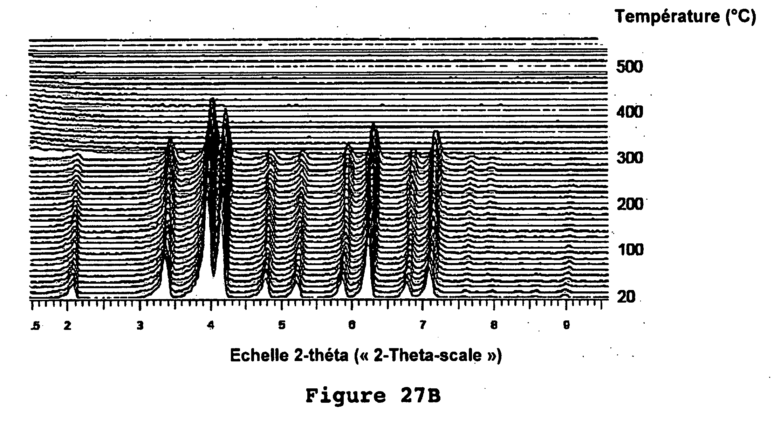

La particularité de ce solide est la stabilité de sa structure après le départ de l'eau coordinnée sur les centres métalliques. [ref. 35] Cette dernière est facilement évacuée par chauffage sous vide et laisse place à des centres métalliques insaturés et accessibles (métal en coordinence cinq). Par ailleurs, lorsque la température d'activation sous vide dépasse les 150°C, une réduction partielle du fer(III) en fer(II) se produit. Cette réduction se fait de manière croissante avec la température et ne déstabilise pas la structure avant 280°C, comme le montre la thermodiffractométrie RX sous vide (figure 27B). Il est possible de réduire le fer(III) en fer(II) à plus basse température que 150°C en laissant activer le solide suffisamment longtemps. Bien entendu, plus la température d'activation est élevée, plus on déshydrate vite et plus la réduction est rapide (effet cinétique). En effet, la séparation C3 est bien meilleure lorqu'on active le solide MOF 12 heures sous He à 150C par rapport à 3 heures. Quant au taux de réduction maximal à une temperature fixée, sans vouloir être lié en aucune façon à une théorie particulière, il semble pouvoir être avancé que celui-ci est sans doute contrôlé par la thermodynamique.

Le solide MIL-100(Fe) a pour composition Fe^{III}₃O(H₂O)₂F.{C₆H₃-(CO₂)₃}₂.nH₂O (n-14.5). Sa charpente est cationique avec un anion de compensation par trimère de fer. Ici, l'anion est un fluorure qui est coordinné sur le fer. La stabilité du MIL-100(Fe) à une réduction partielle du fer(III) en fer(II) pourrait s'expliquer par un départ de fluor conjugué à la réduction du fer. Ainsi, il est tout à fait raisonnable de penser qu'un fer(III) par trimère puisse être réduit en fer(II) en même temps qu'un départ des ions fluorures pour respecter l'électroneutralité. De retour à température ambiante, sous air, le solide se reoxyde avec une probable coordination d'anions OH sur le fer. Cette propriété est fondamentale et unique à notre connaissance dans le domaine des MOFs : la réduction sous vide d'un centre métallique insaturé tout en maintenant l'intégrité de la structure.

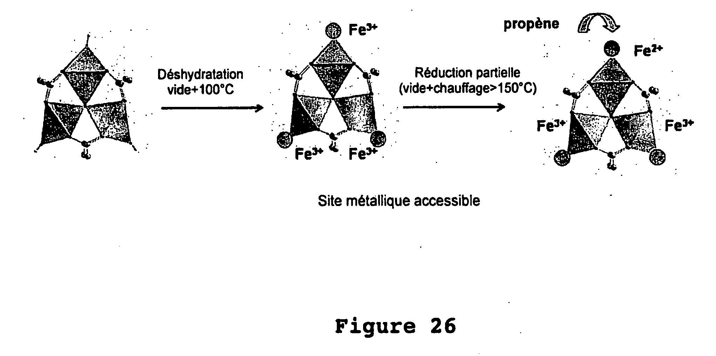

Les centres métalliques insaturés fer(II) et fer (III) possédent un caractère accepteur d'électrons (acide de Lewis) et forment des complexes π avec des molécules possédant un caractère donneur d'électrons (base de Lewis), tel que des alcènes ou des alcynes. La stabilisation des complexes ainsi formés peut être rationalisée d'après le modèle de Dewar-Chatt-Duncanson [ref. 9] en considérant d'une part la structure électronique de la double ou triple liaison carbone-carbone de l'oléfine et d'autre part des orbitales vacantes du site d'adsorption: la liaison entre l'alcène ou l'alcyne implique (i) une délocalisation des électrons des orbitales π liantes de l'hydrocarbure insaturé vers les orbitales vacantes du site d'adsorption (interaction donneur-accepteur par liaison σ) (ii) une délocalisation des electrons des orbitales d partiellement remplies du site d'adsorption vers les orbitales π* anti-liantes de l'hydrocarbure insaturé (liaison π). Le fer (II) possède un électron d supplémentaire par rapport au fer(III), ce qui renforce la liaison π avec l'hydrocarbure et accroit ainsi la stabilité du complexe formé. Ainsi, le MIL-100(Fe) partiellement réduit va pouvoir interagir plus fortement avec de telles molécules (figure 26).

### Exemple 5 : Détermination de la teneur en fer(II) dans le solide MIL-100(Fe) activé

### Activation :

Afin de vider les pores du matériau (solvants, acides résiduels) et de libérer les sites de coordination du métal, l'activation du matériau MIL-100(Fe) a été effectuée par chauffage à 150°C sous vide primaire pendant 15 heures. Le solide résultant ne possède que du fer au degré d'oxydation +III.

### Réduction Fe³⁺/Fe²⁺ :

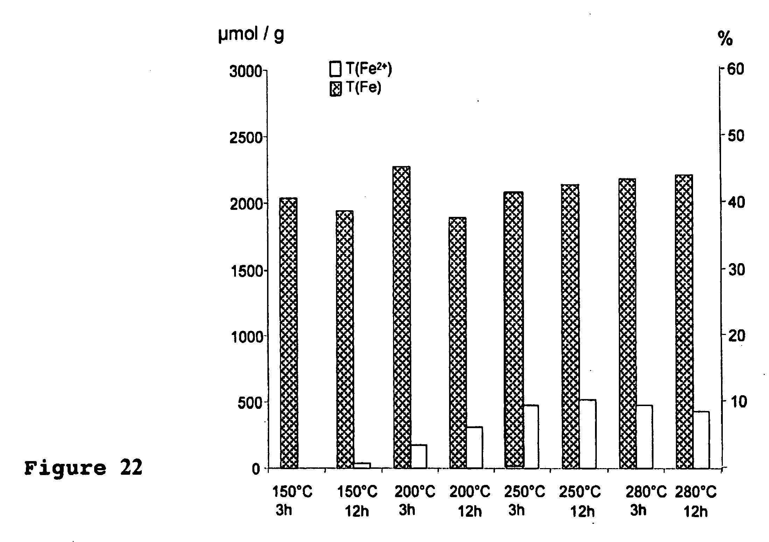

La réduction partielle du matériau MIL-100(Fe) a été effectuée par chauffage à 250°C sous vide primaire pendant 15 heures. La spectroscopie infrarouge a permis de quantifier la teneur relative en fer(II)/fer(III) autour de 20/80 % (figure 22).

La figure 22 représente la quantité de sites Fer coordinativement insaturés présent dans le solide MIL-100(Fe) activé en fonction du traitement thermique effectué. Le solide MIL-100(Fe) est activé sous vide résiduel (environ 0,0075*10⁻⁵ Pa (10⁻⁵ Torr)) à différentes températures et pendant différentes durées. T(Fe) représente la teneur en sites Fer coordinativement instaurés et T(Fe²⁺) représente la teneur en sites Fe²⁺ coordinativement insaturés (en µmole de sites insaturés par gramme de solide activé ou en % de sites Fer insaturés).

Les quantités de sites Fer insaturés sont déterminées par adsorption de CO à 100K suivie par spectroscopie infrarouge. L'incertitude sur les valeurs est estimée à +/-10 %.

### Conditions expérimentales utilisées pour les expériences de perçages et de type thermodésorption des Exemples 6 à 13.

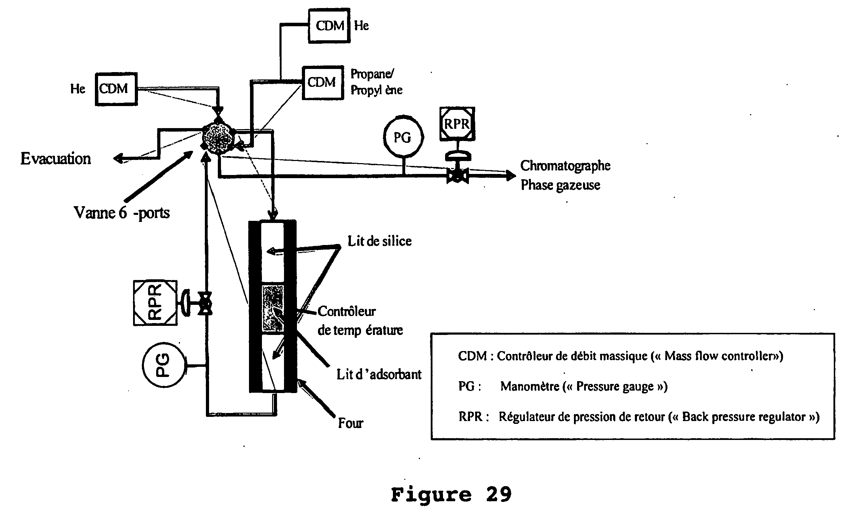

Les propriétés de séparation de gaz du solide MIL-100(Fe) ont été testées lors d'expériences de perçages utilisant des mélanges de C₃H₈/C₃H₆ et C₂H₄/C₂H₂ en proportion équimolaire avec l'hélium comme gaz porteur. 1.3 gramme de solide MIL-100(Fe) hydrate a été introduit et compacté dans une colonne en acier inoxydable (9.5 mm de diamètre externe et 20 cm de long). La colonne a été ensuite attachée à un appareil de séparation de gaz comme décrit figure 29. Préalablement aux tests de séparations, l'échantillon a été activé à la température requise (entre 120 et 300°C) sous un flux d'hélium de 30 ml/min pendant 10 heures. Ensuite, l'échantillon a été refroidi jusqu'à 40°C (ou autre température si spécifié) et maintenu à cette température pendant 2 heures. Pour les expériences à pression atmosphérique (1 bar), un mélange équimolaire de propane/propylène à 7.4 mol% dans de l'hélium a été introduit dans la colonne contenant l'adsorbant MIL-100(Fe) activé à un débit constant de 30 ml/minute. Les mêmes conditions ont été appliquées aux expériences à plus haute pression (5 et 8 bars). La composition en sortie de colonne a été analysée chaque minute par un chromatographe en phase gazeuse (Donam DS6200 GC) équipé avec un détecteur à flamme à ionisation et une colonne capillaire (J&W, Alumine, 30 m x 0.55 mm). La concentration en chaque gaz a été normalisée au même niveau lors de la purge du mélange de gaz (sortie spécifique) avant son passage au travers de la colonne. Après chaque expérience de perçage, des expériences de désorption thermoprogrammées (temperature-programmed desorption ou TPD) sur le solide chargé en mélange gazeux ont été effectuées sur le même appareil avec un flux d'hélium comme gaz vecteur avec une vitesse de chauffe de 10K/min de la température d'adsorption jusqu'à 300°C.

### Conditions expérimentales utilisées pour les expériences d'adsorption à l'équilibre thermodynamique par gravimétrie (Exemples 6 et 8)_

### Description du montage expérimental

L'étude d'un mélange binaire en coadsorption requiert une évaluation de la composition des deux gaz et des phases adsorbées pour chaque point à l'équilibre. Le montage utilisé ici est constitué de trois parties : un système de d'introduction de dose manométrique, un appareil gravimétrique associé avec des mesures de densité *in situ*, et un chromatographe en phase gazeuse, ce qui permet d'étudier la coadsorption de plusieurs types de gaz.

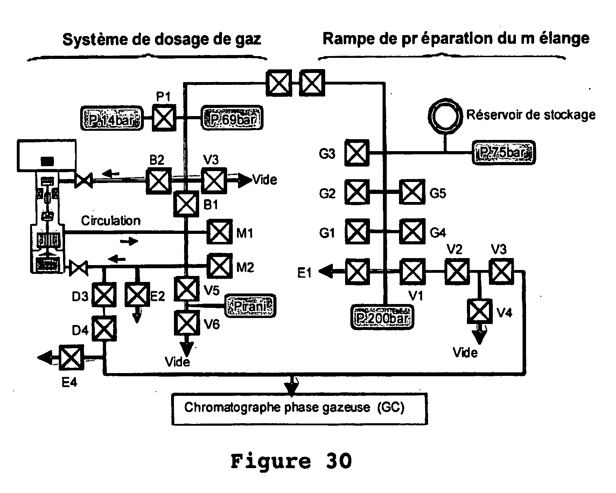

Une représentation schématique de l'appareil manométrique "fait-maison" est décrit Figure 30. Il comporte deux parties. Sur la première, on peut connecter jsuqu'à 5 bouteilles de gaz ce qui permet la préparation de mélanges complexes. Les gaz sont introduits séquentiellement dans une rampe et le mélange stocké dans un réservoir. Pour assurer l'homogénéité du mélange, un agitateur magnétique est placé dans le réservoir. Les gaz sont de qualité Standard (QS) (Air Liquide). Ensuite, une petite quantité de gaz est extraite via les valves V1, V2 et V7, pour aller ensuite vers le chromatographe afin d'analyser la composition.

Le mélange ainsi préparé peut être injecté vers le système de dosage de gaz au travers des valves D1 et D2. Les pressions inférieures à 10 bar sont détectées par la jauge P14 (Marque Mensor avec une sensibilité de 0.01% à pleine échelle), tandis que les pressions plus élevées, la P1 est fermée automatiquement pour protéger la jauge. Une fois dans le système de dosage, le mélange est introduit dans système gravimétrique. Pendant l'équilibrage, le gaz peut être transporté autour du système de dosage et à cette fin, les deux circulateurs utilisant un effet de piston et un contrôle électromagnétique. Le système de dosage peut fonctionner jusqu'à 50 bars. A chaque point d'équilibre, environ 0.8 cm³ de gaz peut être extrait et envoyé à nouveau au chromatographe. Finallement, les deux valves M1 et M2 permettent une possibilité supplémentaire d'envoyer le gaz vers un second appareil contenant le même échantillon, comme par exemple un microcalorimètre.

Le système de mesure gravimétrique a été acheté chez Rubotherm Präzisionsmeßtechnik GmbH. Ce système a été décrit précédemment [De Weireld et al., ref. 38; Dreisbach et al., ref. 39]. Dans le cas présent, le temps d'équilibre a été considéré comme satisfaisant lorsque la masse d'échantillon ne varie pas de plus de 80 mg sur une période de 5 minutes. A chaque point d'équilibre, les masses d 'échantillon et de charge sont toutes les deux enregistrées avant extraction vers le chromatographe. La température de l'expérience a été maintenue constante grâce à un cryo-thermostat avec une précision de 0.01 K.

Le système de dégazage utilise la méthode dite "Sample Controlled Thermal Analysis (SCTA)" permettant de dégazer in situ le solide jusqu'à une température (T<620 K) et une pression (0.02 mbar) déterminés pendant 16 heures [Sorensen et al., ref. 40].

Le chromatographe en phase gazeuse est de marque Agilent 3000, micro-cpg. Le gaz porteur a été ici l'hélium. A chaque point d'équilibre, le gaz extrait est introduit dans un volume 1 cm³ puis transmis au chromatographe. Le programme Cerity a permis de contrôler le système, de calibrer et d'analyser les résultats.

Dans cette étude, les expériences ont été réalisées à 303K et jusqu'à P=30 bar. Pour chaque point, il a été possible de mesurer m^{tot}, la quantité totale adsorbée n^{tot}, la pression totale p^{tot} et la densité de la phase gazeuse.

### Descriptif experimental pour les expériences infrarouges des Exemples 6, 11 et 14

### Préparation de l'échantillon

Les échantillons sont pressés sous forme de disques autosupportées. Le disque possède un diamètre de 1,6 cm et une masse de 13 à 20 milligrammes. La pression de pastillage est de l'ordre de 10⁹ Pa.

### Appareillages utilisés

L'échantillon pastillé est placé dans une cellule infrarouge conçue au laboratoire. La cellule peut être métallique pour les études sous flux gazeux (la description de la cellule est donnée dans l'article suivant T. Lesage, C. Verrier, P. Bazin, J. Saussey, M. Daturi, Phys. Chem. Chem. Phys. 5 (2003) 4435) soit en quartz pour les études sous vide ou à des pressions de gaz inférieures à la pression atmosphérique.

Les spectres infrarouges sont enregistrés grâce à un spectromètre infrarouge à transformée de Fourier de marque Nexus ® ou Magna-550 ® fabriqué par la société Thermo Fisher Scientific. Le spectromètre est équipée d'un détecteur infrarouge de type MCT/A. Les spectres infrarouges sont enregistrés avec une résolution de 4 cm⁻¹.

### Gaz utilisés

Les gaz utilisés pour les experiences infrarouges sont de grandes purétés :
Monoxyde de carbone: fournisseur alphagaz type N47 pureté (>99.997%)
monoxyde d'azote : fournisseur Air Liquide, France pureté >99.9%
Helium, azote, argon : fournisseur air liquide, purete >99.9%

Tous les gaz sont préalablement séchés sur tamis moléculaire et/ou par piégeage cryogénique grâce à de l'azote liquide. Le monoxyde d'azote est purifié par distillation.

### Exemple 6 : Isothermes d'adsorption de propane et de propylène.

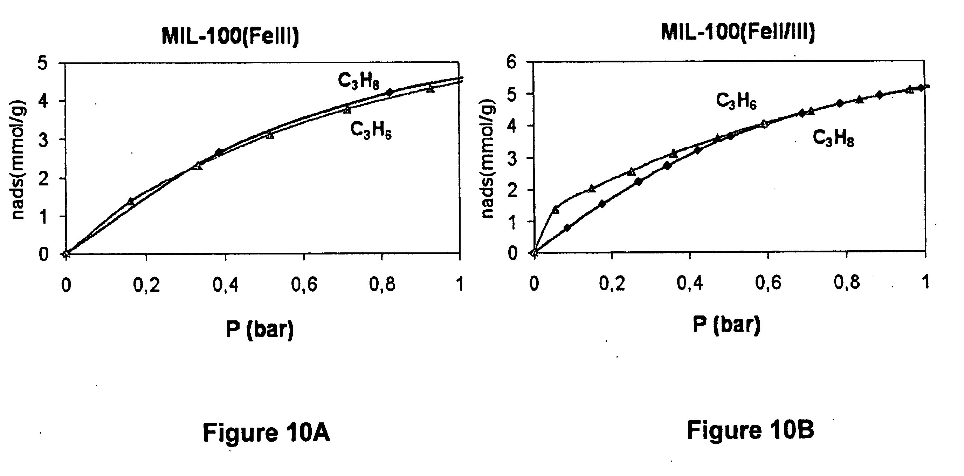

Des mesures d'adsorption de propane et de propylène ont été réalisées à l'équilibre thermodynamique (figure 10) à température ambiante à partir de 1 g de solide MIL-100(Fe). Une première mesure a d'abord été réalisée sur le solide activé à 120°C sous vide primaire (P=1 Pa pendant 16 heures) suivi d'une seconde mesure sur le même solide mais activé à plus haute température (260°C sous vide primaire (P=1 Pa pendant 16 heures). La spectroscopie IR a montré (exemple 5) que l'activation de ce solide à 120°C sous vide (ou sous flux d'hélium) conduit à un solide ne contenant que du fer au degré d'oxydation + III tandis qu'une activation à 260°C sous vide (ou sous flux d'hélium) conduit à un solide contenant à la fois du fer(III) et du fer(II) (proportion Fe(II)/Fer(III) de 20 % environ). En l'absence de réduction du fer (activation à 120°C), il n'y a pas de différence dans les isothermes d'adsorption pour le propane et le propylène tandis que le solide partiellement réduit (activation à 260°C) présente une différence d'adsorption significative à faible taux de recouvrement (P <0.4 bar) avec une quantité de propylène adsorbée nettement supérieure, ce qui traduit une affinité plus importante du fer(II) pour le propylène.

### Exemple 7: tests de séparation

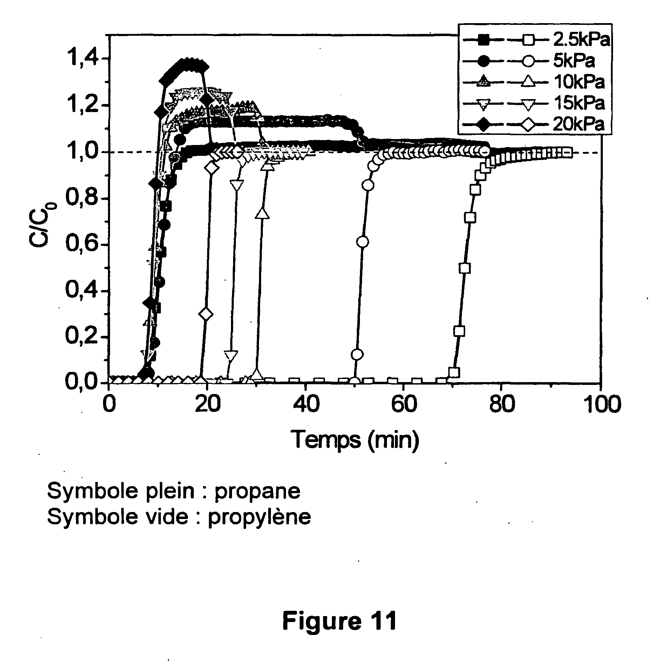

Des tests de séparation dynamique (flux d'hélium, % de propane= % propylène =3.7 mol %) à partir d'un mélange 50/50 propane/propylène ont été ensuite réalisés sur des colonnes (dimensions : 2 mètres * 0,3125 cm (1/8^{ème} de pouce)) chargées avec 1 gramme de solide MIL-100(Fe) préalablement activé sous flux d'hélium à 280°C (P= 100000 Pa, 16 heures) à différentes pressions totales (1, 3 et 5 bars) (figure 11).

Il existe une différenciation très nette entre comportement du propane et du propylène. Le propane n'est pratiquement pas retenu dans la colonne, au vu de son faible temps de rétention alors que les temps de rétention du propylène sont beaucoup plus élevés. De surcroît, la différence de temps de rétention entre les deux gaz est maintenue lorsque la pression augmente, ce qui indique que la sélectivité apparente est maintenue dans cette gamme de pression.

### Exemple 8: influence de la température à l'équilibre thermodynamique

Des mesures d'adsorption de propane et de propylène (flux d'hélium, % de propane= % propylène =3.7 mol %) à deux températures différentes (40 et 100 °C) ont été réalisées à l'équilibre thermodynamique (figure 10) sur le solide MIL-100(Fe) activé à 250°C sous vide primaire (P=1 Pa pendant 16 heures) (figure 12).

### Exemple 9: influence de la température en séparation sur colonne.

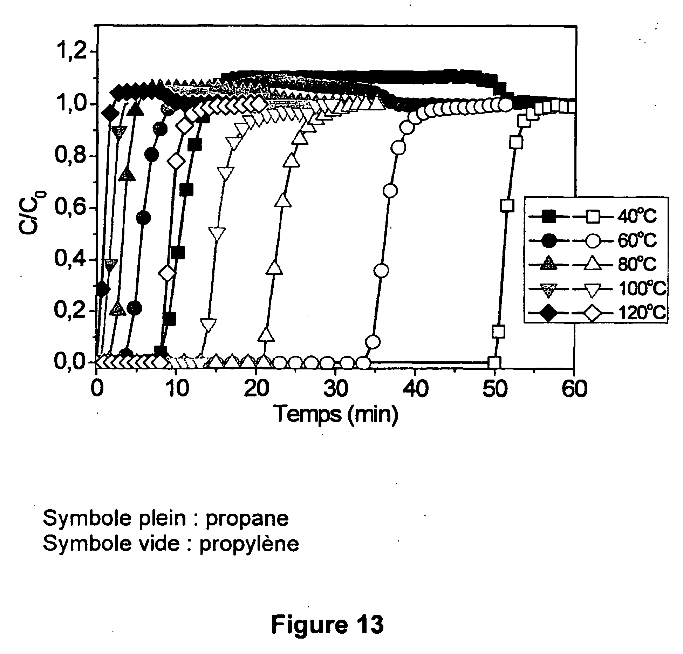

L'influence de la température sur la séparation propane/propylène (flux d'hélium, % de propane= % propylène =3.7 mol %) a été étudiée en colonne (dimensions : 2 mètres * 0,3125 cm (1/8^{ème} de pouce)) sur un gramme de solide MIL-100(Fe) partiellement réduit sou flux d'hélium à 280°C (P= 10000 Pa, 16 heures) (figure 13). Si les temps de rétention des deux gaz diminuent comme prévu avec la température qui augmente, il est intéressant de voir la différenciation entre temps de rétention faible pour le propane et élevés pour le propylène est maintenue même à 120°C.

### Exemple 10: tests de cyclabilité

Afin de vérifier que l'intégrité du solide MIL-100(Fe) est maintenue après les tests de séparation, le test de séparation dynamique (flux d'hélium, % de propane= % propylène =3.7 mol %) à partir d'un mélange 50/50 propane/propylène a été répété à 10 reprises, dans les mêmes conditions, sur une colonne (dimensions : 2 mètres * 0,3125 cm (1/8^{ème} de pouce)) chargée avec 1 gramme de solide MIL-100(Fe) préalablement activé sous flux d'hélium à 280°C (P=10000 Pa, 16 heures). Le solide a été réactivé entre chaque expérience par un passage d'hélium à 200°C entre chaque cycle d'adsorption (P= 10000 Pa, 16 heures). Les courbes de perçage se superposent de manière tout à fait satisfaisante (figure 14), ce qui indique une très bonne cyclabilité de d'adsorbant MIL-100(Fe) pour des mélanges propane/propylène sous pression.

### Exemple 11: influence de la teneur en fer(II)

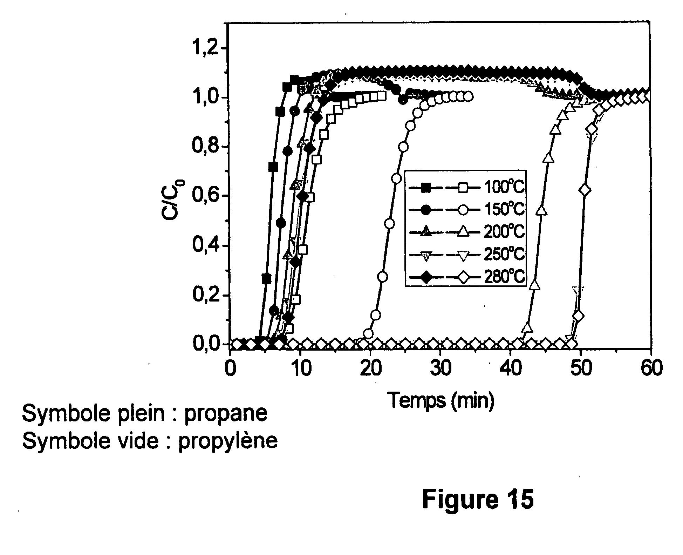

L'influence de la teneur en fer(II) sur les propriétés de séparation dynamique de mélanges propane/propylène (flux d'hélium, % de propane= % propylène =3.7 mol %) a été testée sur colonne (dimensions : 2 mètres * 0,3125 cm (1/8^{ème} de pouce)) chargée avec 1 gramme de solide MIL-100(Fe), activé sous flux d'hélium à différentes températures, entre 100°C et 300°C (figure 15). Rappelons que le taux de fer(II) variait presque linéairement avec la température d'activation (sous vide) lorsque celle-ci est supérieure à 150°C (cf exemple 5, figure 22).

Le solide activé à 100°C, ne contenant pas ou très peu de fer(II), ne présente pas de différence significative dans les temps de rétention du propane et du propylène, ce qui confirme les résultats obtenus à l'équilibre thermodynamique (exemple 6). Le solide activé à plus haute température (150-280°C), présente une augmentation avec la température de la différence entre les temps de rétention du propane et du propylène, ce qui est en accord avec une augmentation de l'affinité du MIL-100(Fe) pour le propylène suite à l'augmentation de la teneur en fer(II) dans le solide MIL-100(Fe). La sélectivité entre propane et propylène varie en fonction de la température d'activation de ce matériau. L'activation du solide à 300°C ne montre pratiquement pas de différence entre les temps de rétention pour propane et propylène et donc une absence de sélectivité. La thermodiffraction RX sous vide a montré préalablement (cf figure 4) que la charpente du MIL-100(Fe) se dégrade au-delà d'une température d'activation de 280°C (vide primaire), ce qui explique l'absence d'adsorption dans ces conditions.

### Exemple 12: régénération

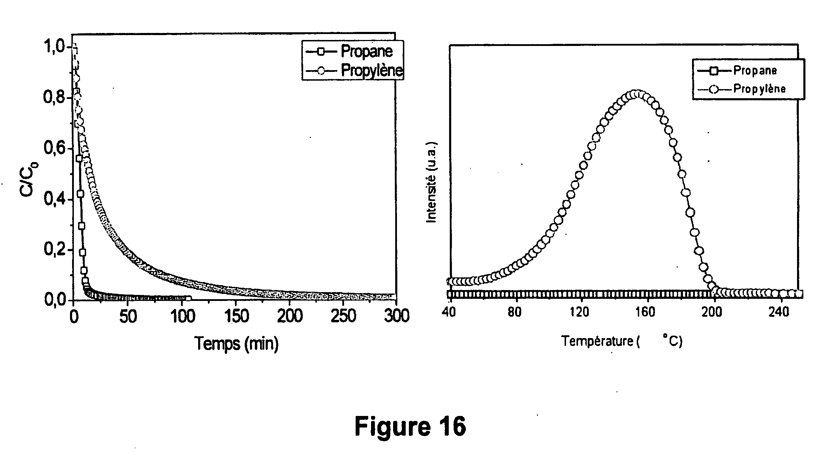

La régénération du solide MIL-100(Fe), activé à 280°C sous flux d'hélium, après adsorption sur colonne (dimensions : 2 mètres * 0,3125 cm (1/8^{ème} de pouce)) du mélange propane/propylène (flux d'hélium, % de propane= % propylène =3.7 mol %) a été étudiée par thermodésorption. Le propane présent en très faible quantité, est désorbé à basse température (<100°C). Le propylène, espèce la plus fortement adsorbée, est libéré entre 70 et 200°C (figure 16), ce qui confirme une interaction plus forte entre le MIL-100(Fe) partiellement réduit et le propylène.

### Exemple 13: séparation par pulse de mélanges d'hydrocarbures gazeux.

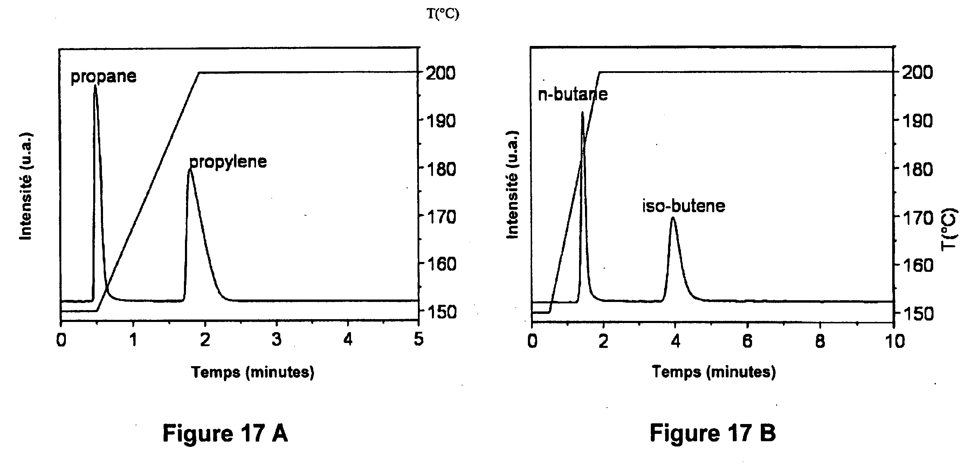

Des mélanges 50/50 propane/propylène et 50/50 n-butane/iso-butène dans un gaz porteur (1 % en poids dans l'azote) ont été introduits sous forme d'un pulse à température ambiante dans une colonne (dimensions : 2 mètres * 0,3125 cm (1/8^{ème} de pouce)) chargée avec 1 gramme de solide MIL-100(Fe) préalablement réduit sous flux d'hélium à 280°C (P = 10000 Pa, durée : 16 heures). La proportion en chaque gaz a été analysée par chromatographie gazeuse en sortie de colonne. Pour ces deux mélanges, il apparaît que les alcanes présentent des temps de rétentions plus faibles que les alcènes avec une différence qui augmenté avec la taille de l'oléfine (figure 17). En effet, le mélange n-butane/iso-butène (en pulse) est séparé de la même manière que le mélange propane/propylène, mais avec des temps de rétentions plus élevés, en accord avec des interactions plus fortes générées typiquement par un hydrocarbure à quatre carbones par rapport à un hydrocarbure à trois carbones [réf. 43: (a) Denayer, J. F. M.; De Meyer, K. ; Martens, J. A.; Baron, G. V. Angew. Chem., Int. Ed. 2003, 42, 2774-2777. (b) Denayer, J. F. M. ; Ocakoglu, R. A.; Arik, I. C.; Kirschhock, C. E. A.; Martens, J. A.; Baron, G. V. Angew. Chem., Int. Ed. 2005, 44, 400-403].

En outre, la quantité relative de propane et de propène adsorbée dans le solide MIL-100(Fe) a été déterminée en fonction de la température d'activation de ce solide (même conditions, sous flux d'hélium, P=10000 Pa, 16 heures) (figure 15). Il apparaît clairement que la proportion relative propène/propane est fonction croissante de la température, entre 100 et 280°C, avec un maximum de propène retenu dans le solide lorsque la température d'activation est de 280°C ; à 300°C, en accord avec une destruction de la charpente, le solide n'adsorbe presque plus de propane et de propène.

### Exemple 14 : faible acidité de Brønsted.

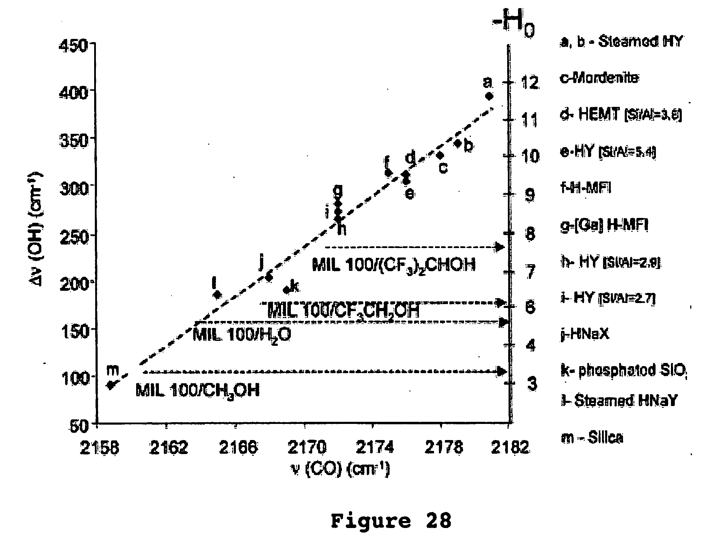

Des études préalables sur un système MIL-100(Cr) ont montré la présence d'un grand nombre de sites acides de Lewis (Vimont et al., ref. 35 (a)), qui sont transformés en sites acides de Brønsted par adsorption d'eau. L'ajout de petites doses calibrées de CO a confirmé l'existence de deux types de molécules d'eau de force acide similaire, globalement assez faible. L'introduction d'alcools de basicité différente a précisé que la force des sites acides de Brønsted dépend de la nature de la molécule coordinnée et croit avec la basicité de l'espèce protique (Figure 28).

Des études complémentaires ont montré que le solide MIL-100(Fe) est un peu moins acide (acidité de Brønsted) que le solide MIL-100(Cr) (cf. Vimont et al. réf. 35(b)).

Ainsi, bien que le solide MIL-100 possède des centres métalliques insaturés (Fer, Vanadium) qui sont des centres acides de Lewis, même si ceux-ci peuvent se transformer en acidité de Brønsted par coordination de molécules donneur de proton telles que l'eau (Vimont et al., ref. 35 (b)(, l'acidité mesurée par adsorption de CO dans le matériau MOF homologue MIL-100(Cr) n'étant pas très forte, il est attendu qu'il en est de même pour MIL-100(Fe). Cette acidité de Brønsted n'est pas suffisante pour déclencher ou provoquer une éventuelle polymérisation d'hydrobarbures insaturés (par exemple le propylène) dans les pores du matériau MOF. Des tests de non polymérisation du propène en fonction de la température confirment l'absence de polymérisation pour des températures inférieures à 200°C.

### Exemple 15:-séparation acétylène/éthylène

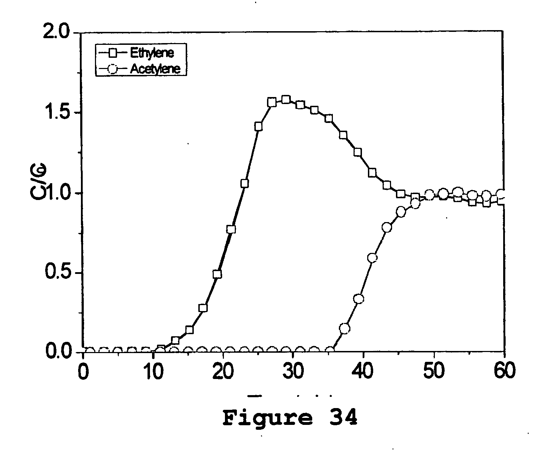

Il est observé en début de séparation (temps <20 minutes) que l'acétylène sort plus tard de la colonne que l'éthylène, ce qui traduit une plus grande rétention de l'alcyne par rapport à l'alcène (figure 34). A temps plus long (t> 20 minutes), les deux espèces sont retenues de la même manière traduisant une compétition d'adsorption.

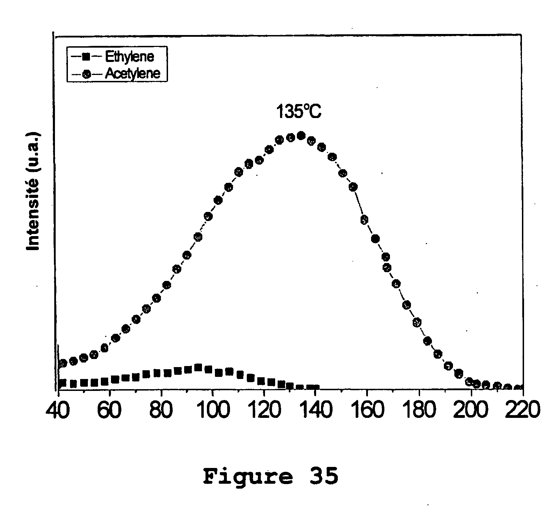

Pour comprendre la différence de comportement entre acétylène et éthylène, une expérience de thermodésorption (désorption en fonction de la température) a été réalisée (figure 35) en prenant le solide MIL-100(Fe) après passage du mélange gazeux acétylène/éthylène dans les conditions définies ci-dessus.

### Exemple 16: courbe de thermodésorption acétylène/éthylène

Nous observons ici qu'une partie importante de l'acétylène adsorbé dans le MIL-100(Fe) quitte le matériau à des températures supérieures à l'éthylène, même si une proportion non négligeable de l'acétylène est désorbée en même temps que la majorité de l'éthylène (figure 35). Cela traduit une hétérogénéité des sites d'adsorption. Sans doute les sites d'adsorption forts interagissent par effet de rétrodonation su l'alcyne et correspondent aux sites insaturés Fe(II) et les sites acides de Lewis, Fe(III), adsorbent avec la même force d'interaction l'alcène et l'alcyne.

### Exemple 18 : séparation acétylène/éthylène

L'expérience de l'exemple 15 a été répétée en utilisant des conditions expérimentales différentes : à savoir, 0.5 g de solide MIL-100(Fe) dans un tube de diamètre 3/8" (au lieu de 0.15g dans un tube de 1/8") (Figure 34).

Il est observé en début de séparation (temps <35 minutes) que l'acétylène sort plus tard de la colonne que l'éthylène, ce qui traduit une très grande rétention de l'alcyne par rapport à l'alcène. A temps plus long (t> 40 minutes), les deux espèces sont retenues de la même manière traduisant une compétition d'adsorption (Figure 34).

### Exemple 19 : courbe de thermodésorption acétylène/éthylène

Pour comprendre la différence de comportement entre acétylène et éthylène, une expérience de thermodésorption (désorption en fonction de la température) a été réalisée en prenant le solide MIL-100(Fe) après passage du mélange gazeux acétylène/éthylène dans les conditions définies ci-dessus (Figure 35).

La figure 35 indique qu'une partie très importante de l'acétylène adsorbé dans le MIL-100(Fe) quitte le matériau à des températures supérieures à l'éthylène (T=40-200°C avec un maximum de désorption à 135°C), tandis que la faible proportion d'acétylène adsorbée intialement est désorbée à plus basse température (T=40-140°C avec un maximum de désorption à 90°C). Cela traduit à la fois une bonne sélectivité acétylène/éthylène mais aussi une hétérogénéité des sites d'adsorption. Sans doute les sites d'adsorption forts interagissent par effet de rétrodonation su l'alcyne et correspondent aux sites insaturés Fe(II) et les sites acides de Lewis, Fe(III), adsorbent avec une moins bonne sélectivité l'alcyne et l'alcène.

### Exemple 17: séparation par pulse de mélanges d'hydrocarbures gazeux C4.

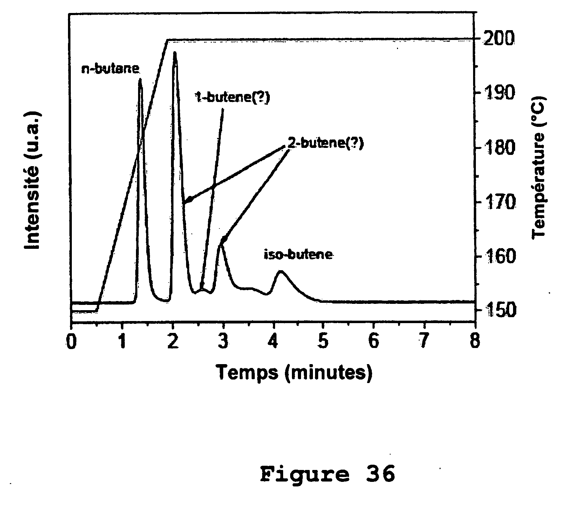

Des mélanges 50/50 propane/propylène et 50/50 n-butane/isobutène dans un gaz porteur (1 % en poids dans l'azote) ont été introduits sous forme d'un pulse à température ambiante dans une colonne (dimensions : 2 mètres * 0,3125 cm (1/8^{ème} de pouce)) chargée avec 2.2 gramme de solide MIL-100(Fe) préalablement déshydraté et réduit sous flux d'hélium à 280°C (P = 10000Pa, durée : 16 heures). La proportion en chaque gaz a été analysée par chromatographie gazeuse en sortie de colonne. Pour ce mélange, il apparaît que non seulement l'alcane (butane) est l'espèce la moins retenue dans la colonne mais qu'il est également possible de séparer les différents isomères du butène, avec des différences de temps de rétention significatives (Figure 36).

### Exemple 18 : Isothermes d'adsorption de CO et CO₂.

Des mesures d'adsorption de CO et de CO₂ ont été réalisés à l'équilibre thermodynamique (figure 37) à température ambiante à partir de 1 g de solide MIL-100(Fe). Une première mesure a d'abord été réalisée sur le solide activé à 100°C sous vide primaire (P=1 Pa pendant 16 heures) suivi d'un seconde mesure sur le même solide mais activé à plus haute température (260°C sous vide primaire (P=1 Pa pendant 16 heures). En l'absence de réduction du fer (activation à 100°C), les quantités de CO₂ adsorbées sont largement supérieures à celles de CO tandis que le solide partiellement réduit (activation à 260°C) présente une augmentation très importante de la quantité de CO, et une augmentation plus faible de celle de CO₂. A basse pression (P<0,2 bar), la quantité de CO adsorbée est même supérieure ou égale à celle de CO₂.

### Exemple 19 : sélectivité en fonction de la température d'activation du MIL-100(Fe).

A partir des résultats des tests de séparation dynamique (flux d'hélium, % de propane= % propylène =3.7 mol %) avec un mélange 50/50 propane/propylène sur colonne (2 mètres * 0,3125 cm (1/8^{ème} de pouce)) et 1 gramme de MIL-100(Fe) activé sous flux d'hélium à différentes températures °C, des calculs de sélectivité ont été réalisés (figure, 38).

Le calcul a été fait à partir des quantités de gaz désorbées lors des expériences de thermodésorption. En considérant que ces deux gaz se comportent en gaz parfaits, la sélectivité est le ratio de la quantité molaire de propylène/ quantité totale en moles de propane et propylène (référence 46 : Newalkar, B. L.; Choudary, N. V.; Turaga, U. T.; vijayalakshimi,R. P.; Kumar, P.; Komarneni, S.; Bhat, S. G. T. Chem. Mater.2003, 15, 1474).

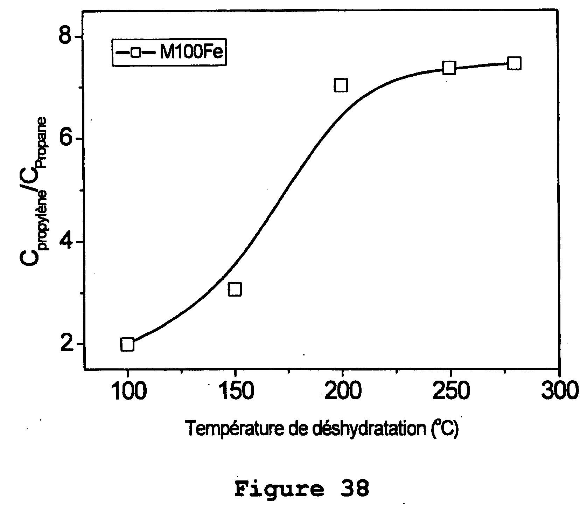

La teneur en propène ainsi que la sélectivité (ratio propène/propane adsorbé) augmente entre 1 et 4,8 avec la température d'activation, avec un maximum à 280°C (figure 38). Au-delà, le matériau se dégrade et n'est plus du tout sélectif.

### Exemple 20 : « Pré-activation » du solide MOF MIL-100(Fe) par traitement préalable avec NH₄F

Le solide MOF MIL-100(Fe) est préparé selon l'Exemple 1)a).

Le solide est dispersé dans une solution 1 mol/l de solution aqueuse de NH₄F à 70°C pendant 24 heures et immédiatement filtré à chaud et lavé avec de l'eau chaude. Le solide est finalement séché pendant une nuit à 100°C dans un four.

Ce mode de « pré-activation » est applicable à l'ensemble des solides MOF décrits dans la présente demande.

Le solide est ensuite soumis à l'étape d'activation usuelle, par exemple par chauffage à 250°C pendant 3 à 12 heures sous vide secondaire.

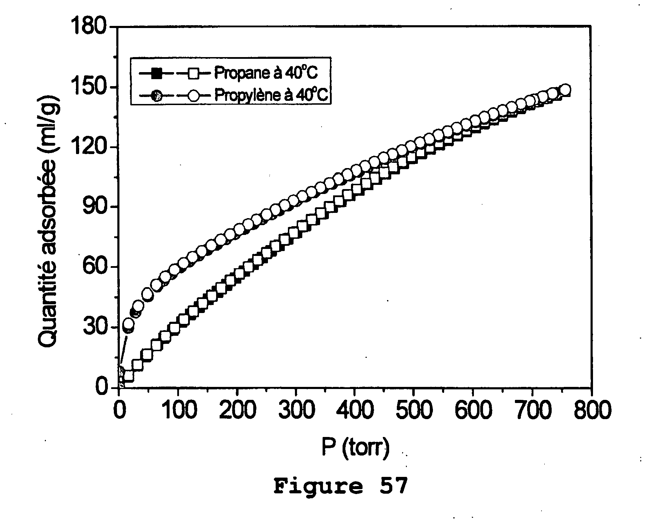

Les propriétés et performances de séparation du solide MOF MIL-100(Fe) préalablement traité au NH4F ont été étudiées (figures 57 à 67).

Les isothermes d'adsorption propane/propylène à 40°C à partir du solide MIL-100(Fe) prétraité avec NH₄F sont données en figure 57. Le solide a été activé 12 heures à 250°C sous vide secondaire et a une surface spécifique S_{BET}=2340 m²/g.

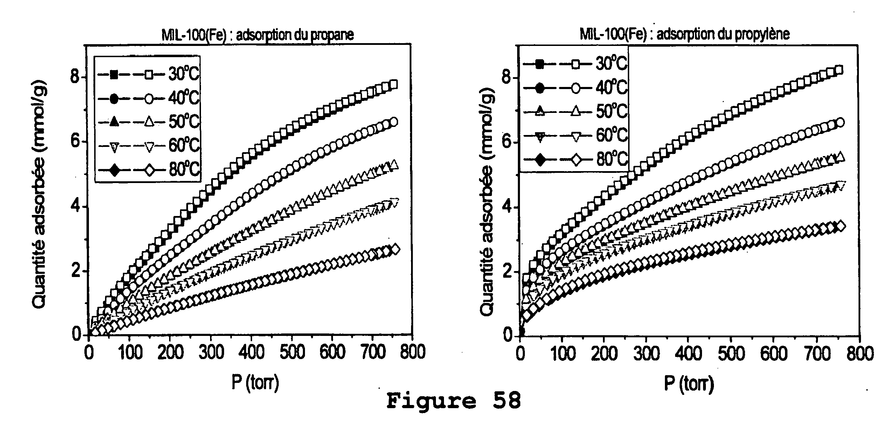

La figure 58 montre les isothermes d'adsorption propane (figure de gauche), propylène (figure de droite) à différentes températures. Il s'agit du MIL-100(Fe) prétraité avec NH₄F, activé à 250°C sous vide secondaire (12 heures) .

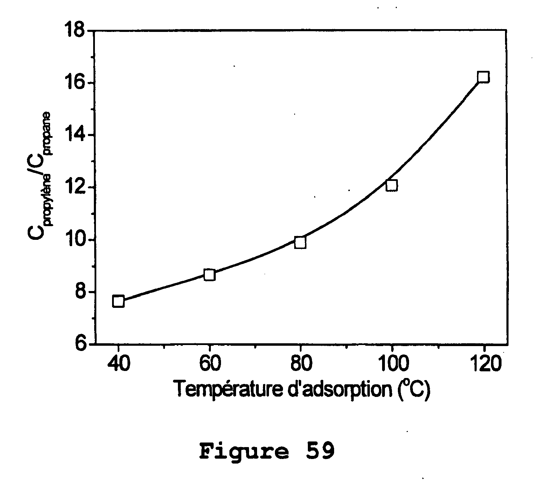

La figure 59 montre la sélectivité de la séparation propane/propylène, à P(partielle)=5 kPa, en fonction de la température, pour un mélange équimolaire propane/propylène dans un flux d'hélium sur du MIL-100(Fe) prétraité avec NH4F, activé à 250°C sous hélium (3 heures). Il est observé qu'à pression partielle fixe de 5 kPa, la sélectivité est une fonction croissante de la température et passe d'environ 7.8 à 40°C à 16.2 à 120°C.

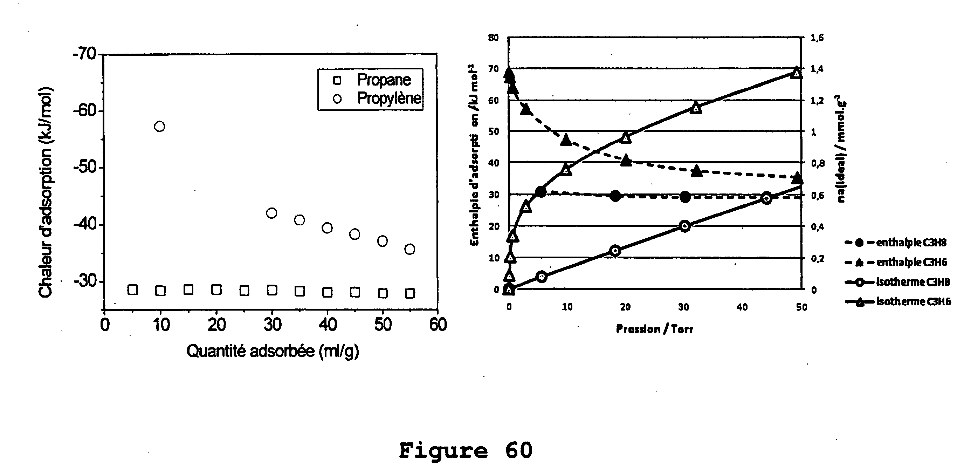

La figure 60 montre les chaleurs d'adsorption de propane et de propylène pour le solide MIL-100(Fe) prétraité avec NH4F, et dégazé 12h à 250°C sous vide secondaire. Figure de gauche : valeurs calculées à partir des isothermes figure 58 (méthode Clausius Clapeyron) ; figure de droite : Isothermes d'adsorption et chaleurs d'adsorption de propane et propylène à 303K dans le solide MIL-100(Fe) après activation à 250°C sous vide secondaire pendant 12 heures.

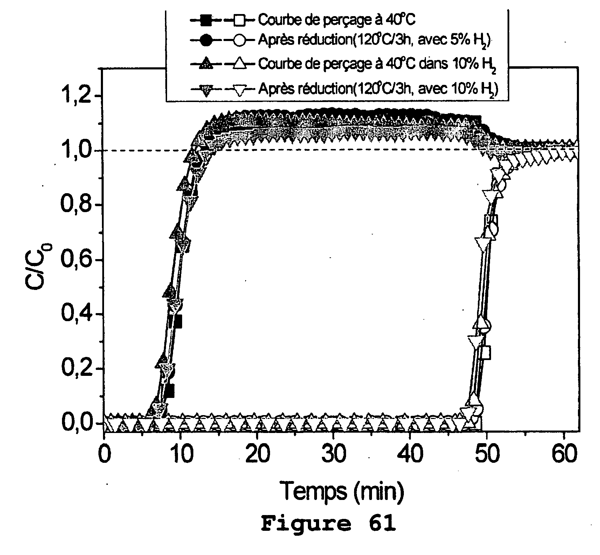

La figure 61 montre l'effet de l'activation 3 heures sous flux hydrogène (à 10 ou 20 % dans de l'hélium) sur la séparation propane/propylène pour le solide MIL-100(Fe) prétraité avec NH4F. L'hydrogène est souvent présent dans les flux gazeux industriels et est capable de réduire dans les silices dopées en ions Ag⁺ ou Cu⁺ en Argent ou Cuivre métallique, et donc désactiver l'adsorbant. Dans le cas d'espèce, après traitement à 120°C sous flux contenant une proportion importante d'hydrogène, aucune différence en termes de sélectivité propane/propylène n'est observée, avant et après activation ou adsorption en présence d'hydrogène, indiquant que cet adsorbant ne se désactive pas en présence d'hydrogène.

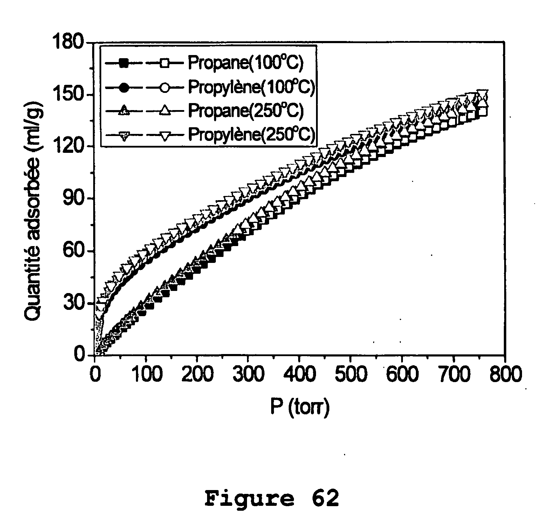

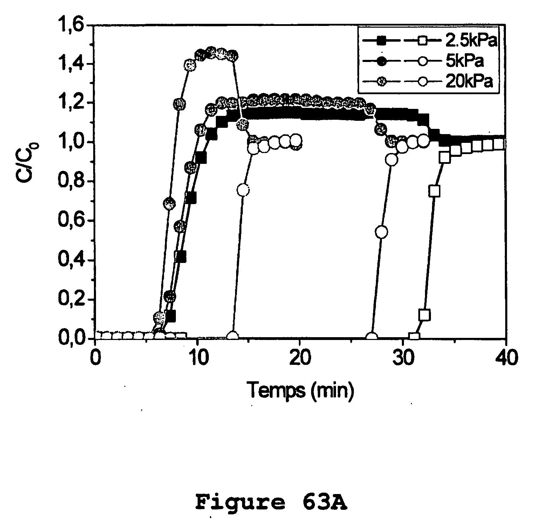

Un adsorbant ne peut pas être utilisé en séparation ou adsorption des gaz sous forme de poudre. Il est alors important de mettre en forme l'adsorbant sous forme de granulats ou d'extrudats. Cela conduit souvent à une modification sensible des propriétés d'adsorption. Dans le cas du MIL-100(Fe) prétraité avec NH₄F, des pastilles de ce solide ont été faites à partir d'une presse typiquement utilisée pour faire des pastilles pour la spectroscopie Infra-rouge (pression de 75 Kgf/cm² pendant 2 minutes). La surface spécifique des granulats ainsi formés est de 1950 m2.g-1 (surface BET) et leur taille de 1 mm. Les courbes de perçages propane/propylène sur le solide activé à 250°C sous flux d'hélium pendant 3 heures sont similaires à celles obtenues à partir de la poudre du même échantillon, confirmant ainsi que les propriétés de séparation du MIL-100(Fe) ne changent pas après mise en forme (figure 62). La figure 63A montre les courbes de perçage propane/propylène à 40°C obtenus à partir d'un mélange équimolaire dans l'hélium, à différentes pressions partielles, et du solide MIL-100(Fe) prétraité avec NH₄F dégazé 3 heures sous flux d'hélium à 250°C (S_{BET} = 2300 m²/g), et mis en forme sous la forme de granulats (Taille de particules: 1 mm ; S_{BET}: 1900 m²/g).

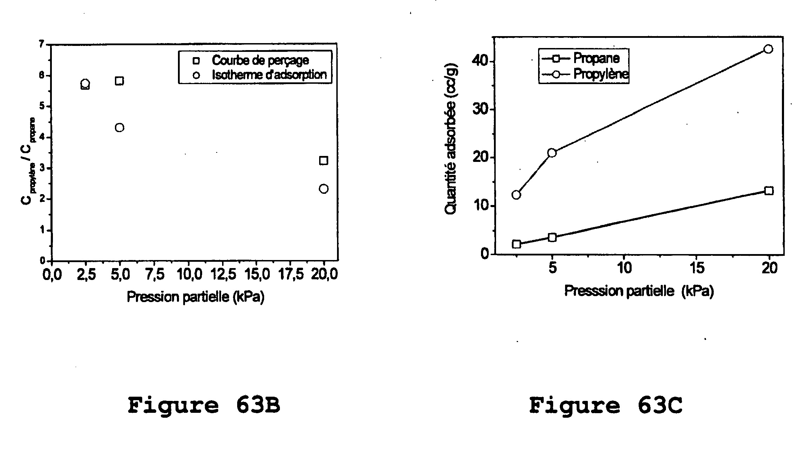

La figure 63B montre une comparaison de sélectivités propylène/propane obtenues avec le solide mis en forme à partir des isothermes d'adsorption (solide activé sous vide pendant 12h à 250°C) ou des courbes de perçages (solide activé sous flux d'hélium pendant 3h à 250°C).

La figure 63C montre des isothermes d'adsorption propane/propylène à 40°C obtenus à partir du solide mis en forme.

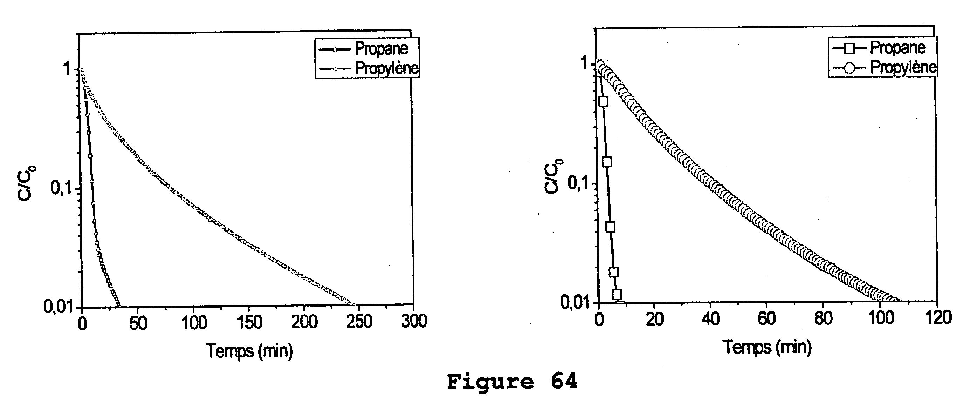

La figure 64 montre l'effet de la régénération du solide MIL-100(Fe) prétraité avec NH₄F, dégazé une nuit sous flux d'hélium à 250°C pendant 3 heures. Un mélange propane/propylène à pression de 5Kpa est passé à 40°C sur la colonne contenant le MIL-100(Fe) activé, jusqu'à saturation de l'adsorbant. Le solide est ensuite rénégéré sous flux d'hélium à 40°C sous un débit d'hélium de 30 cc/min. Comme le montrent les courbes de désorption à 40°C, le propane est désorbé totalement en moins de 50 minutes tandis que le propylène est évacué totalement en 250 minutes.

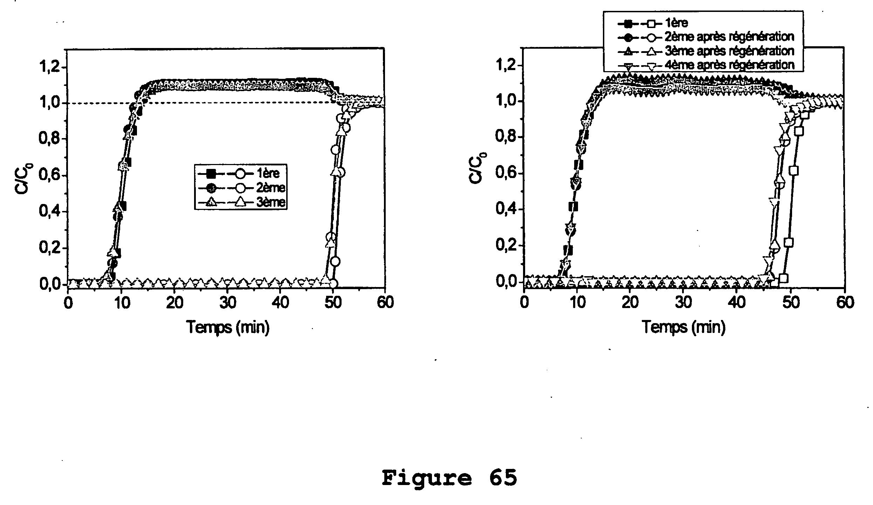

La figure 65 montre des courbes de perçages répétitives avec régénération sous hélium (solide MIL-100(Fe) prétraité avec NH4F), avec un flux de 30 cc/min. à 200°C pendant 1 heure (figure de gauche) ou de 100 ml/min. à 40°C pendant 2 heures (figure de droite). Mis à part une légère perte de sélectivité (ou de capacité d'adsorption) pour le propylène après la première régénération, il est clair que la régénération sous flux d'hélium est très efficace.

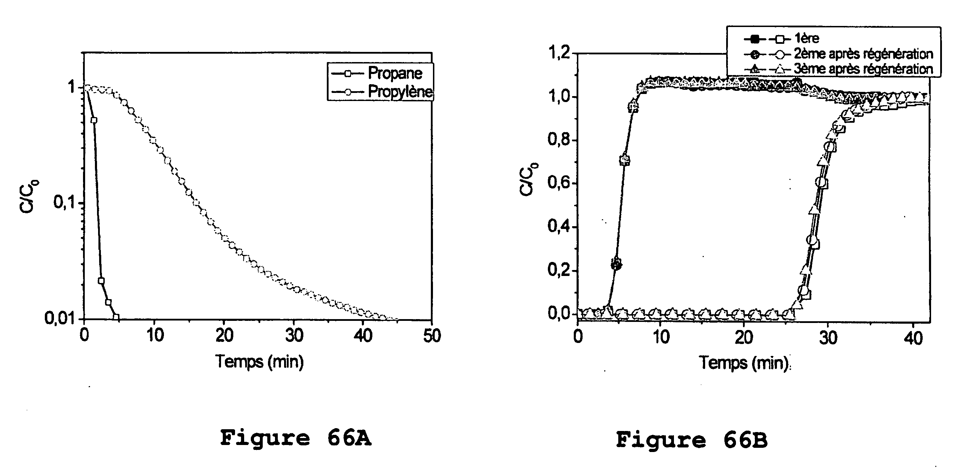

La figure 66 montre l'effet de la régénération du solide MIL=100(Fe) prétraité avec NH4F, dégazé une nuit sous flux d'hélium à 250°C pendant 3 heures. Un mélange propane/propylène à pression de 5 Kpa est passé à 80°C sur la colonne contenant le MIL-100(Fe) activé, jusqu'à saturation de l'adsorbant. Le solide est ensuite rénégéré sous flux d'hélium à 80°C sous un débit d'hélium de 100 cc/min. Comme le montrent les courbes de désorption à 80°C (figure de gauche), le propane est désorbé totalement en moins de 5 minutes tandis que le propylène est évacué totalement en 45 minutes. Augmenter la température et le débit de l'hélium permet donc de réduire de manière significative la durée de régénération de la colonne de MIL-100(Fe). La figure de droite montre ensuite des courbes de perçages répétitives avec régénération sous hélium (solide MIL-100(Fe) prétraité avec NH4F), avec un flux de 100 cc/min. à 80°C pendant 2 heures. Aucune perte de sélectivité (ou de capacité d'adsorption) n'est observée tant pour le propylène que pour le propane après la première régénération, il est clair que la régénération sous flux d'hélium à 80°C pendant 2 heures est très efficace.

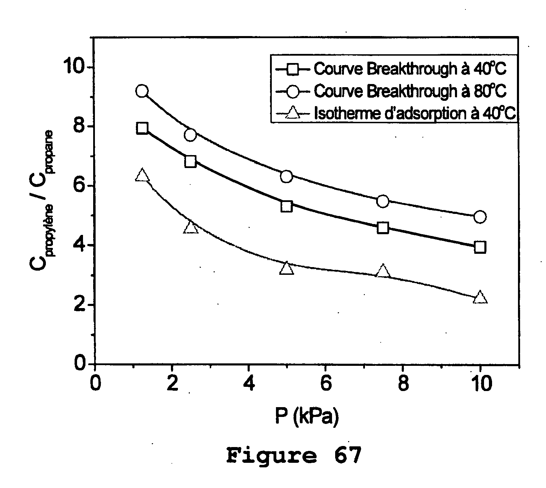

La figure 67 montre les sélectivités propylène/propane mesurées à partir des données en courbes de perçage (à 40 et 80°C) de mélanges propane/propylène à différentes pressions partielles dans l'hélium à partir du solide MIL-100(Fe) prétraité avec NH4F, et activé sous flux d'hélium pendant 3h à 250°C. A titre de comparaison, les sélectivités déduites des isothermes d'adsorption (à 40°C) obtenues à partir du même solide, activé sous vide secondaire à 250°C pendant 12 heures, sont rajoutés. Il est observé tout d'abord que les sélectivités déduites des courbes de perçages sont légèrement supérieures à celles obtenues à partir des isothermes d'adsorption en corps pur, et d'autre part qu'augmenter la température permet d'augmenter les valeurs des sélectivités propylène/propane.

### Exemple 21 : isothermes d'adsorption de propane-propène dans le CPO-27(Ni)

La synthèse du solide CPO-27(Ni) ou Ni₂[C₆H₂(OH)₂-(CO₂)₂](H₂O)₂.8H₂O a été rapporte dans « Hydrogen adsorption in a nickel based coordination polymer with open metal sites in the cylindrical cavities of the desolvated framework », P.D.C. Dietzel, B. Panella, M. Hirscher, R. Blom, H. Fjellvag, Chem. Commun., 2006, 959-961 [ref 53].

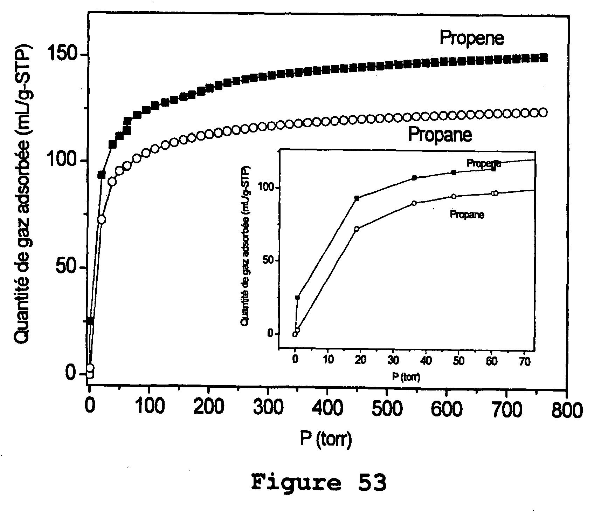

Des mesures d'adsorption de propane et de propylène ont été réalisés à l'équilibre thermodynamique (figure 53) à 40°C à partir de 1 g de solide CPO-27(Ni) préalablement activé à 250°C sous vide primaire (P=1 Pa pendant 16 heures)

Le profil des isothermes d'adsorption de propane et propylène montre une petite différence entre les quantités de gaz adsorbées, 125 et 150 mL/g pour le propane et propylène, respectivement (figure 53). La sélectivité propane-propylène a une valeur de 10 à basse pression (1 torr). Par contre, à des pressions supérieures à 15 torr la sélectivité passe à 1.5.

### Exemple 22 : isothermes d'adsorption de propane-propène dans le CPO-27(Fe)

Des mesures d'adsorption de propane et de propylène peuvent être réalisés à l'équilibre thermodynamique à 40°C à partir de 1 g de solide CPO-27(Fe) préalablement activé à différentes températures (20, 50, 100, 150, 200, 250°C) sous vide primaire (P=1 Pa pendant 16 heures)

Le profil des isothermes d'adsorption de propane et propylène attendu sera très similaire à celui obtenu pour le composé au Ni (montré dans l'exemple précédent), avec une sélectivité propane-propylène plus élevée à basses pressions et des valeurs plus importantes à pressions plus élevées. Considérant l'effet de rétrodonation du fer, une meilleure séparation propane-propylène est attendue.

### Exemple 23 : isothermes d'adsorption de propane-propylène dans le MIL-127(Fe)

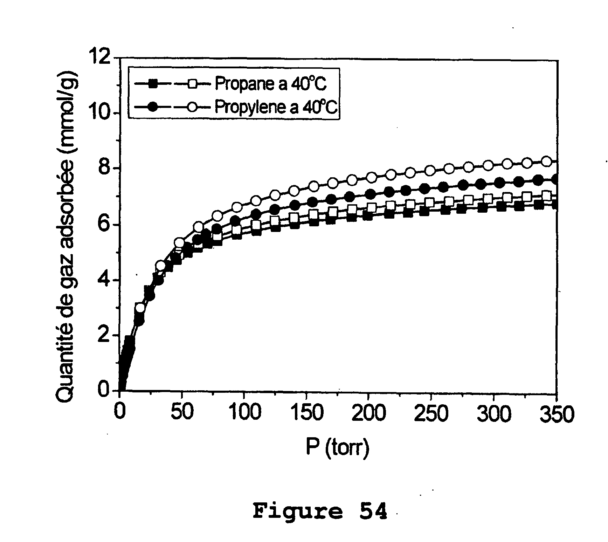

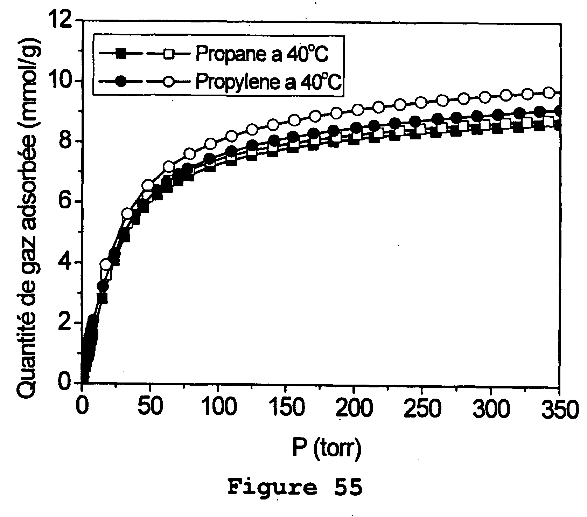

Des mesures d'adsorption de propane et de propylène ont été réalisés à l'équilibre thermodynamique à 40°C à partir de 1 g de solide MIL-127(Fe) préalablement activé à 150 (figure 54) et 250°C (figure 55) sous vide primaire (P=1 Pa pendant 16 heures).

Le profil des isothermes d'adsorption de propane et propylène sont très similaires, tant dans le matériau dégazé à 150°C comme à 250°C. La sélectivité propane-propylène observée est très faible (de 1.2). La sélectivité ne montre pas de changements significatifs avec la pression.

### Exemple 24. Comparaison de la réduction des différents matériaux MIL-100(Fe) fluorés activés par deux méthodes, MIL-100(Fe) non fluoré et MIL-127(Fe)

La réduction des différents solides a été déterminée par l'adsorption de gaz CO par infrarouge, selon l'exemple 5. Ainsi, la réduction a été étudiée sur les solides traités sous vide primaire à différentes températures (de 150 à 250°C) pendant des temps variables (3 ou 12 h).

Les matériaux suivants ont été comparés:
a) solide MIL-100(Fe) fluoré (X=F ; figure 22) préalablement prétraité dans l'eau ou dans l'éthanol sous reflux pendant 3 heures
b)solide MIL-100(Fe) non fluoré (X=OH,Cl) préalablement prétraité dans l'eau ou dans l'éthanol sous reflux pendant 3 heures
c) solide MIL-100(Fe) fluoré prétraité dans une solution de NH₄F
d) solide MIL-127(Fe)

Pour le prétraitement c), le solide est dispersé dans une solution 1 mol/l de solution aqueuse de NH₄F à 70°C pendant 24 heures et immédiatement filtré à chaud et lavé avec de l'eau chaude. Le solide est finalement séché pendant une nuit à 100°C dans un four.

Cette étude a permis de comparer, sur la même structure, l'influence de la présence ou pas de fluor dans la synthèse du MIL-100(Fe) ou Fe3O[C6H3-(CO2)3]2.X.nH2O, donc solide MIL-100(Fe) fluoré (X=F ; figure 22) ou non fluoré (X=OH,Cl), les deux préalablement prétraités dans l'eau ou dans l'éthanol sous reflux pendant 3 heures, et le MIL-100(Fe) fluoré prétraité dans une solution de NH4F.

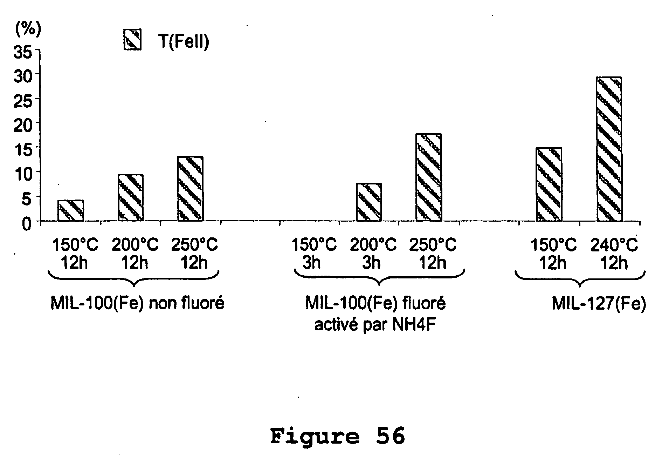

Comme discuté dans l'exemple 5, la température d'activation a une influence claire sur la réduction du fer. Comme le montre la figure 56, une température optimale autour de 250°C (sous vide pendant 12h) a été observée tant pour les matériaux MIL-100(Fe) comme pour le MIL-127.

Le traitement avec NH₄F s'est révélé plus efficace, en obtenant des valeurs de réduction plus importantes (autour de 18%), que dans le solide MIL-100(Fe) avec ou sans F prétraité sous reflux dans l'eau (autour de 13-15%).

De plus, le solide MIL-127 a montré une réduction du fer très élevée (proche au 30%) quand il est activé à 240°C sous vide.

### Exemple 25: Microcalorimétrie et isothermes d'adsorption de propane/ propylène et CO/CO2 dans le MIL-100(Fe).

Des mesures d'adsorption et de chaleurs d'adsorption de propane/propylène et CO/CO2 ont été réalisées à l'équilibre thermodynamique à température ambiante à partir de 0.4 g de solide MIL-100(Fe) activé à 100 ou 250°C (montée linéaire de 0.2 °C/min puis palier de 12h à la température finale) sous vide secondaire (10-5 mbar,). Les Expériences ont été effectuées avec des doses de gaz jusqu'à une pression de 1 bar.

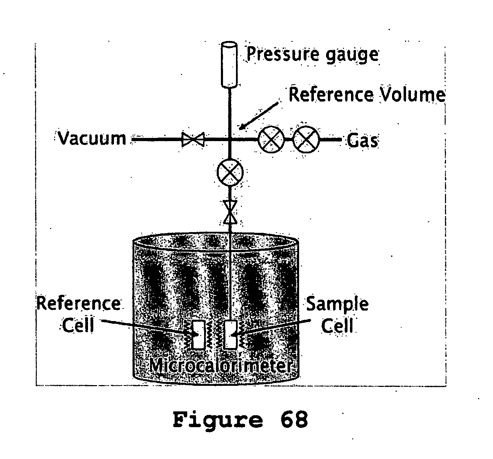

L'appareil utilisé pour la réalisation de ces mesures a été décrit dans P. L. Llewellyn & G. Maurin, C.R.Chimie, 8, 283-302 (2005) [ref 55]. Le microcalorimètre d'adsorption en phase gazeuse, fait maison est représenté schématiquement dans la Figure 68, est couplé avec un appareil volumétrique d'adsorption de gaz (Micromeritics ASAP 2010). Ainsi, les mesures d'adsorption de gaz et de microcalorimétrie sont réalisées simultanément.

### Propane/Propylène

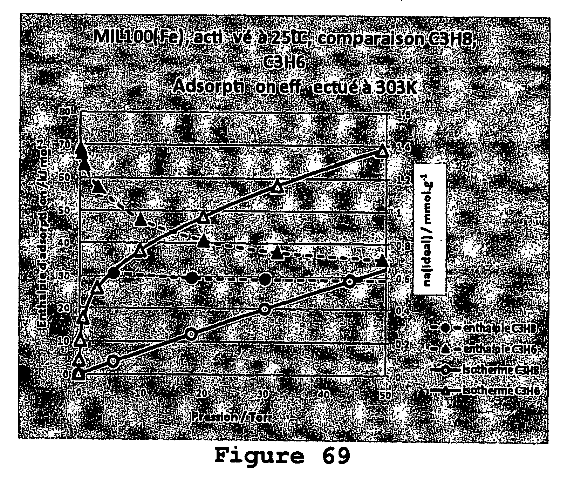

Le matériau MIL100(Fe) se réduit partialement après le traitement à 250°C sous vide et montre une bonne sélectivité propane/propylène sur toute la gamme de pression explorée (Figure 69). Ainsi, à basse pression (5 Torr) et à une valeur de 7.5 Torr en diminuant avec la pression croissante (4 à 20 Torr ou 3 à 30 Torr). Les enthalpies d'adsorption du propylène sont supérieures à celles observées pour le propane (-30 KJ/mol), avec cependant une différence d'énergie très marquée aux basses pressions (-65 KJ/mol), en accord avec une interaction beaucoup plus forte du propylène avec les sites métalliques insaturés Fe2+ par rapport au propane.

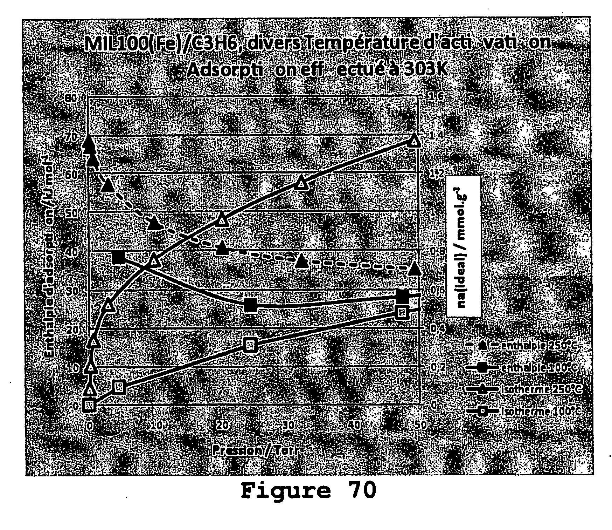

L'influence de la température de prétraitement sur l'adsorption de propylène ainsi que sur les chaleurs d'adsorption a été étudiée (Figure 70). Le solide MIL-100(Fe) activé à 250°C (sous vide primaire pendant 16 heures) adsorbe une quantité beaucoup plus importante de propylène que le matériau activé dans les mêmes conditions à une température de 100°C (vide primaire, 16 heures), en accord avec des chaleurs d'adsorption plus élevées lorsque la température d'activation augmente. Cela est dû à la présence de sites métalliques insaturés Fe²⁺ qui interagissent plus fortement avec le propylène, par effet de rétrodonation, que les sites métalliques insaturés Fe³⁺.

### CO/CO2

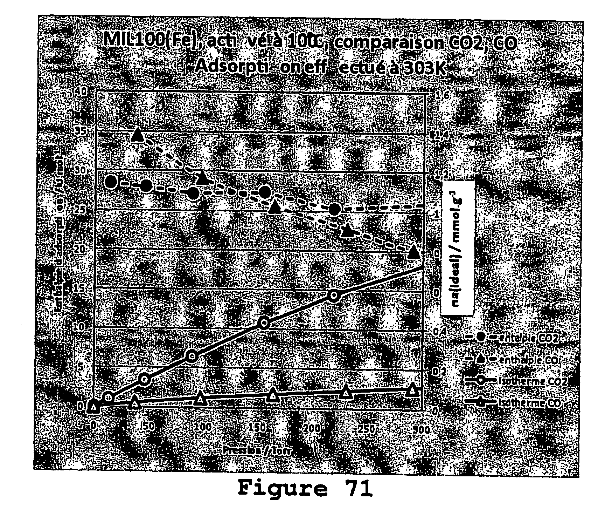

Le matériau MIL-100(Fe) après activation sous vide à 100°C montre une sélectivité CO2/CO d'environ 7 (Figure 71). Les enthalpies d'adsorption du CO2 (28.4 kJ/mol) sont cependant légèrement inférieures à celles pour le CO (34.5 kJ/mol) car au contraire du CO qui n'interagit qu'avec les sites métalliques insaturés, le CO2 peut interagir avec un plus grand nombre de sites (métalliques, cycles aromatiques, oxygènes des carboxylates...).

Le Tableau 13 montre les enthalpies d'adsorption en kJ/mol extrapolées à taux de recouvrement nul pour quantifier uniquement les interactions gaz - solide (Erreur sur la mesure +/- 0.2 kJ/mol)

**Tableau 13**

| Gaz | 100°C | P mesure / Torr | 200°C | P mesure / Torr | 250°C | Pmesure / Torr |
|---|---|---|---|---|---|---|
| CO2 | 28.4 | 13.6 | 34.2 | 7.6 | 33.6 | 33.6 |
| CO | 34.5 | 37 | 49.6 | 7.0 | 50.5 | 4.2 |
| C3H8 | | | | | 30.9 | 5.6 |
| C3H6 | 38.5 | 4.6 | | | 68.7 | 0.1 |

### Exemple 26: influence de la nature du ligand sur la propriété de rétrodonation des sites FeII

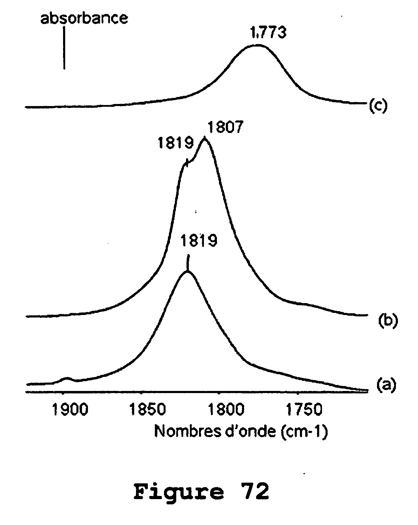

La position de la bande d'élongation vNO des espèces nitrosyles coordinnée sur les sites coordinativement insaturés Fe(II) rend compte de la force de l'interaction entre la molécule NO et le site métallique. Plus le nombre d'onde est petit, plus l'interaction impliquant de la rétro donation sera forte (A.A. Davidov, Infrared Spectroscopy of Adsorbed Species on the Surface of Transition Metal Oxides, Wiley Interscience, 1990 p123-130 chapter 2 [ref 58]). Cette interaction peut être modulée par la nature du ligand organique (Structure and nuclearity of active sites in Fe-zeolites: comparison with iron sites in enzymes and homogeneous catalysts Adriano Zecchina, Mickaël Rivallan, Gloria Berlier, Carlo Lamberti, Gabriele Ricchiardi, Phys. Chem. Chem. Phys., 2007, (27), 3483-3499 [ref 56]). La figure 72 montre que la position de la bande vNO des espèces nitrosyles sur Fer(II) est fonction de la structure et du ligand utilisé. Pour le MIL-127(Fe), la valeur plus faible de la bande NO rend compte d'un effet de rétro donation plus important que sur les autres solides.

### Exemple 27: influence de la teneur en FeII sur la purification d'un mélange H2/CO2/CO.

Le solide MIL-127(Fe) a été pastillé dans une cellule infrarouge puis chauffé sous flux d'argon (20 cc.min) pendant 3 heures à 150°C ou 250°C.

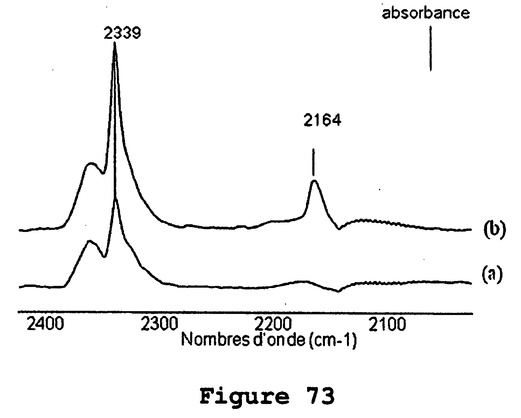

Le solide a ensuite été soumis à un flux (20 cc.min) composé d'argon (48% vol), de H2(50%), de CO₂(1%) et de CO(1%) à 30°C. La figure 73 montre que le CO2 est adsorbé sur le solide activé à 150 et 250°C (bande à 2339 cm-1) alors que seule l'activation à 250°C donne lieu à l'adsorption de CO à la surface (bande à 2339 cm-1). La position de la bande v(CO) (2164 cm-1) est caractéristique de CO coordinné sur les sites FeII. Ce résultat montre que le solide sous un flux de H2/CO2/CO possède une plus forte affinité vis à vis du CO lorsqu'il présente des sites FeII. Le solide préalablement reduit devrait ainsi présenter de meilleurs performances dans les procédés de purification de l'hydrogène.

### Exemple 28: influence de la teneur en FeII sur l'affinité du MIL-100(Fe) avec le propyne (C3H4).

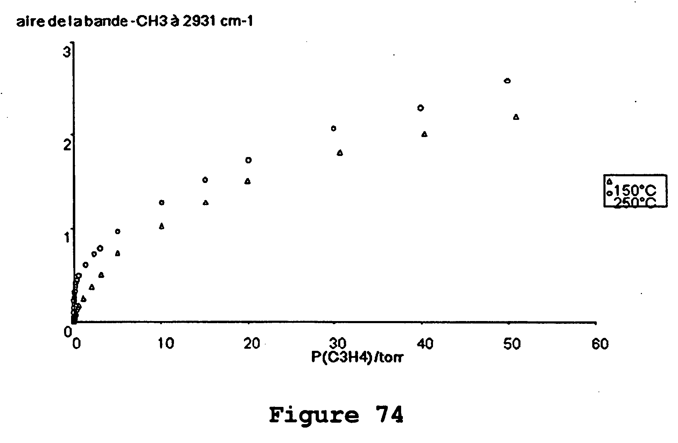

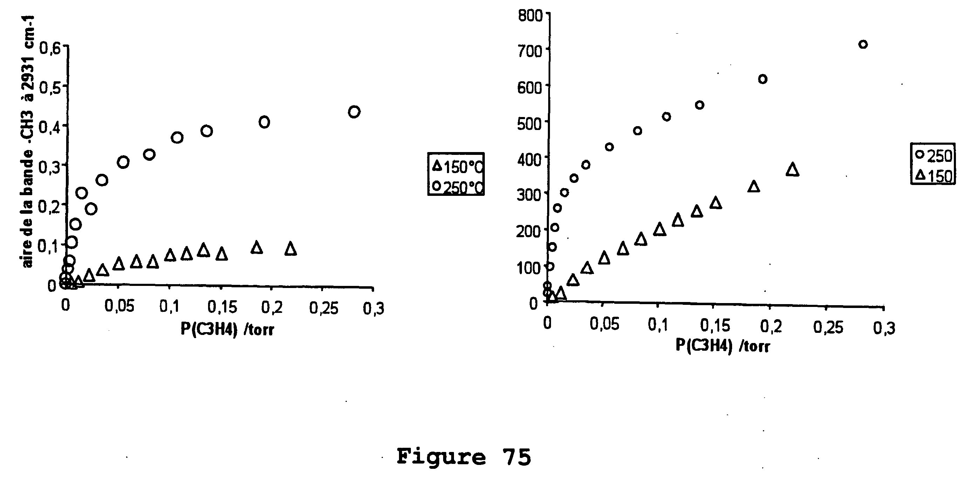

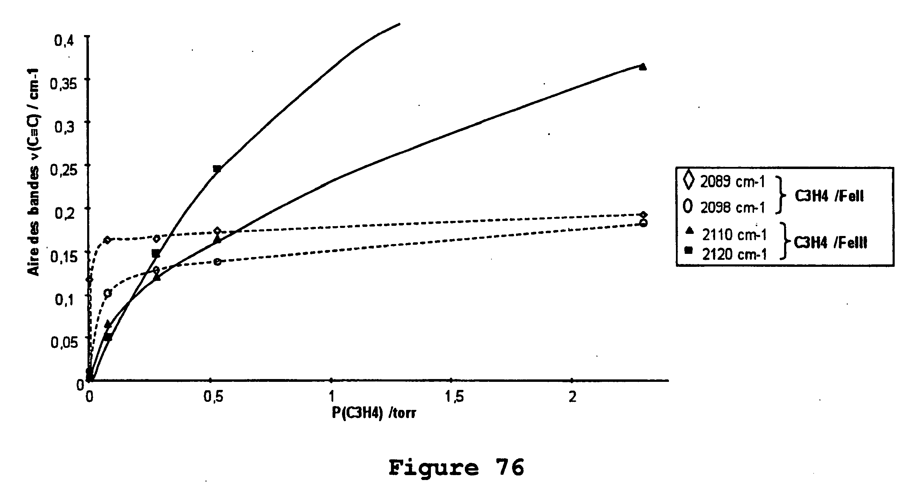

Le solide MIL-100(Fe) prétraité avec NH₄F a été placé dans une cellule infrarouge en quartz puis activé sous vide secondaire pendant 3 heures à 150°C ou 12 heures à 250°C. Le solide a été mis en contact avec des pressions croissantes en propyne à 30°C. La quantité de propyne adsorbée a été estimée par spectroscopie infrarouge et simultanément par méthode volumétrique (Figure 74). Les figures 74 et 75 montrent que le solide réduit à 250°C adsorbe plus de propyne à très basse pression (0-1 torr). La figure 76 montre que le propyne est adsorbé préferentiellement sur les sites fer II à basse pression.

### Exemple 29: influence de la teneur en FeII sur l'affinité du MIL-100(Fe) avec le propene (C3H6)

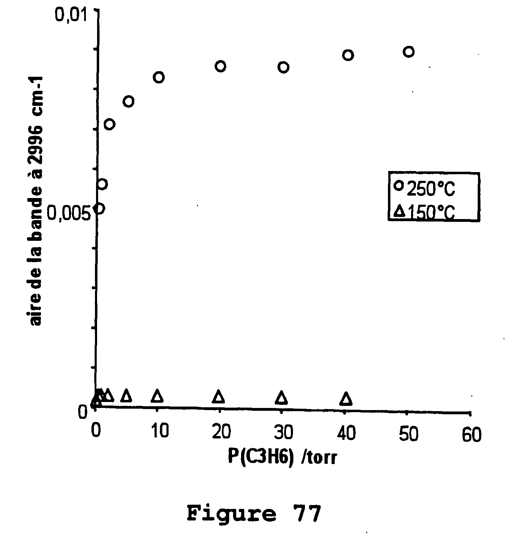

Le solide MIL-100(Fe) fluoré (X=F) a été placé dans une cellule infrarouge en quartz puis activé sous vide secondaire pendant 12 heures à 150°C ou 12 heures à 250°C. Le solide a été mis en contact avec des pressions croissantes en propyne à 30°C. La quantité de propène adsorbée sur FerII en fonction de la pression en phase gaz a qualitativement été estimée par spectroscopie infrarouge (figure 77): la quantité adsorbée sur les sites Fe(II) est nettement plus importante sur le solide activé à 250°C (gamme 0-20 torrs).

### Exemple 30 : Réduction d'un MOF à base de Co

La synthèse de la metalloporphyrine de cobalt (III) poreuse [CoT(*p*-CO2)PPCol.5], nommé PIZA-1, a été rapportée dans « A functional zeolite analogue assembled from metalloporphyrins », M.E. Kosal, J-H Chou, S. R. Wilson, K. S. Suslick, Nature, 2002, 1, 118-121 [ref 52].

La réduction du cobalt (III) à cobalt (II) peut être réalisée par exemple par l'une des deux méthodes suivantes:
- en présence d'un gaz réducteur (He, H₂). 200mg du solide sont placés dans une colonne par laquelle nous faisons passer un flux de gaz (He, H₂) à un débit variable (de 0.01 à 10 mL/min).
- 200 mg du solide sont placés dans une rampe slink sous vide primaire (10⁻² bar) et chauffés à différentes températures (du 50 à 250°C) pendant 24 heures.

Le solide ainsi réduit peut être utilisé pour la séparation de gaz.

### Exemple 31 : Réduction d'un métal du MOF avec compensation de charge par oxydation du spaceur (ligand redox) : MIL-88B 2OH

La réduction du solide poreux MOF peut être réalisée par la compensation de charge par la perte du contre-ion du métal, mais aussi par l'oxydation du ligand organique, en utilisant un ligand redox. Ainsi, la présence de ligands redox dans le MOF peut être utile dans la réduction de MOF pour la séparation de gaz.

Par exemple dans le carboxylate de fer MIL-88B-2OH(Fe), Fe₃O(C₆H₂ (OH)₂-(CO₂)₂]₃.X.nH₂O (X=F, Cl, OH), le ligand organique est l'acide 2,5-dihydroxotéréphthalique. Ce ligand peut se présenter dans sa forme réduite, donc hydroquinone, ou dans sa forme oxydée, quinone (voir schéma ci-dessous).

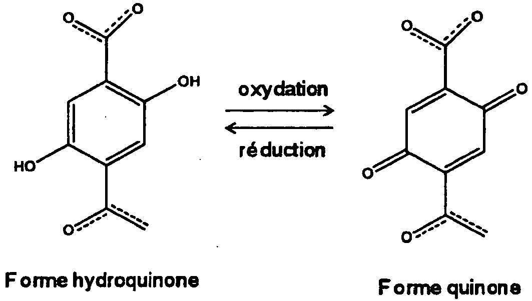

### Synthèse du solide MIL-88B-20H(Fe) voir Exemple 1, section j)

### Réduction du MIL-88H-20H(Fe) :

La réduction du fer (III) à fer (II) peut être réalisée par l'oxydation du ligand redox, l'acide 2,5-dihydroxoterephthalique, par exemple par l'une des deux méthodes suivantes:
- 100 mg du MIL-88B_20H(Fe) sont placés dans une colonne par laquelle on fait passer un flux de gaz réducteur (He, H2) à un débit variable (de 0.01 à 10 mL/min) pendant 2-48 heures.
- 200 mg du solide sont placés dans une rampe slink sous vide primaire (10⁻² bar) et chauffés à différentes températures (du 50 à 250°C) pendant 24 heures.

### Liste des Références

[1] Demande de brevet US 2003-0078311
[2] Brevet US 6,929,679
[3] Brevet US 6,930,193
[4] (a) C. Staudt-Bickel, J.W. Koros, J. Membr. Sci., 1993, 82, 117; (b) J.S. Yang G.H. Hsuie, J. Membr. Sci., 1998, 138, 203; (c) A. Sungpet, J.D. Way, C.A. Koval, M. E. Eberhart, J. Membr. Sci., 2001, 189, 271; (d) O.H. Leblanc, W. J. Ward, S. L. Matson, S. G. Kimura, J. Membr. Sci., 1980, 6, 339 ; (e) I. Pinnau, L.G. Toyl, J. Membr. Sci., 2001, 184, 39 ; (f) C.M. Shue, S. Kulvaranon, M.E. Findlay, A. I. Liapis, Sep. Technol., 1990, 1, 18
[5] P. Glanz, B. Körner, G. H. Findenegg, Adsorption Sci. Technol., 1984, 1, 41
[6] H. Järvelin, J.R. Fair, Ind. Eng. Chem. Res.1993, 32, 2201
[7] M. Kargol, J. Zajac, D. J. Jones, Th. Steriotis, J. Rozeire, P. Vitse, Chem. Mater., 2004, 16, 3911
[8] E.R. Gilliland, H.L. Bliss, C.E. Kip, J. Am. Chem. Soc., 1941, 63, 2088
[9] (a) Dewar, M. Bull. Soc. Chim. Fr. 1951, 1 8 , C79; (b) J. Chatt and L. A. Duncanson, J. Chem. Soc., 1953, 2939 doi:10.1039/JR9530002939 "Olefin co-ordination compounds. Part III. Infra-red spectra and structure: attempted preparation of acetylene complexes*"; (c)* J. Chatt, L. A. Duncanson, L. M. Venanzi, J. Chem. Soc., 1955, 4456-4460 doi:10.1039/JR9550004456 Directing effects in inorganic substitution reactions. Part I. A hypothesis to explain the trans-effect*; (d)* Miessler, Gary L.; Donald A. Tarr (2004). Inorganic Chemistry. Upper Saddle River, New Jersey: Pearson Education, Inc. Pearson Prentice Hall. ISBN 0-13-035471-6.; (e) Herrmann/Brauer: Synthetic Methods of Organometallic and Inorganic Chemistry Georg Thieme, Stuttgart, 1996
[10] (a) D.L. Peterson, F. Helfferich, R. K. Griep, in : Molecular Sieves p. 217-229 Proc. 1st Int. Conf. On Molecular Sieves, London 1967 published by Soc. for Chem. Ind. in 1968**.** (b) C. M. Shu, K. Kulvaranon, M. E. Findley, A.T. Liapis, Sep. Sci. Technol. 1990, 1, 18
[11] (a) Y. Boucheffa, C. Thomazeau, P. Cartraud, P. Magnoux, M. Guisnet, S. Jullian, Ind. Eng. Chem. Res. 1997, 36, 3198 ; (b) H. Järvelin, J.R. Fair, Ind. Eng. Chem. Res.1993, 32, 2201 ; (c) J. Kärger, D.M. Ruthven, Diffusion in zeolites, John Wiley & Sons: New York, 1992, p 104
[12] D. M. Ruthven, S. C. Reyes, Microp. Mesop. Mater., 2007, 104, 59
[13] D. H. Olson, X. Yang, M. A. Camblor, J. Phys. Chem. B, 2004, 108, 11044
[14] B. Xiao, P. S. Wheatley, X. Zhao, A. J. Fletcher, S. Fox, A. G. Rossi, S. Bordiga, L. Regli, I. L. Megson, K. M. Thomas and R. E. Morris, J. Am. Chem. Soc. 2007, 129, 1203
[15] A. Wagener, M. Schindler, F. Rudolphi, S. Ernst," Metallorganische Koordinationspolymere zur adsorptiven Trennung von Propan/Propen-Gemischen », Chem. Ing. Tech., 2007, Volume 79, Issue 6, Pages 851 - 855
[16] 6,491,740
[17] Serre et al., « A new route to the synthesis of trivalent transition metals porous carboxylates with trimeric SBU", Angew. Chem. Int. Ed. 2004, 43, 6286
[18] K. Byrapsa, M. Yoshimura, « Handbook of hydrothermal technology », Noyés Publications, Parkridge, New Jersey USA, William Andrew Publishing, LLC, Norwich NY USA, 2001
[19] G. Tompsett, W. C. Conner, K. S. Yngvesson, ChemPhysChem. 2006, 7, 296
[20] S.-E. Park, J.-S. Chang, Y. K. Hwang, D. S. Kim, S. H. Jhung, J.-S. Hwang, Catal. Survey Asia 2004, 8, 91
[21] C. S. Cundy, Collect. Czech. Chem. Commum. 1998, 63, 1699
[22] S. H. Jhung, J.-H. Lee, J.-S. Chang, Bull. Kor. Chem. Soc. 2005, 26, 880
[23] A. Pichon, Cryst. Eng. Comm. 8, 2006, 211-214
[24] D. Braga, Angew. Chem. Int. Ed. 45, 2006, 142-246
[25] D. Braga, Dalton Trans., 2006, 1249-1263.
[26] Rostrup-Nielsen, Catal. Today, 2005, 106:293-296
[27] J. Kärger, D.M. Ruthven, Diffusion in zeolites, John Wiley & Sons: New York, 1992, p 104
[28] Patricia Horcajada, Suzy Surblé, Christian Serre, Do-Young Hong, You-Kyong Seo, Jong-San Chang, Jean-Marc Grenèche, Irene Margiolaki and Gérard Férey, Chem Comm, 2007, 2820 : Synthesis and catalytic properties of MIL-100(Fe), an iron(III) carboxylate with large pore
[29] Dziobkowski et al., Inorg. Chem. 1982, 20, 671
[30] Anzalone et al., J. Org. Chem. 1985, 50, 2128
[31] Ameerunisha et al., J. Chem. Soc. Perkin Trans.2 1995, 1679
[32] Shiotani Akinori, Z. Naturforsch. 1994, 49, 12, 1731-1736
[33] Serre et al., « Role of solvent-host interactions that lead to very large swelling of hybrid frameworks », Science, 2007, 315, 1828-1831
[34] Serre et al. J. Am. Chem. Soc., 2005, 127, 16273-16278
[35] (a) A. Vimont, J.-M. Goupil, J.-C. Lavalley, and M. Daturi, S. Surblé, C. Serre, F. Millange, G. Férey, N. Audebrand, J. Am. Chem. Soc. , 2006, 128, 3218-3227*:* First characterization of acid sites in a new chromium(III) dicarboxylate with giant pores; (b) A. Vimont, H. Leclerc, F. Mauge, M. Daturi, J. C. Lavalley, S. Surblé, C. Serre et G. Férey, , Journal of Physical Chemistry C, 111 (2007), 383-388: Creation of Controlled Brønsted Acidity on a Zeotypic Mesoporous Chromium(III) Carboxylate by Grafting Water and Alcohol Molecules
[36] (a) R.B. Eldridge, Ind. Eng. Chem. Res., 1993, 32, 2208; (b) H. Jarvelin, J.R. Fair, Ind. Eng. Chem. Res., 1993, 32, 2201
[37] Suzy Surblé, Christian Serre, Caroline Mellot-Draznieks, Franck Millange, and Gérard Férey: Chem. Comm. 2006 284-286 : A new isoreticular class of Metal-Organic-Frameworks with the MIL-88 topology
[38] De Weireld, G.; Frere, M.; Jadot R. Meas.Sci. Technol. 10, 117, 1999.
[39] Dreisbach, F.; Seif, R.; Losch, H.W. Fundamental of Adsorption 7, 255, 2002.
[40] Sorensen, O.; Rouquerol, J. Sample Controlled Thermal Analysis, Kluwer Academic Publishers, 2003.
[41] Battiston A.A. et al., "Reactivity of Fe-binuclear complexes in over-exchanged Fe/ZSM5 studied by in situ XAFS spectroscopy. Part 1: Heat treatment in He and O2". Journal of Catalysis, 215, 279-293, 2003.
[42] Magnacca G. et al., "Structural and surface characterization of pure and sulfated iron oxides", Chem. Mater., 15, 675-687, 2003.
[43] (a) Denayer, J. F. M.; De Meyer, K. ; Martens, J. A.; Baron, G. V. Angew. Chem., Int. Ed. 2003, 42, 2774-2777. (b) Denayer, J. F. M.; Ocakoglu, R. A.; Arik, I. C.; Kirschhock, C. E. A.; Martens, J. A.; Baron, G. V. Angew. Chem., Int. Ed. 2005, 44, 400-403.
[44] Brevet US 6,024,781
[45] Publication Internationale WO 98/006684
[46] Newalkar, B. L.; Choudary, N. V.; Turaga, U. T.; Vijayalakshimi,R. P.; Kumar, P.; Komarneni, S.; Bhat, S. G. T. Chem. Mater.2003, 15, 1474
[47] Surblé et al., « A new isoreticular class of metal-organic frameworks with the MIL-88 topology », Chem. Comm., 2006, 284-286
[48] Mellot-Draznieks et al., « Very large swelling in hybrid frameworks : a combined computational and power diffraction study », J. Am. Chem. Soc., 2005, Vol. 127, 16273-16278
[49] Férey et al., « A chromium terephthalate-based solid with unusally large pore volumes and surface area », Science, 2005, Vol. 309, 2040-2042
[50] S. Surblé, F. Millange, C. Serre, T. Düren, M. Latroche, S. Bourrelly, P.L. Llewellyn and G. Férey « MIL-102: A Chromium Carboxylate Métal Organic Framework with Gas Sorption Analysis » J. Am. Chem. Soc. 128 (2006), 46, 14890
[51] Y. Liu et al, Angew. Chem. Int. Ed. 2007, 46, 3278-3283
[52] Kosal et al., « A functional zeolite analogue assembled from metalloporphyrins », Nature, 2002, 1, 118-121
[53] Dietzel et al., « Hydrogen adsorption in a nickel based coordination polymer with open metal sites in the cylindrical cavities of the desolvated framework », Chem. Commun., 2006, 959-961
[54] P.D.C. Dietzel, R.E. Johnsen, R. Blom, H. Fjellvag, P.Chem. Eur. J., 2008, 14, 2389-2397
[55] P. L. Llewellyn & G. Maurin, C.R.Chimie, 8, 283-302 (2005).
[56] Adriano Zecchina, Mickaël Rivallan, Gloria Berlier, Carlo Lamberti, Gabriele Ricchiardi, Phys. Chem. Chem. Phys., 2007, (27),3483-3499
[57] Philip L. Llewellyn,Sandrine Bourrelly, Christian Serre, Alexandre Vimont, Marco Daturi, Lomig Hamon,Guy De Weireld, Jong-San Chang, Do-Young Hong, Young Kyu Hwang, Sung Hwa Jhung, Gérard Férey "High Uptakes of CO2 and CH4 in Mesoporous Metal Organic Frameworks MIL-100 and MIL-101", Langmuir, 2008, 24, 7245-7250
[58] A.A. Davidov, Infrared Spectroscopy of Adsorbed Species on the Surface of Transition Metal Oxides, Wiley Interscience, 1990 p123-130 chapter 2

## Revendications

1. Utilisation d'un solide MOF cristallin poreux comprenant des sites métalliques réduits pour séparer un mélange de molécules ayant des degrés et/ou un nombre d'insaturations différents ledit solide comprenant une structure tridimensionnelle de motifs répondant à la formule (I) suivante :
MₘOₖXₗLₚ (I)
dans laquelle :
● chaque occurrence de M représente indépendamment un ion d'un métal de transition M^{z+} ou sa forme réduite M^{(z-1)+} choisi dans le groupe comprenant Fe, Mn, Co, Ni et V, et dans lequel z est 3 ou 4; étant entendu que le rapport y=M^{(z-1)+}/M^{z+} est compris entre 0<y≤x; où x est la fraction d'ions M^{z+} accessibles du solide MOF;
● m est 1 à 12 ;
● k est 0 à 4 ;
● 1 est 0 à 18 ;
● p est 1 à 6 ;
● X est un anion choisi dans le groupe comprenant OH, Cl⁻, F-, I⁻, Br⁻, SO₄2⁻, NO₃⁻, ClO₄⁻, PF₆⁻, BF₄⁻, R-(COO)ₙ⁻ où R est tel que défini ci-dessous, R¹-(COO)ₙ⁻, R¹-(SO₃)ₙ⁻, R¹-(PO₃)ₙ⁻, où R¹ est un hydrogène, un alkyle en C₁ à C₁₂, linéaire ou ramifié, éventuellement substitué, un aryle en C₆ à C₁₀ éventuellement substitué; où n représente un entier de 1 à 4 ;
● L est un ligand espaceur comprenant un radical R comportant q groupements carboxylates 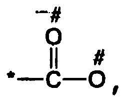 où
- q est 1, 2, 3, 4, 5 ou 6 ;
- * désigne le point d'attachement du carboxylate avec le radical R ;
- # désigne les points d'attachement possibles du carboxylate à l'ion métallique ;
- R représente :
(i) un radical C₁₋₁₂alkyle, C₂₋₁₂alcène ou C₂₋₁₂alcyne ;
(ii) un radical aryle mono- ou poly-cyclique, fusionné ou non, comprenant 6 à 50 atomes de carbone ;
(iii) un hétéroaryle mono- ou poly-cyclique, fusionné ou non, comprenant 1 à 50 atomes de carbone ;
(iv) un radical organique comprenant un élément métallique choisi dans le groupe comprenant le ferrocène, la porphyrine, la phtalocyanine;
le radical R étant éventuellement substitué par un ou plusieurs groupes R², indépendamment choisis dans le groupe comprenant C₁₋₁₀alkyle; C₂₋₁₀alcène; C₂₋₁₀alcyne; C₃₋₁₀cycloalkyle; C₁₋₁₀hétéroalkyle; C₁₋₁₀haloalkyle; C₆₋₁₀aryle; C₃₋₂₀hétérocyclique; C₁₋₁₀alkylC₆₋₁₀aryle ; C₁₋₁₀alkylC₃₋₁hétéroaryle ; F ; Cl ; Br; I; -NO₂ ; -CN ; -CF₃ ; -CH₂CF₃ ; -OH ; -CH₂OH ; - CH₂CH₂OH ; -NH₂ ; -CH₂NH₂ ; -NHCHO ; -COOH ; - CONH₂ ; -SO₃H ; -CH₂SO₂CH₃ ; -PO₃H₂ ; ou une fonction ―GR^{G1} dans laquelle G est ―O-, -S-, -NR^{G2}-, -C(=O)-, -S(=O)-, -SO₂-, -C(=O)O-, -C(=O)NR^{G2}-, -OC(=O)-, -NR^{G2}C(=O)-, -OC(=O)O-, - OC(=O)NR^{G2}-, -NR^{G2}C(=O)O-, -NR^{G2}C(=O)NR^{G2}-, - C(=S)-, où chaque occurrence de R^{G2} est indépendamment des autres occurrences de R^{G2} un atome d'hydrogène ; ou une fonction C₁₋₁₂alkyle, C₁₋₁₂hétéroalkyle, C₂₋₁₀alcène ou C₂₋₁₀alcyne, linéaire, ramifiée ou cyclique, éventuellement substituée ; ou un groupe C₆₋₁₀aryle, C₃₋₁₀hétéroaryle, C₅₋₁₀hétérocyclique, C₁₋₁₀alkyleC₆₋₁₀aryle ou C₁₋₁₀alkyleC₃₋₁₀hétéroaryle dans lequel le radical aryle, hétéroaryle ou hétérocyclique est éventuellement substitué ; ou bien, lorsque G représente -NR^{G2}-, R^{G1} et R^{G2} conjointement avec l'atome d'azote auquel ils sont liés forment un hétérocycle ou un hétéroaryle éventuellement substitué.

2. Utilisation selon la revendication 1, dans laquelle le ligand L est un ligand di-, tri-, tétra- ou hexa-carboxylate choisi dans le groupe comprenant : C₂H₂(CO₂⁻)₂ (fumarate), C₂H₄(CO₂⁻)₂ (succinate), C₃H₆(CO₂⁻)₂ (glutarate), C₄H₄(CO₂⁻)₂ (muconate), C₄H₈(CO₂⁻)₂ (adipate), C₅H₃S(CO₂⁻)₂ (2,5-thiophènedicarboxylate), C₆H₄(CO₂⁻)₂ (téréphtalate), C₆H₂N₂(CO₂⁻)₂ (2,5-pyrazine dicarboxylate), C₇H₁₆(CO₂⁻)₂ (azélate), C₁₀H₆(CO₂⁻)₂ (naphtalène-2,6-dicarboxylate), C₁₂H₈(CO₂⁻)₂ (biphényle-4,4'-dicarboxylate), C₁₂H₈N₂(CO₂⁻)₂ (azobenzènedicarboxylate), C₁₂H₆Cl₂N₂(CO₂⁻)₂ (dichloroazobenzènedicarboxylate), C₁₂H₆N₂(CO₂⁻)₄ (azobenzènetetracarboxylate), C₁₂H₆N₂(OH)₂(CO₂⁻)₂ (dihydroxoazobenzènedicarboxylate), C₆H₃(CO₂⁻)₃ (benzène-1, 2, 4-tricarboxylate), C₆H₃(CO₂⁻)₃ (benzène-1,3,5-tricarboxylate), C₂₄H₁₅(CO₂⁻)₃ (benzène-1, 3, 5-tribenzoate), C₄₂H₂₇(CO₂⁻)₃ (1,3,5-tris[4'-carboxy(1,1'-biphenyl-4-yl)benzene), C₆H₂(CO₂⁻)₄ (benzène-1,2,4,5-tétracarboxylate, C₁₀H₄(CO₂⁻)₄ (naphtalène-2,3,6,7-tétracarboxylate), C₁₀H₄(CO₂⁻)₄ (naphtalène=1,4,5,8-tétracarboxylate), C₁₂H₆(CO₂⁻)₄ (biphényl-3,5,3',5'-tétracarboxylate, et les analogues modifiés choisis dans le groupe comprenant le 2-aminotéréphtalate, le 2-nitrotéréphtalate, le 2-méthyltéréphtalate, le 2-chlorotéréphtalate, le 2-bromotéréphtalate, le 2,5-dihydroxotéréphtalate, le 2,5-diperfluorotéréphtalate, le tétrafluorotéréphtalate, le 2,5-dicarboxytéréphtalate, le diméthyl-4,4'-biphénydicarboxylate, le tétraméthyl-4,4'-biphénydicarboxylate, le dicarboxy-4,4'-biphénydicarboxylate.

3. Utilisation selon l'une des revendications 1 ou 2, dans laquelle l'anion X est choisi dans le groupe comprenant OH⁻, Cl⁻, Br⁻, F⁻, R-(COO)ₙ⁻, PF₆⁻, NO₃⁻, SO₄²⁻, ClO₄⁻, avec R et n tel que défini dans la revendication 1.

4. Utilisation selon l'une quelconque des revendications 1 à 3, dans laquelle ledit mélange de molécules est choisi parmi :
- un mélange d'au moins un hydrocarbure saturé et au moins un hydrocarbure insaturé,
- un mélange d'au moins un composé comprenant un ou plusieurs doublet(s) libre(s) et d'au moins un composé ne comprenant pas de doublet libre, et
- un mélange d'hydrogène et de monoxyde et/ou dioxyde de carbone.

5. Utilisation selon la revendication 4, dans laquelle le mélange d'hydrocarbures est un mélange de propane et de propène, un mélange de n-butane et d'isobutène, un mélange de n-butane et de butènes, un mélange d'acétylène et d'éthylène, un mélange d'acétylène et de dioxyde de carbone.

6. Utilisation selon l'une quelconque des revendications 1 à 5, dans laquelle M est Fe, z est 3 et le rapport y=M^{(z-1)+}/M^{z+} est compris entre 0<y≤1/3.

7. Utilisation selon l'une quelconque des revendications 1 à 6, dans laquelle le solide MOF comprend une succession tridimensionnelle de motifs répondant à la formule (I) choisis dans le groupe comprenant :
- M₃OX[C₆H₃(CO₂)₃]₂ (structure de type MIL-100)
- M₃OX[C₆H₄(CO₂)₂]₃ (structure de type MIL-101)
- M₃OX[C₆(CH₃)₄(CO₂)₂]₃ (structure de type MIL-88B_4CH₃)
- M₆O₂X₂[C₁₀H₂(CO₂)₄]₃ (structure de type MIL-102)
- Fe₆O₂[C₁₂H₆N₂-(CO₂)₄]₃.X₂.nH₂O (structure de type MIL-127) dans laquelle, M est choisi parmi Fe, Mn, Co; et X est tel que défini dans les revendications 1 ou 2.

8. Utilisation selon la revendication 7, dans laquelle M est Fe.

9. Procédé pour séparer un mélange de molécules ayant des degrés et/ou un nombre d'insaturations différents par adsorption préférentielle des molécules à degré et/ou nombre d'insaturations supérieur sur un solide MOF cristallin poreux tel que défini dans l'une quelconque des revendications 1 à 8, ledit procédé comprenant au moins une étape réactionnelle consistant
(i) à mélanger dans un solvant polaire :
- au moins une solution comprenant au moins un précurseur inorganique métallique se présentant sous la forme de métal M, d'un sel métallique de M ou d'un complexe de coordination comprenant un ion métallique de M ;
- au moins un ligand L' comprenant un radical R comportant q groupements *-C(=O)-R³, où
• M, q et R sont tels que définis dans la revendication 1,
• * désigne le point d'attachement du groupement avec le radical R,
• R³ est choisi dans le groupe comprenant un OH, un OY, avec Y est un cation alcalin, un halogène, un radical ―OR⁴, -O-C(=O)R⁴, -NR⁴R^{4'}, où R⁴ et R^{4'} sont des radicaux alkyles en C₁₋₁₂,
pour obtenir un matériau MOF ;
(ii) à activer le matériau MOF obtenu en (i) ; et
(iii) à mettre en contact le matériau MOF obtenu à l'étape (ii) avec un mélange de molécules ayant des degrés et/ou un nombre d'insaturations différents.

10. Procédé selon la revendication 9, dans lequel l'étape (ii) est une étape de réduction d'au moins une partie des centres métalliques M^{z+} dudit matériau MOF en ions M^{(z-1)+} dans lequel z est 3.

11. Procédé selon l'une des revendications 9 ou 10, dans lequel l'étape (ii) d'activation est réalisée à une température de 25 à 300°C.

12. Procédé selon l'une des revendications 9 ou 10, comprenant en outre une étape (i') de traitement du matériau MOF obtenu à l'étape (i) avec une source d'ions F⁻.

13. Procédé selon la revendication 12, dans lequel l'étape (ii) d'activation est réalisée à une température de 50 à 250°C.

14. Procédé selon l'une quelconque des revendications 9 à 13, dans lequel le mélange de molécules est choisi parmi :
- un mélange d'au moins une paraffine et au moins une oléfine.
- un mélange d'au mois une oléfine et un alcyne.
- un mélange d'acétylène et de dioxyde de carbone.
- un mélange d'hydrogène et de monoxyde et/ou dioxyde de carbone.

15. Procédé selon la revendication 14, dans lequel le mélange de molécules est un mélange de propane et de propène, un mélange de n-butane et d'isobutène, un mélange de n-butane et de butènes, un mélange d'acétylène et d'éthylène, un mélange d'acétylène et de dioxyde de carbone.

16. Procédé selon l'une quelconque des revendications 9 à 15, dans lequel le mélange de molécules est un mélange d'au moins un hydrocarbure saturé et au moins un hydrocarbure insaturé ou de dioxyde de carbone, et l'acidité de Brönsted du matériau MOF obtenu à l'étape (ii) ne déclenche pas la polymérisation de l'hydrocarbure insaturé.

17. Procédé selon l'une quelconque des revendications 9 à 16, dans lequel le mélange de molécules est en phase gazeuse.

18. Procédé selon l'une quelconque des revendications 9 à 17 comprenant en outre une étape (iv) de désorption de la molécule préférentiellement adsorbée.

19. Procédé selon la revendication 18, dans lequel la désorption est effectuée par une technique de désorption choisie parmi le déplacement avec un gaz au moins 98% pur à basse pression, un changement de pression, un changement de température, ou une combinaison de celles-ci.

20. Utilisation selon l'une quelconque des revendications 1 à 8, pour la purification de l'hydrogène.

## Claims

1. Use of a porous crystalline MOF solid containing reduced metal sites to separate a mixture of molecules having different unsaturation degrees and/or a different number of unsaturations said solid having a three-dimensional succession of units having the following formula (I):
MₘOₖXₗLₚ (I)
in which:
● each occurrence of M independently represents an ion of a transition metal M^{z+} or the reduced form thereof M^{(z-1)+} selected from the group comprising Fe, Mn, Co, Ni and V, and in which z is 3 or 4, provided that the ratio y=M^{(z-1)+}/M^{z+} lies between 0<y≤x, where x is the fraction of accessible ions M^{z+} of the MOF solid,
● m is 1 to 12,
● k is 0 to 4,
● l is 0 to 18,
● p is 1 to 6,
● X is an anion selected from the group comprising OH, Cl⁻, F⁻, I⁻, Br⁻, SO₄²⁻, NO₃⁻, ClO₄⁻, PF₆⁻, BF₄⁻, R-(COO)ₙ⁻ where R is as defined below, R¹-(COO)ₙ⁻, R¹-(SO₃)ₙ⁻ and R¹-(PO₃)ₙ⁻, where R¹ is a hydrogen atom, a linear or branched, optionally substituted C₁ to C₁₂ alkyl or an optionally substituted C₆ to C₁₀ aryl, where n represents an integer from 1 to 4,
● L is a spacer ligand comprising a radical R containing q carboxylate groups 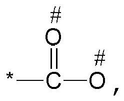 where
- q is 1, 2, 3, 4, 5 or 6,
- * designates the point of attachment of the carboxylate to the R radical,
- # designates the possible points of attachment of the carboxylate to the metal ion,
- R represents:
(i) a C₁₋₁₂ alkyl, C₂₋₁₂ alkene or C₂₋₁₂ alkyne radical,
(ii) a mono- or polycyclic, fused or non-fused aryl radical, comprising 6 to 50 carbon atoms,
(iii) a mono- or polycyclic, fused or non-fused heteroaryl, comprising 1 to 50 carbon atoms,
(iv) an organic radical containing a metallic element selected from the group comprising ferrocene, porphyrin and phthalocyanine,
the radical R optionally being substituted by one or more groups R², independently selected from the group comprising C₁₋₁₀ alkyl, C₂₋₁₀ alkene, C₂₋₁₀ alkyne, C₃₋₁₀ cycloalkyl, C₁₋₁₀ heteroalkyl, C₁₋₁₀ haloalkyl, C₆₋₁₀ aryl, C₃₋₂₀ heterocycle, C₁₋₁₀alkylC₆₋₁₀aryl, C₁₋₁₀alkylC₃₋₁₀heteroaryl, F, Cl, Br, I, -NO₂, -CN, -CF₃, -CH₂CF₃, -OH, -CH₂OH, -CH₂CH₂OH, -NH₂, - CH₂NH₂, -NHCHO, -COOH, -CONH₂, -SO₃H, -CH₂SO₂CH₃, -PO₃H₂, or a -GR^{G1} group in which G is -O-, -S-, -NR^{G2}-, -C(=O)-, -S(=O)-, -SO₂-, -C(=O)O-, -C(=O)NR^{G2}-, -OC(=O)-, -NR^{G2}C(=O)-, -OC(=O)O-, -OC(=O)NR^{G2}-, -NR^{G2}C (=O)O-, -NR^{G2}C(=O)NR^{G2}-, -C(=S)-, where each occurrence of R^{G2} is independently from the other occurrences of R^{G2} a hydrogen atom; or a linear, branched or cyclic, optionally substituted C₁₋₁₂alkyl, C₁₋₁₂heteroalkyl, C₂₋₁₀alkene or C₂₋₁₀alkyne group; or a C₆₋₁₀aryl, C₃₋₁₀heteroaryl, C₅₋₁₀heterocycle, C₁₋₁₀alkylC₆₋₁₀aryl or C₁₋₁₀alkylC₃₋₁₀heteroaryl group in which the aryl, heteroaryl or heterocyclic radical is optionally substituted, or else, when G represents -NR^{G2}-, R^{G1} and R^{G2} together with the nitrogen atom to which they are attached form an optionally substituted heterocycle or heteroaryl.

2. Use as claimed in claim 1, wherein the ligand L is a di-, tri-, tetra- or hexacarboxylate ligand selected from the group comprising: C₂H₂(CO₂⁻)₂ (fumarate), C₂H₄(CO₂⁻)₂ (succinate), C₃H₆(CO₂⁻)₂ (glutarate), C₄H₄(CO₂⁻)₂ (muconate), C₄H₈(CO₂⁻)₂ (adipate), C₅H₃S (CO₂⁻) (2,5-thiophenedicarboxylate), C₆H₄(CO₂⁻)₂ (terephthalate), C₆H₂N₂(CO₂⁻)₂ (2,5-pyrazine dicarboxylate), C₇H₁₆(CO₂⁻)₂ (azelate), C₁₀H₆(CO₂⁻)₂ (naphthalene-2,6-dicarboxylate), C₁₂H₈(CO₂⁻)₂ (biphenyl-4,4'-dicarboxylate), C₁₂H₈N₂(CO₂⁻)₂ (azobenzenedicarboxylate), C₁₂H₆Cl₂N₂(CO₂⁻)₂ (dichloroazobenzenedicarboxylate), C₁₂H₆N₂(CO₂⁻)₄ (azobenzenetetracarboxylate), C₁₂H₆N₂(OH)₂(CO₂⁻)₂ (dihydroxo-azobenzenedicarboxylate), C₆H₃(CO₂⁻)₃ (benzene-1,2,4-tricarboxylate), C₆H₃(CO₂⁻)₃ (benzene-1,3,5-tricarboxylate), C₂₄H₁₅(CO₂⁻)₃ (benzene-1,3,5-tribenzoate), C₄₂H₂₇(CO₂⁻)₃ (1,3,5-tris[4'-carboxy(1,1'-biphenyl-4-yl)benzene), C₆H₂(CO₂⁻)₄ (benzene-1,2,4,5-tetracarboxylate, C₁₀H₄(CO₂⁻)₄ (naphthalene-2,3,6,7-tetracarboxylate), C₁₀H₄(CO₂⁻)₄ (naphthalene-1,4,5,8-tetracarboxylate), C₁₂H₆(CO₂⁻)₄ (biphenyl-3,5,3',5'-tetracarboxylate, and the modified analogs selected from the group comprising 2-amino-terephthalate, 2-nitroterephthalate, 2-methyltere-phthalate, 2-chloroterephthalate, 2-bromoterephthalate, 2,5-dihydroxoterephthalate, 2,5-diperfluoroterephthalate, tetrafluoroterephthalate, 2,5-dicarboxyterephthalate, dimethyl-4,4'-biphenyldicarboxylate, tetramethyl-4,4'-biphenyldicarboxylate and dicarboxy-4,4'-biphenyldicarboxylate.

3. Use as claimed in one of claims 1 or 2, wherein the anion X is selected from the group comprising OH⁻, Cl⁻, Br⁻, F⁻, R-(COO)ₙ⁻, PF₆⁻, NO₃⁻, SO₄²⁻ and ClO₄⁻, with R and n as defined in claim 1.

4. Use as claimed in any one of claims 1 to 3, wherein said mixture of molecules is selected from:
a mixture of at least one saturated hydrocarbon and at least one unsaturated hydrocarbon,
a mixture of at least one compound containing one or more free electron pair(s) and at least one compound not containing a free electron pair, and
a mixture of hydrogen (H₂) and carbon monoxide and/or dioxide.

5. Use as claimed in claim 4, wherein the mixture of hydrocarbons is a mixture of propane and propene, a mixture of n-butane and isobutene, a mixture of n-butane and butenes, a mixture of acetylene and ethylene, a mixture of acetylene and carbon dioxide.

6. Use as claimed in any one of claims 1 to 5, wherein M is Fe, z is 3 and the ratio y=M^{(z-1)+}/M^{z+} lies between 0<y≤1/3.

7. Use as claimed in any one of claims 1 to 6, wherein the MOF solid has a three-dimensional succession of units having formula (I) selected from the group comprising:
- M₃OX[C₆H₃(CO₂)₃]₂ (structure of MIL-100 type)
- M₃OX[C₆H₄(CO₂)₂]₃ (structure of MIL-101 type)
- M₃OX[C₆(CH₃)₄(CO₂)₂]₃ (structure of MIL-88B_4CH₃ type)
- M₆O₂X₂[C₁₀H₂(CO₂)₄]₃ (structure of MIL-102 type)
- Fe₆O₂[C₁₂H₆N₂-(CO₂)₄]₃.X₂.nH₂O (structure of MIL-127 type) in which M is selected from Fe, Mn, Co, and X is as defined in claim 1 or 2.

8. Use as claimed in claim 7, wherein M is Fe.

9. A process for separating a mixture of molecules having different unsaturation degrees and/or a different number of unsaturations by preferential adsorption of the molecules with a greater unsaturation degree and/or number of unsaturations on a porous crystalline MOF solid as defined in any one of claims 1 to 8, wherein said process comprises at least one reaction step consisting (i) in mixing in a polar solvent:
- at least one solution containing at least one inorganic metal precursor present in the form of metal M, a metal salt of M or a coordination complex containing a metal ion of M,
- at least one ligand L' comprising a radical R containing q groups *-C(=O)-R³, where
● M, q and R are as defined in claim 1,
● * designates the point of attachment of the group to the radical R,
● R³ is selected from the group comprising an OH, an OY, wherein Y is an alkaline cation, a halogen, a radical
-OR⁴, -O-C(=O)R⁴ or -NR⁴R^{4'}, where R⁴ and R^{4'} are C₁₋₁₂alkyl radicals,
to obtain an MOF material,
(ii) in activating the MOF material obtained in (i), and
(iii) in placing the MOF material obtained in step (ii) in contact with a mixture of molecules having different unsaturation degrees and/or a different number of unsaturations.

10. process as claimed in claim 9, wherein step (ii) is a step of reduction of at least some of the metal centers M^{z+} of said MOF material to M^{(z-1)+} ions in which z is 3.

11. Process as claimed in one of claims 9 or 10, wherein the activation step (ii) is performed at a temperature of 25 to 300°C.

12. Process as claimed in one of claims 9 or 10, further comprising a step (i') of treatment of the MOF material obtained in step (i) with a source of F⁻ ions.

13. Process as claimed in claim 12, wherein the activation step (ii) is performed at a temperature of 50 to 250°C.

14. Process as claimed in any one of claims 9 to 13, wherein the mixture of molecules is selected from:
- a mixture of at least one paraffin and at least one olefin.
- a mixture of at least one olefin and one alkyne.
- a mixture of acetylene and carbon dioxide.
- a mixture of hydrogen (H₂) and carbon monoxide and/or dioxide.

15. Process as claimed in claim 14, wherein the mixture of molecules is a mixture of propane and propene, a mixture of n-butane and isobutene, a mixture of n-butane and butenes, a mixture of acetylene and ethylene, a mixture of acetylene and carbon dioxide.

16. Process as claimed in any one of claims 9 to 15, wherein the mixture of molecules is a mixture of at least one saturated hydrocarbon and at least one unsaturated hydrocarbon or carbon dioxide, and the Brönsted acidity of the MOF material obtained in step (ii) does not trigger polymerization of the unsaturated hydrocarbon.

17. Process as claimed in any one of claims 9 to 16, wherein the mixture of molecules is in the gaseous phase.

18. Process as claimed in any one of claims 9 to 17, further comprising a step (iv) of desorption of the molecule preferentially adsorbed.

19. Process as claimed in claim 18, wherein the desorption is effected by a desorption technique selected from displacement with a gas, at least 98% pure, at low pressure; a pressure change; a temperature change; or a combination thereof.

20. Use as claimed in any one of claims 1 to 8, for the purification of hydrogen (H₂).

## Patentansprüche

1. Verwendung eines porösen kristallinen MOF-Feststoffs, reduzierte Metall-Sites umfassend zur Trennung eines Molekülgemischs, die unterschiedliche Grade und/oder eine unterschiedliche Anzahl der Ungesättigtheiten haben, wobei besagter Feststoff eine dreidimensionale Struktur von Einheiten der folgenden Formel (I) umfasst:
MₘOₖXₗLₚ (I)
worin:
● Jedes Auftreten von M unabhängig ein Ion eines Übergangsmetalls M^{z+} oder die reduzierte Form hiervon M^{(z-1)+} darstellt, ausgewählt aus der Gruppe umfassend Fe, Mn, Co, Ni und V, und in welcher z gleich 3 oder 4 ist; vorausgesetzt, dass das Verhältnis y=M^{(z-1)+}/M^{z+} zwischen 0<y≤x liegt; wobei x der zugängliche Teil der M^{z+} -Ionen des MOF-Feststoffs ist;
● m für 1 bis 12 steht;
● k für 0 bis 4 steht;
● l für 0 bis 18 steht;
● p für 1 bis 6 steht;
● X für ein Anion steht, ausgewählt aus der Gruppe umfassend OH, Cl⁻, F⁻, I⁻, Br⁻, SO₄²⁻, NO₃⁻, ClO₄⁻, PF₆⁻, BF₄⁻, R-(COO)ₙ⁻ wobei R wie nachstehend definiert ist, R¹-(COO)ₙ⁻, R¹-(SO₃)ₙ⁻ und R¹-(PO₃)ₙ⁻, wobei R¹ für ein Wasserstoffatom, ein lineares oder verzweigtes, gegebenenfalls substituiertes C₁ bis C₁₂-Alkyl oder ein gegebenenfalls substituiertes C₆ bis C₁₀-Aryl steht, wobei n eine ganze Zahl von 1 bis 4 ist;
● L ein Spacer-Ligand ist, umfassend ein Radikal R, das q Carboxylatgruppen 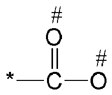 enthält, wobei
- q für 1, 2, 3, 4, 5 oder 6 steht;
- * die möglichen Verknüpfungspunkte des Carboxylats mit dem R-Radikal bezeichnet;
- # die möglichen Verknüpfungspunkte des Carboxylats mit dem Metallion bezeichnet;
- R steht für:
(i) ein C₁₋₁₂-Alkyl-, C₂₋₁₂-Alken- oder C₂₋₁₂-Alkin-Radikal;
(ii) ein mono- oder polyzyklisches, fusioniertes oder nicht fusioniertes Aryl-Radikal, umfassend 6 bis 50 Kohlenstoffatome;
(iii) ein mono- oder polyzyklisches, fusioniertes oder nicht fusioniertes Heteroaryl, umfassend 1 bis 50 Kohlenstoffatome;
(iv) ein organisches Radikal, das ein Metallelement enthält ausgewählt aus der Gruppe umfassend Ferrocen, Porphyrin und Phthalocyanin;
das Radikal R kann gegebenenfalls durch eine oder mehrere R² -Gruppen substituiert sein, die unabhängig aus der Gruppe ausgewählt werden umfassend C₁₋₁₀-Alkyl; C₂₋₁₀-Alken; C₂₋₁₀-Alkin; C₃₋₁₀-Cycloalkyl; C₁₋₁₀-Heteroalkyl; C₁₋₁₀⁻ Haloalkyl; C₆₋₁₀-Aryl, C₃₋₂₀-Heterozyclus; C₁₋₁₀-AlkylC₆₋₁₀-Aryl; C₁₋₁₀-AlkylC₃₋₁₀-Heteroaryl; F; Cl; Br; I; -NO₂; -CN; -CF₃; -CH₂CF₃; -OH; -CH₂OH; -CH₂CH₂OH; -NH₂; -CH₂NH₂; -NHCHO; -COOH; -CONH₂; -SO₃H; -CH₂SO₂CH₃; -PO₃H₂; oder eine -GR^{G1}-Funktion; in welcher G für -O-, -S-, -NR^{G2}-, -C(=O)-, -S(=O)-, -SO₂-, -C(=O)O-, -C(=O)NR^{G2}-, -OC(=O)-, -NR^{G2}C(=O)-, -OC(=O)O-, -OC (=O) NR^{G2}-, -NR^{G2}C(=O)O-, -NR^{G2}C(=O)NR^{G2}-, -C(=S)- steht, wobei jedes Auftreten von R^{G2} unabhängig von einem anderem Auftreten von R^{G2} ein Wasserstoffatom ist; oder eine lineare, verzweigte oder zyklische, gegebenenfalls substituierte C₁₋₁₂ - Alkylfunktion, ein C₁₋₁₂-Heteroalkyl, C₂₋₁₀-Alken oder eine C₂₋₁₀-Alkin-Gruppe ist; oder ein C₆₋₁₀-Aryl, C₃₋₁₀ - Heteroaryl, C₅₋₁₀-Heterocyclus, C₁₋₁₀-AlkylC₆₋₁₀Aryl oder C₁₋₁₀AlkylC₃₋₁₀Heteroaryl-Gruppe, worin das Aryl-, Heteroaryl- oder heterocyclische Radikal gegebenenfalls substituiert ist; oder aber, wenn G -NR^{G2}-, R^{G1} und R^{G2} darstellt, zusammen mit dem Stickstoffatom, mit dem sie verbunden sind, einen gegebenenfalls substituierten Heterocyclus oder ein Heteroaryl bilden.

2. Verwendung nach Anspruch 1, wobei der Ligand L ein Di-, Tri-, Tetra- oder Hexacarboxylat-Ligand ist aus der Gruppe umfassend: C₂H₂(CO₂⁻)₂ (Fumarat), C₂H₄(CO₂⁻) (Succinat), C₃H₆(CO₂⁻)₂ (Glutarat), C₄H₄(CO₂⁻)₂ (Muconat), C₄H₈(CO₂⁻)₂ (Adipat), C₅H₃S(CO₂⁻)₂ (2,5-Thiophendicarboxylat), C₆H₄(CO₂⁻)₂ (Terephthalat), C₆H₂N₂(CO₂⁻)₂ (2,5-Pyrazin-Dicarboxylat), C₇H₁₆(CO₂⁻)₂ (Azelat), C₁₀H₆(CO₂)₂ (Naphthalin-2,6-Dicarboxylat), C₁₂H₈(CO₂⁻)₂ (Biphenyl-4,4'-Dicarboxylat), C₁₂H₈N₂(CO₂⁻)₂ (Azobenzoldicarboxylat), C₁₂H₆Cl₂N₂(CO₂⁻)₂ (Dichlorazobenzoldicarboxylat), C₁₂H₆N₂(CO₂⁻)₄ (Azobenzoltetracarboxylat), C₁₂H₆N₂(OH)₂(CO₂⁻)₂ (Dihydroxoazobenzoldicarboxylat), C₆H₃(CO₂⁻)₃ (Benzol-1,2,4-tricarboxylat), C₆H₃(CO₂⁻)₃ (Benzol-1,3,5-tricarboxylat), C₂₄H₁₅(CO₂⁻)₃ (Benzol-1,3,5-tribenzoat), C₄₂H₂₇(CO₂⁻)₃ (1,3,5-Tris[4'-carboxy(1,1'-biphenyl-4-yl)benzol), C₆H₂(CO₂⁻)₄ (Benzol-1,2,4,5-tetracarboxylat, C₁₀H₄(CO₂⁻)₄ (Naphthalin-2,3,6,7-tetracarboxylat), C₁₀H₄(CO₂⁻)₄ (Naphthalin-1,4,5,8-tetracarboxylat), C₁₂H₆(CO₂⁻)₄ (Biphenyl-3,5,3',5'-tetracarboxylat und den modifizierten Analogen ausgewählt aus der Gruppe umfassend 2-Aminoterephthalat, 2-Nitroterephthalat, 2-Methylterephthalat, 2-Chloroterephthalat, 2-Bromterephthalat, 2,5-Dihydroxoterephthalat, 2,5-Diperfluoroterephthalat, Tetrafluoroterephthalat, 2,5-Dicarboxyterephthalat, Dimethyl-4,4'-biphenyldicarboxylat, Tetramethyl-4,4'-biphenyldicarboxylat und Dicarboxy-4,4'-biphenyl-dicarboxylat.

3. Verwendung nach Anspruch 1 oder 2, wobei das Anion X aus der Gruppe ausgewählt ist, die OH⁻, Cl⁻, Br⁻, F⁻, R-(COO)ₙ⁻, PF₆⁻, NO₃⁻, SO₄²⁻ und ClO₄⁻ umfasst, wobei R und n wie in Anspruch 1 definiert sind.

4. Verwendung nach einem beliebigen der Ansprüche 1 bis 3, wobei besagtes Molekülgemisch unter Folgenden ausgewählt wird:
- Ein Gemisch aus mindestens einem gesättigten Kohlenwasserstoff und mindestens einem ungesättigten Kohlenwasserstoff,
- Ein Gemisch aus mindestens einer Zusammensetzung, die ein freies oder mehrere freie Elektronenpaar(e) umfasst und mindestens eine Zusammensetzung die kein freies Elektronenpaar enthält, und
- Ein Gemisch aus Wasserstoff und Kohlenmonoxid und/oder Kohlendioxid.

5. Verwendung nach Anspruch 4, wobei das Kohlenwasserstoffgemisch ein Gemisch aus Propan und Propen, ein Gemisch aus n-Butan und Isobuten, ein Gemisch aus n-Butan und Butenen, ein Gemisch aus Acetylen und Ethylen, ein Gemisch aus Acetylen und Kohledioxid ist.

6. Verwendung nach einem beliebigen der Ansprüche 1 bis 5, wobei M für Fe steht, z gleich 3 ist und das Verhältnis y=M^{(z-1)+}/M^{z+} zwischen 0<y≤1/3 liegt.

7. Verwendung nach einem beliebigen der Ansprüche 1 bis 6, wobei der MOF-Feststoff eine dreidimensionale Abfolge von Einheiten hat, die der Formel (I) entsprechen ausgewählt aus der Gruppe umfassend:
- M₃OX[C₆H₃(CO₂)₃]₂ (Struktur des Typs MIL-100)
- M₃OX[C₆H₄(CO₂)₂]₃ (Struktur des Typs MIL-101)
- M₃OX[C₆(CH₃)₄(CO₂)₂]₃ (Struktur des Typs MIL-88B_4CH₃)
- M₆O₂X₂[C₁₀H₂(CO₂)₄]₃ (Struktur des Typs MIL-102)
- Fe₆O₂[C₁₂H₆N₂-(CO₂)₄]₃.X₂.nH₂O (Struktur des Typs MIL-127) wobei M ausgewählt ist unter Fe, Mn, Co; und X wie in Anspruch 1 oder 2 definiert ist.

8. Verwendung nach Anspruch 7, wobei M für Fe steht.

9. Ein Verfahren zur Trennung von Molekülen, die unterschiedliche Grade und/oder eine unterschiedliche Anzahl der Ungesättigtheiten haben durch vorzugsweise Adsorption von Molekülen mit einem höheren Grad und/oder Anzahl der Ungesättigtheiten auf einem porösen kristallinen MOF-Feststoff wie in einem beliebigen der Ansprüche 1 bis 8 definiert, wobei das Verfahren mindestens einen Reaktionsschritt umfasst, der aus folgendem besteht:
(i) aus dem Mischen in einem polaren Lösungsmittel:
- mindestens einer Lösung, die einen anorganischen Metallausgangsstoff enthält, der in Form des Metalls M, eines metallischen Salzes von M oder eines Koordinationskomplexes umfassend ein Metallion von M vorliegt;
- mindestens einem Liganden L' umfassend ein Radikal R, das q Gruppen *-C(=O)-R³ enthält, wobei :
● M, q und R wie in Anspruch 1 definiert sind,
● * den Verknüpfungspunkt der Gruppe mit dem Radikal R bezeichnet,
● R³ aus der Gruppe ausgewählt ist, die ein OH, ein OY umfasst, wobei Y ein Alkalikation, ein Halogen, ein - OR⁴ --O-C(=O)R⁴- oder -NR⁴R^{4'}-Radikal ist, wobei R⁴ und R^{4'} C₁₋₁₂ -Alkylradikale sind,
um ein MOF-Material zu erhalten;
(ii) im Aktivieren des in (i) erhaltenen MOF-Materials; und
(iii) im Kontakt bringen des in Schritt (ii) erhaltenen MOF-Materials mit einem Gemisch von Molekülen, die unterschiedliche Grade und/oder eine unterschiedliche Anzahl der Ungesättigtheiten haben.

10. Verfahren nach Anspruch 9, wobei Schritt (ii) ein Schritt der Reduzierung von mindestens einigen der Metallzentren M^{z+} des MOF-Materials an M^{(z-1)+}-Ionen ist, wobei z für 3 steht.

11. Verfahren nach einem beliebigen der Ansprüche 9 oder 10, wobei der Aktivierungsschritt (ii) bei Temperaturen von 25 bis 300°C durchgeführt wird.

12. Verfahren nach einem der Ansprüche 9 oder 10, ferner umfassend einen Behandlungsschritt (i') des in Schritt (i) erhaltenen MOF-Materials mit einer Quelle von F⁻-Ionen.

13. Verfahren nach Anspruch 12, wobei der Aktivierungsschritt (ii) bei Temperaturen von 50 bis 250°C durchgeführt wird.

14. Verfahren nach einem beliebigen der Ansprüche 9 bis 13, wobei das Molekülgemisch unter Folgenden ausgewählt wird:
- Ein Gemisch aus mindestens einem Paraffin und mindestens einem Olefin.
- Ein Gemisch aus mindestens einem Olefin und einem Alkin.
- Ein Gemisch aus Acetylen und Kohlendioxid.
- Ein Gemisch aus Wasserstoff und Kohlenmonoxid und/oder -dioxid.

15. Verfahren nach Anspruch 14, wobei das Molekülgemisch ein Gemisch aus Propan und Propen, ein Gemisch aus n-Butan und Isobuten, ein Gemisch aus Acetylen und Ethylen, ein Gemisch aus Acetylen und Kohlendioxid ist.

16. Verfahren nach einem beliebigen der Ansprüche 9 bis 15, wobei das Molekülgemisch ein Gemisch aus mindestens einem gesättigten Kohlenwasserstoff und mindestens einem ungesättigten Kohlenwasserstoff oder Kohlendioxid ist, und der Brönsted-Säuregehalt des in Schritt (ii) erhaltenen MOF-Materials nicht die Polymerisation der ungesättigten Kohlenwasserstoffe auslöst.

17. Verfahren nach einem beliebigen der Ansprüche 9 bis 16, wobei das Molekülgemisch in einer Gasphase vorliegt.

18. Verfahren nach einem beliebigen der Ansprüche 9 bis 17, umfassend ferner einen Schritt (iv) der Desorption des vorzugsweise adsorbierten Moleküls.

19. Verfahren nach Anspruch 18, wobei die Desorption mit Hilfe einer Desorptionstechnik durchgeführt wird, ausgewählt unter einer Verdrängung mit einem mindestens zu 98% reinem Gas bei Niederdruck, eine Druckänderung oder ein Temperaturänderung oder eine Kombination hiervon.

20. Verwendung nach einem beliebigen der Ansprüche 1 bis 8 zur Reinigung von Wasserstoff.
